# EUROPEAN PATENT SPECIFICATION

(11) **EP 3 206 491 B1**
(45) Date of publication and mention of the grant of the patent: **27.11.2019**
(21) Application number: 15851308.5
(22) Date of filing: 16.10.2015
(51) Int. Cl.: A61K 45/06, A61P 35/00

(54) **USE OF METHYLNALTREXONE TO ATTENUATE TUMOR PROGRESSION**
VERWENDUNG VON METHYLNALTREXON ZUR VERRINGERUNG DER TUMORPROGRESSION
UTILISATION DE MÉTHYLNALTREXONE POUR ATTÉNUER UNE PROGRESSION DE TUMEUR

(30) Priority: 17.10.2014 US 201462065550 P; 31.03.2015 US 201562140876 P; 23.09.2015 US 201562222746 P
(43) Date of publication of application: 23.08.2017
(73) Proprietor: Salix Pharmaceuticals, Inc., Bridgewater, NJ 08807 (US); The University of Chicago, Chicago, IL 60637 (US)
(72) Inventor: MOSS, Jonathan, Chicago, IL 60611 (US); JOHNSON, Lorin K., Los Altos Hills, California 94022 (US)
(74) Representative: Adam, Holger
(86) International application number: PCT/US2015/056060
(87) International publication number: WO 2016/061531

(56) References cited:
- WO-A1-2013/165577
- US-A1- 2006 258 696
- US-A1- 2010 286 059
- US-A1- 2013 317 050
- CLEMENS ET AL.: 'Managing opioid induced constipation in advanced illness' THER. CLIN. RISK MANAG. vol. 6, 2010, pages 77 - 82, XP055432741
- MOSS ET AL.: 'Development of peripheral opioid antagonists' MAYO CLIN PROC. vol. 83, no. 10, 2008, pages 1116 - 1130, XP008104559

## Description

### Related Applications

This application claims priority to U.S. Provisional Application No. 62/065,550, filed October 17, 2014, U.S. Provisional Application No. 62/140,876, filed March 31, 2015, and U.S. Provisional Application No. 62/222,746, filed September 23, 2015.

### Background

Opioids are widely used in treating subjects with pain. Such subjects include those with advanced cancers and other terminal diseases and also those with chronic non-malignant pain and acute non-malignant pain. Opioids are narcotic medications that activate opioid receptors located in the central nervous system to relieve pain. Opioids, however, also react with receptors outside of the central nervous system, resulting in side effects including constipation, nausea, vomiting, urinary retention, and severe itching. Notable are the effects of opioids in the gastrointestinal (GI) tract where these drugs inhibit gastric emptying and peristalsis in the intestines, thereby decreasing the rate of intestinal transit and producing constipation.

In addition, anaesthesia and analgesia may play a role in the recurrence and metastatic rate of malignancies. Several retrospective studies have demonstrated a diminished incidence of cancer recurrence following regional anaesthesia with lower doses of opioids following surgery for breast, prostate, colon cancer and melanoma, although other studies have failed to detect significant differences. In addition, the *mu* opioid receptor (MOR) has been shown to be upregulated in several types of human non-small cell lung cancer (NSCLC), and overexpression of MOR in human NSCLC can increase primary tumor growth and metastasis in xenograft models. The peripheral µ opioid receptor antagonist methylnaltrexone has been studied since the late 1970s. Accordingly, there is a need in the art for compounds and compositions useful in attenuating tumor growth.

### Summary

Provided herein is a method of treating cancer in a subject who is a fast responder to administration of a *mu* opioid receptor antagonist for constipation, wherein the method includes administering a composition containing the *mu* opioid receptor antagonist to the subject.

Also provided herein is a method of increasing survival of a subject suffering from cancer who is a fast responder to administration of a *mu* opioid receptor antagonist for constipation, wherein the method includes administering a composition containing the *mu* opioid receptor antagonist to the subject.

Embodiments are also directed to a method of slowing or stopping the growth of a tumor in a subject who is a fast responder to administration of a *mu* opioid receptor antagonist for constipation, wherein the method includes administering a composition containing the *mu* opioid receptor antagonist to the subject, wherein administration of the composition results in slowing or stopping the growth of the tumor.

Embodiments are also directed to a method of inhibiting or slowing the proliferation of tumor cells in a subject who is a fast responder to administration of a *mu* opioid receptor antagonist for constipation, wherein the method includes administering a composition containing the *mu* opioid receptor antagonist to the subject, wherein administration of the composition results in the inhibition or attenuation of tumor cell proliferation.

In some embodiments, a fast responder includes a subject who has a bowel movement or laxation response within about 1 hour, 4 hours, 8 hours, 12 hours, or 24 hours after a single-dose administration of the *mu* opioid receptor antagonist. In some embodiments, a fast responder includes a subject who has a bowel movement or laxation response within about 0 - 1 hour, 0 - 4 hours, 30 minutes to 4 hours, 0 - 8 hours, 0 - 12 hours, or 0 - 24 hours after a single-dose administration of the *mu* opioid receptor antagonist.

In some embodiments, a fast responder includes a subject who has a bowel movement or laxation response within about 4 hours post-dosing for at least 2 out of the first 4 doses of the *mu* opioid receptor antagonist. In some embodiments, a fast responder includes a subject who has a bowel movement or laxation response in about 0-4 hours post-dosing for at least 2 out of the first 4 doses of the *mu* opioid receptor antagonist.

In some embodiments, a fast responder includes a subject who has a bowel movement or laxation response within about 4 hours post-dosing for at least 4 out of 7 doses of the *mu* opioid receptor antagonist. In some embodiments, a fast responder includes a subject who has a bowel movement or laxation response in about 0-4 hours post-dosing for at least 4 out of 7 doses of the *mu* opioid receptor antagonist.

In some embodiments, the bowel movement or laxation response is rescue-free.

In some embodiments, the compositions containing the *mu* opioid receptor antagonist are administered once per day or once every other day.

In any of the foregoing embodiments, the subject can be administered at least one opioid.

In some embodiments, administration of the composition containing the *mu* opioid receptor antagonist extends the survival of the subject.

Embodiments are also directed to a method of treating a subject suffering from cancer, wherein the method includes identifying a candidate for *mu* opioid receptor antagonist therapy and administering a composition comprising a *mu* opioid receptor antagonist to the subject to prolong survival from cancer.

In some embodiments, identifying the candidate includes administering a composition containing the *mu* opioid receptor antagonist to a subject having constipation, and determining the time to a first bowel movement, wherein if the time to a first bowel movement is within about 0 - 1 hour, 0 - 4 hours, 30 minutes to 4 hours, 0 - 8 hours, 0 - 12 hours or 0 - 24 hours of administering the composition to the subject, the subject is a candidate. In some embodiments, the constipation is opioid-induced constipation.

In some embodiments, identifying the candidate includes administering an opioid and a composition containing the *mu* opioid receptor antagonist to a subject, and determining the time to a first bowel movement, wherein if the time to a first bowel movement is within about 0 - 1 hour, 0 - 4 hours, 30 minutes to 4 hours, 0 - 8 hours, 0 - 12 hours, or 0 - 24 hours of administering the composition to the subject, the subject is a candidate.

In some embodiments, the bowel movement or laxation response is rescue-free.

In some embodiments, the opioid is administered prior to or concomitantly with the composition containing the *mu* opioid receptor antagonist. For example, the opioid can be administered from about 15 minutes up to about 12 hours prior to administration of the composition, or about 12 - 24 hours prior to administration of the composition, or about 24 - 72 hours prior to administration of the composition. In some embodiments, the opioid can be administered from about 72 hours up to about 7 days prior to administration of the composition, or about 7 days up to about 30 days prior to administration of the composition.

In some embodiments, prior administration of the opioid includes administering the opioid at least once per day or once every other day.

In some embodiments, concomitant administration of the opioid includes administration at or almost at the same time, one after the other, or on the same day as the composition.

In some embodiments, the subject is administered at least one opioid.

In any of the foregoing embodiments, wherein administration of the composition extends the survival of the subject

In some embodiments, the *mu* opioid receptor antagonist is selected from the group of: a tertiary derivative of noroxymorphone, a quaternary derivative of noroxymorphone, a quaternary derivative of benzomorphans, and an N-substituted piperidine.

In one embodiment, the *mu* opioid receptor antagonist contains a compound of formula **I:** 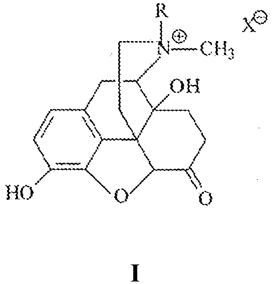 wherein R is allyl, chlorallyl, cyclopropylmethyl or propargyl, and X- is a suitable anion.

In one embodiment, the *mu* opioid receptor antagonist includes a tertiary opioid antagonist. Exemplary tertiary opioid antagonists include, but are not limited to, naloxone and naltrexone.

In some embodiments, the *mu* opioid receptor antagonist includes a peripherally acting *mu* opioid receptor antagonist (PAMORA). In some embodiments, the PAMORA is selected from the group of: naloxegol, alvimopan, axelopran and methylnaltrexone.

In some embodiments, the *mu* opioid receptor antagonist includes a tertiary opioid antagonist and/or a PAMORA. In some embodiments, the tertiary opioid antagonist is selected from naltrexone and naloxone. In some embodiments, the PAMORA is selected from the group of: naloxegol, alvimopan, axelopran and methylnaltrexone.

In some embodiments, the *mu* opioid receptor antagonist includes at least one of: naloxone, naltrexone and a PAMORA. In some embodiments, the PAMORA is selected from the group of: naloxegol, alvimopan, axelopran and methylnaltrexone.

In one embodiment, the subject expresses fewer-than-average numbers of peripheral *mu* opioid receptors.

In one embodiment, administration of the composition inhibits or attenuates endothelial cell migration.

In one embodiment, the endothelial cell migration is opioid-induced.

In one embodiment, the endothelial cell migration is induced by VEGF.

In some embodiments, the composition comprises one or more of a tablet, a capsule, a sachet, a liquid solution, powder for suspension, or a packaged composition.

In some embodiments, the composition is orally administered as about 150 mg, about 300 mg, or about 450 mg of methylnaltrexone, or a salt thereof. In some embodiments, the methylnaltrexone is administered as one or more tablets, wherein each tablet comprises about 150 mg of methylnaltrexone.

In some embodiments, the composition comprises from about 1 to about 1000 mg of methylnaltrexone, or a salt thereof.

In some embodiments, the composition is administered at a daily dose of from about 0.075 mg/kg body weight to about 0.45 mg/kg body weight. In some embodiments, the composition is administered at a daily dose of about 0.075 mg/kg body weight. In some embodiments, the composition is administered at a daily dose of about 0.15 mg/kg body weight. In some embodiments, the composition is administered at a daily dose of about 0.30 mg/kg body weight. In some embodiments, the composition is administered at a daily dose of about 0.45 mg/kg body weight.

In some embodiments, the composition is administered at a dose of from about 0.075 mg/kg body weight to about 0.45 mg/kg body weight at least once every 24 hours. In some embodiments, the composition is administered at a dose of about 0.075 mg/kg body weight, about 0.15 mg/kg body weight, about 0.30 mg/kg body weight, or about 0.45 mg/kg body weight at least once every 24 hours.

In some embodiments, the composition is administered at a dose of from about 0.075 mg/kg body weight to about 0.45 mg/kg body weight at least once every other day. In some embodiments, the composition is administered at a dose of about 0.075 mg/kg body weight, about 0.15 mg/kg body weight, about 0.30 mg/kg body weight, or about 0.45 mg/kg body weight at least once every other day.

In any of the foregoing embodiments, the subject can be administered the composition for at least about 2 weeks. In some embodiments, the subject is administered the composition for at least about 14 weeks. In some embodiments, the subject is administered the composition for at least about 16 weeks. In some embodiments, the subject is administered the composition for at least about 24 weeks.

In some embodiments, the subject is administered the composition for the duration of the subject's life. In some embodiments, the subject is administered the composition for the duration of their cancer treatment.

In some embodiments, the subject has received opioid treatment prior to administration of the composition. For example, in some embodiments, the subject has received opioid treatment for at least one month. In some embodiments, the subject has received opioid treatment for at least 1 day, 7 days, 14 days or 30 days.

In some embodiments, the subject has received opioid treatment comprising from about 10 to 300 mg of oral morphine equivalents per day. In some embodiments, the subject has received opioid treatment comprising from about 20 to 200 mg of oral morphine equivalents per day. In some embodiments, the subject has received opioid treatment comprising from about 25 to 100 mg of oral morphine equivalents per day.

In some embodiments, the subject has received opioid treatment for at least 1 day, 7 days or 14 days. In some embodiments, the subject has received opioid treatment comprising at least 50 mg of oral morphine equivalents per day for at least 14 days.

In some embodiments, the subject has had opioid induced constipation for at least one day. In some embodiments, the subject has had opioid induced constipation from 1 hour to about 30 days. In some embodiments, the subject has had opioid induced constipation for at least 30 days.

In some embodiments, the subject has experienced less than 3 rescue free bowel movements for at least one week. In some embodiments, the subject has experienced less than 3 rescue free bowel movements per week for at least four consecutive weeks.

In some embodiments, the subject will start opioid treatment in less than 1, 2, 3 or 4 weeks.

In one embodiment, the subject is also administered a cancer or an anti-tumor therapy that does not include a *mu* opioid receptor antagonist.

In one embodiment, the cancer or anti-tumor therapy includes at least one selected from the group of: a chemotherapeutic agent, radiotherapy, an anti-angiogenic agent and surgery.

In one embodiment, the cancer or anti-tumor therapy includes at least one selected from the group of: dasatinib, bevacizumab, and paclitaxel.

In some embodiments, the cancer or anti-tumor therapy includes an anti-angiogenic agent.

In one embodiment, the anti-angiogenic agent inhibits the activity of VEGF.

In one embodiment, the anti-angiogenic agent is selected from the group of: anti-VEGF antibody, thalidomide, SU5416, ribozyme, SU6668, PTK787/ZK22584, interferon-alpha and suramin.

In one embodiment, administration of the composition containing the *mu* opioid receptor antagonist blocks Src phosphorylation.

In one embodiment, the cancer or anti-tumor therapy includes an inhibitor of Src phosphorylation.

In one embodiment, the composition containing the *mu* opioid receptor antagonist includes methylnaltrexone, and the cancer or anti-tumor therapy includes administration of dasatinib, bevacizumab and/or paclitaxel.

In one embodiment, administration of the composition containing the *mu* opioid receptor antagonist inhibits or attenuates epithelial mesenchymal transition. In one embodiment, the epithelial mesenchymal transition is opioid-induced and/or growth-factor-induced.

In one embodiment, the subject is suffering from one or more of a carcinoma, sarcoma, lymphoma, leukemia or blastoma.

In one embodiment, the subject suffers from one or more of a cancer of: breast, liver, head and neck, esophageal, stomach, small intestine, colon, rectal, anal, skin, glandular, circulatory, prostate, pancreas, hematopoietic, bone marrow, bone, cartilage, fat, nerve, lung or lymph.

Also presented herein is a method of treating breast cancer in a subject who is a fast responder to administration of a *mu* opioid receptor antagonist for constipation, wherein the method includes administering a composition containing a *mu* opioid receptor antagonist to the subject.

Embodiments are also directed to a method of treating pancreatic cancer in a subject who is a fast responder to administration of a *mu* opioid receptor antagonist for constipation, wherein the method includes administering a composition containing a *mu* opioid receptor antagonist to the subject.

Embodiments are also directed to a method of treating prostate cancer in a subject who is a fast responder to administration of a *mu* opioid receptor antagonist for constipation, wherein the method includes administering a composition containing a *mu* opioid receptor antagonist to the subject.

Embodiments are also directed to a method of treating colon cancer in a subject who is a fast responder to administration of a *mu* opioid receptor antagonist for constipation, wherein the method includes administering a composition containing a *mu* opioid receptor antagonist to the subject.

Embodiments are also directed to a method of treating lung cancer in a subject who is a fast responder to administration of a *mu* opioid receptor antagonist for constipation, wherein the method includes administering a composition containing a *mu* opioid receptor antagonist to the subject.

Also presented herein is a method of identifying a subject suffering from cancer who is a candidate for *mu* opioid receptor antagonist therapy to prolong survival, wherein the method includes selecting a subject suffering from cancer who also suffers from constipation, administering to the subject a composition containing a *mu* opioid receptor antagonist; and determining the time to a first bowel movement, wherein the subject is being administered at least one opioid and wherein if the subject experiences a first bowel movement within 0 - 1 hour, 0 - 4 hours, 30 minutes to 4 hours, 0 - 8 hours, 0 - 12 hours or 0 - 24 hours of administration of the composition, the subject is a candidate. In some embodiments, the constipation is opioid-induced constipation.

Embodiments are also directed to a method of increasing survival or progression-free survival in a subject suffering from cancer, wherein the method includes determining that the subject is a candidate for *mu* opioid receptor antagonist therapy to prolong survival from cancer, and administering a composition containing a *mu* opioid receptor antagonist to the subject, wherein the subject is being administered at least one opioid and wherein administration of the composition effectively increases the survival or progression-free survival of the subject.

In some embodiments, determining that the subject is candidate includes diagnosing the subject as suffering from constipation, administering a composition containing the *mu* opioid receptor antagonist to the subject, and determining the time to a first bowel movement, wherein if the time to a first bowel movement is within 0 - 1 hour, 0 - 4 hours, 30 minutes to 4 hours, 0 - 8 hours, 0 - 12 hours or 0 - 24 hours of administering the composition to the subject, the subject is a candidate. In some embodiments, the constipation is opioid-induced constipation.

In some embodiments, the survival of the subject is increased by at least 30 days. In some embodiments, the survival of the subject is increased by at least 45 days. In some embodiments, the survival of the subject is increased by at least 60 days. In some embodiments, the survival of the subject is increased by at least 90 days.

Also provided herein is a method of preventing or treating tumor growth in a subject, wherein the method includes administering to the subject a pharmaceutical composition containing methylnaltrexone, and administration of the composition results in arrest of tumor growth or decrease in tumor size.

Also provided herein is a method of preventing or treating tumor metastasis in a subject, wherein the method includes administering to the subject a pharmaceutical composition comprising methylnaltrexone, and administration of the composition results in arrest of tumor growth or decrease in tumor size.

Embodiments are also directed to a method of preventing or treating abnormal proliferation of cells in a subject, wherein the method includes administering to the subject a pharmaceutical composition comprising methylnaltrexone, and administration of the composition results in arrest of abnormal cell proliferation.

Provided herein are methods of slowing or stopping the growth of a tumor in a subject, comprising, selecting a subject who is a suitable candidate for treatment with a *mu* opioid receptor antagonist; and administering a composition comprising the *mu* opioid receptor antagonist to the subject, wherein administration of the composition results in slowing or stopping the growth of the tumor.

Provided herein are methods of inhibiting or slowing the proliferation of tumor cells in a subject, comprising, determining if a subject is suitable candidate for treatment with a *mu* opioid receptor antagonist; and administering a composition comprising a *mu* opioid receptor antagonist to the subject, wherein administration of the composition results in the inhibition or attenuation of tumor cell proliferation.

In one embodiment, the subject is a suitable candidate if s/he has a bowel movement or laxation response within about 1 hour after administration of the composition.

In one embodiment, the bowel movement or laxation response are rescue-free.

In one embodiment, the subject is a suitable candidate if s/he has a rescue-free bowel movement or rescue-free laxation response within about 4 hours after administration of the composition.

In one embodiment, the subject is a suitable candidate if s/he has a rescue-free bowel movement or rescue-free laxation response within about 24 hours after administration of the composition.

In one embodiment, the subject is a suitable candidate if s/he has at least three rescue-free bowel movements per week following administration of the composition.

In one embodiment, the subject is a suitable candidate if the median time to rescue-free laxation after administration of the composition is within about 1 hour.

In one embodiment, the subject is a suitable candidate if the subject experiences an improvement in laxation difficulty score of ≥ 1 after administration of the compound.

In one embodiment, the subject is a suitable candidate if the subject experiences an improvement in constipation distress score of ≥ 1 after administration of the compound.

In one embodiment, the subject is a suitable candidate if the subject experiences an improvement in bowel status score of ≥ 1 after administration of the compound.

In one embodiment, the subject is a suitable candidate if the subject reduces or eliminates use of a rescue laxative after administration of the compound.

In one embodiment, the subject is a suitable candidate if s/he has a rescue-free bowel movement with about 4 hours after at least two doses of the composition.

In one embodiment, selecting the subject comprises administering the *mu* opioid receptor antagonist to a subject and determining the time to bowel movement or laxation response, wherein if the subject has a bowel movement or laxation response within 24 hours of administration of the *mu* opioid receptor antagonist, the subject is a suitable candidate.

In one embodiment, if the bowel movement or laxation response is within 1 hour of administration of the *mu* opioid receptor antagonist, the subject is a suitable candidate.

In one embodiment, if the bowel movement or laxation response is within 4 hours of administration of the *mu* opioid receptor antagonist, the subject is a suitable candidate.

In one embodiment, if the bowel movement or laxation response is within 12 hours of administration of the *mu* opioid receptor antagonist, the subject is a suitable candidate.

In one embodiment, determining if the subject is a suitable candidate comprises administering the *mu* opioid receptor antagonist to the subject and determining the time to bowel movement or laxation response, wherein if the subject has a bowel movement or laxation response within 24 hours of administration of the *mu* opioid receptor, the subject is a suitable candidate.

In one embodiment, if the bowel movement or laxation response is within 1 hour of administration of the *mu* opioid receptor, the subject is a suitable candidate.

In one embodiment, if the bowel movement or laxation response is within 4 hours of administration of the *mu* opioid receptor, the subject is a suitable candidate.

In one embodiment, if the bowel movement or laxation response is within 12 hours of administration of the *mu* opioid receptor, the subject is a suitable candidate.

In one embodiment, the tumor growth, tumor metastasis or abnormal proliferation of cells is opioid-induced.

In one embodiment, the tumor growth, tumor metastasis or abnormal proliferation of cells is activated or enhanced by *mu* opioid receptor activity.

In one embodiment, the tumor growth, tumor metastasis or abnormal proliferation of cells is induced by VEGF.

In one embodiment, the composition comprises one or more of a tablet, a capsule, a sachet, a liquid solution, or a packaged composition.

In one embodiment, the composition is orally administered as about 150 mg, about 300 mg, or about 450 mg of methylnaltrexone, or a salt thereof.

In one embodiment, the methylnaltrexone is administered as one or more tablets, wherein each tablet comprises about 150 mg of methylnaltrexone.

In one embodiment, the methylnaltrexone is administered between about 1 to about 1000 mg of methylnaltrexone, or a salt thereof.

In one embodiment, the composition is administered at a daily dose of from about 0.075 mg/kg body weight to about 0.45 mg/kg body weight.

In one embodiment, the composition is administered at a daily dose of about 0.075 mg/kg body weight, about 0.15 mg/kg body weight, about 0.30 mg/kg body weight, or about 0.45 mg/kg body weight.

In one embodiment, the composition is administered at a dose of from about 0.075 mg/kg body weight to about 0.45 mg/kg body weight at least once every 24 hours.

In one embodiment, the pharmaceutical composition is administered at a dose of about 0.075 mg/kg body weight, about 0.15 mg/kg body weight, about 0.30 mg/kg body weight, or about 0.45 mg/kg body weight at least once every 24 hours.

In one embodiment, the pharmaceutical composition is administered at a dose of from about 0.075 mg/kg body weight to about 0.45 mg/kg body weight at least once every other day.

In one embodiment, the pharmaceutical composition is administered at a dose of about 0.075 mg/kg body weight, about 0.15 mg/kg body weight, about 0.30 mg/kg body weight, or about 0.45 mg/kg body weight at least once every other day.

In one embodiment, the subject is administered the composition for at least about 2 weeks.

In one embodiment, the subject is administered the composition for at least about 4 weeks.

In one embodiment, the subject is administered the composition for at least about 14 weeks.

In one embodiment, the subject is administered the composition for at least about 16 weeks.

In one embodiment, the subject is administered the composition for at least about 24 weeks.

In one embodiment, the subject is administered the composition for the duration of the subject's life.

In one embodiment, the subject is administered the composition for the duration of their cancer treatment.

In one embodiment, the subject has been receiving opioid treatment prior to administration of the pharmaceutical composition.

In one embodiment, the subject has been receiving opioid treatment for at least one month.

In one embodiment, the subject has been receiving opioid treatment comprising at least 50 mg of oral morphine equivalents per day for at least 14 days.

In one embodiment, the subject will start opioid treatment in less than 1, 2, 3 or 4 weeks.

In one embodiment, the subject has had opioid induced constipation for at least one day.

In one embodiment, the subject has had opioid induced constipation from 1 hour to about 30 days.

In one embodiment, the subject has had opioid induced constipation for at least 30 days.

In one embodiment, the subject has experienced less than 3 rescue free bowel movements per week for at least four consecutive weeks.

In one embodiment, the composition comprises methylnaltrexone, and the subject is also administered an anti-tumor therapy that does not comprise methylnaltrexone.

In one embodiment, the anti-tumor therapy is at least one selected from the group of: a chemotherapeutic agent, radiotherapy, an anti-angiogenic agent and surgery.

In one embodiment, the anti-tumor therapy is at least one selected from the group of: dasatinib, bevacizumab, and paclitaxel.

In one embodiment, the anti-angiogenic agent inhibits the activity of VEGF.

In one embodiment, the anti-angiogenic agent is selected from the group of: anti-VEGF antibody, thalidomide, SU5416, ribozyme, SU6668, PTK787/ZK22584, interferon-alpha and suramin.

In one embodiment, administration of the composition blocks Src phosphorylation.

In one embodiment, the anti-tumor therapy comprises an inhibitor of Src phosphorylation.

In one embodiment, the composition comprises methylnaltrexone, and the anti-tumor therapy comprises administration of dasatinib, bevacizumab and/or paclitaxel.

In one embodiment, administration of the composition inhibits or attenuates epithelial mesenchymal transition.

In one embodiment, the epithelial mesenchymal transition is opioid-induced and/or growth-factor-induced.

Presented herein are methods of slowing or stopping the growth of a tumor in a subject, comprising, selecting a subject who is a fast-responder to treatment for opioid-induced constipation; and administering a composition comprising a *mu* opioid receptor antagonist to the subject, wherein administration of the composition results in slowing or stopping the growth of the tumor.

In one embodiment, a fast responder comprises a subject who has a rescue-free bowel movement or rescue-free laxation response within about 1 hour after administration of the composition.

In one embodiment, a fast responder comprises a subject who has a rescue-free bowel movement or rescue-free laxation response within about 4 hour after administration of the composition.

In one embodiment, a fast responder comprises a subject who has a rescue-free bowel movement or rescue-free laxation response within about 24 hours after administration of the composition.

In one embodiment, the *mu* opioid receptor antagonist is a peripheral *mu* opioid receptor antagonist.

In one embodiment, the peripheral *mu* opioid receptor antagonist is a quaternary-derived noroxymorphone compound.

In one embodiment, the peripheral *mu* opioid receptor antagonist is methylnaltrexone.

Presented herein are methods of preventing, treating, slowing or stopping the growth of a tumor in a subject, comprising, selecting a subject who is a fast-responder to a *mu* opioid receptor antagonist; and administering a composition comprising a *mu* opioid receptor antagonist to the subject, wherein administration of the composition results in slowing or stopping the growth of the tumor.

In one embodiment, selecting a subject who is a fast responder comprises administering a *mu* opioid receptor antagonist to a subject and determining the time to bowel movement or laxation response.

In one embodiment, selecting a fast responder comprises administering a *mu* opioid receptor antagonist to a subject and determining the time to bowel movement or laxation response, wherein a fast responder comprises a subject who has a rescue-free bowel movement or rescue-free laxation response within about 1 hour, 4 hours, 12 hours or 24 hours after administration of the composition.

In one embodiment, a fast responder comprises a subject who has a bowel movement or laxation response within about 1 hour, 4 hours, 12 hours, or 24 hours after administration of the composition.

In one embodiment, the bowel movement or laxation response comprise rescue-free responses.

In one embodiment, the *mu* opioid receptor antagonist comprises methylnaltrexone.

In one embodiment, the *mu* opioid receptor antagonist includes a tertiary opioid antagonist. Exemplary tertiary opioid antagonists include, but are not limited to, naloxone and naltrexone.

In some embodiments, the *mu* opioid receptor antagonist includes a peripherally acting *mu* opioid receptor antagonist (PAMORA). In some embodiments, the PAMORA is selected from the group of: naloxegol, alvimopan, axelopran and methylnaltrexone.

In some embodiments, the *mu* opioid receptor antagonist includes a tertiary opioid antagonist and/or a PAMORA. In some embodiments, the tertiary opioid antagonist is selected from naltrexone and naloxone. In some embodiments, the PAMORA is selected from the group of: naloxegol, alvimopan, axelopran and methylnaltrexone.

In some embodiments, the *mu* opioid receptor antagonist includes at least one of: naloxone, naltrexone and a PAMORA. In some embodiments, the PAMORA is selected from the group of: naloxegol, alvimopan, axelopran and methylnaltrexone.

In one embodiment, the method further comprises administering an anticancer agent.

In one embodiment, the tumor comprises one or more of a carcinoma, sarcoma, lymphoma, leukemia or blastoma.

In one embodiment, the tumor comprises one or more of a breast, liver, breast, head and neck, liver, oesophageal, stomach, small intestine, colon, rectal, anal, skin, glandular, circulatory, prostate, pancreas, hematopoietic, bone marrow, bone, cartilage, fat, nerve, or lymph tumor.

In one embodiment, the *mu* opioid receptor antagonist includes a tertiary opioid antagonist. Exemplary tertiary opioid antagonists include, but are not limited to, naloxone and naltrexone.

In some embodiments, the *mu* opioid receptor antagonist includes a peripherally acting *mu* opioid receptor antagonist (PAMORA). In some embodiments, the PAMORA is selected from the group of: naloxegol, alvimopan, axelopran and methylnaltrexone.

In some embodiments, the *mu* opioid receptor antagonist includes a tertiary opioid antagonist and/or a PAMORA. In some embodiments, the tertiary opioid antagonist is selected from naltrexone and naloxone. In some embodiments, the PAMORA is selected from the group of: naloxegol, alvimopan, axelopran and methylnaltrexone.

In some embodiments, the *mu* opioid receptor antagonist includes at least one of: naloxone, naltrexone and a PAMORA. In some embodiments, the PAMORA is selected from the group of: naloxegol, alvimopan, axelopran and methylnaltrexone.

In one embodiment, the responder is a fast-responder.

In one embodiment, selecting a subject who is a fast responder comprises administering a *mu* opioid receptor antagonist to a subject and determining the time to bowel movement or laxation response, wherein a fast responder comprises a subject who has a rescue-free bowel movement or rescue-free laxation response within about 1 hour, within 4 hours, within 12 hours or within 24 hours after administration of the composition.

In one embodiment, a fast responder comprises a subject who has a bowel movement or laxation response within about 1 hour, 4 hours, 12 hours or 24 hours after administration of the composition.

In one embodiment, the bowel movement or laxation response are rescue-free.

In one embodiment, the subject is not being administered opioids.

In one embodiment, the composition comprises a *mu* opioid receptor antagonist.

The methods presented herein may further comprise administering an opioid to the subject.

In one embodiment, the subject is administered from between 10 mg and 300 mg of morphine equivalents per day.

In one embodiment, the subject is administered the opioid before, during and/or after the administration of the composition comprising the *mu* opioid receptor antagonist.

In one embodiment, the subject is administered the opioid with the administration of the composition comprising the *mu* opioid receptor antagonist.

In one embodiment, inhibition or attenuation of tumor cell proliferation is assessed using histology, snp analysis, biomarker assessment, tumor markers, biopsy, MRI, CT scan, tissue microarray analysis, immunohistochemistry.

In one embodiment, inhibition or attenuation of tumor cell proliferation is assessed using VEGF, VEGFR-2, VEGF, VEGFR-2, phospho-VEGFR-2, microvessel density, CD 31, hypoxia-inducible factor-1 (HIF-1), caspase-3, p53, Ki67, autophagy-related events, total Src, pSrcY500 (negative regulation), pSrc Y419 (positive regulation) assays.

In one embodiment, inhibition or attenuation of tumor cell proliferation is assessed using tumor biopsies, skin biopsies and peripheral blood mononuclear cell (PBMC) levels.

In one embodiment, inhibition or attenuation of tumor cell proliferation is assessed using peripheral blood markers of angiogenesis and other serum biomarkers.

In one embodiment, inhibition or attenuation of tumor cell proliferation is assessed using VEGF levels, VCAM-1 levels and soluble VEGFR-2 levels.

In one embodiment, inhibition or attenuation of tumor cell proliferation is assessed using circulating cytokines, peripheral blood correlates, peripheral blood markers.

In one embodiment, the circulating cytokines, peripheral blood correlates, peripheral blood markers are measured at one or more of the following time-points, at baseline (within one week prior to Day 1 of Cycle 1 of treatment); 48 hours +/- 6 hours after dose on Day 1, Cycle 1; and/or Day 28 of Cycle 1 and/or of Cycle 2.

In one embodiment, the peripheral blood markers can be examined at the following time-points for subjects not doing DCE-MRI: (1) at baseline (within two weeks prior to Day 1 of Cycle 1 of treatment), (2) Day 6-8, Cycle 1, and (3) Day 27-28 of Cycle 1 and/or of Cycle 2.

In one embodiment, inhibition or attenuation of tumor cell proliferation is assessed using WHO criteria in subjects with lymphoma, and all others are evaluated using RECIST criteria.

In one embodiment, slowing or stopping the growth of the tumor is assessed using histology, snp analysis, biomarker assessment, tumor markers, biopsy, MRI, CT scan, tissue microarray analysis, immunohistochemistry.

In one embodiment, slowing or stopping the growth of the tumor is assessed using VEGF, VEGFR-2, VEGF, VEGFR-2, phospho-VEGFR-2, microvessel density, CD 31, hypoxia-inducible factor-1 (HIF-1), caspase-3, p53, Ki67, autophagy-related events, total Src, pSrcY500 (negative regulation), pSrc Y419 (positive regulation) assays.

In one embodiment, slowing or stopping the growth of the tumor is assessed using tumor biopsies, skin biopsies and peripheral blood mononuclear cell (PBMC) levels.

In one embodiment, slowing or stopping the growth of the tumor is assessed using peripheral blood markers of angiogenesis and other serum biomarkers.

In one embodiment, slowing or stopping the growth of the tumor is assessed using VEGF levels, VCAM-1 levels and soluble VEGFR-2 levels.

In one embodiment, slowing or stopping the growth of the tumor is assessed using WHO criteria in subjects with lymphoma, and all others are evaluated using RECIST criteria.

In one embodiment, slowing or stopping the growth of the tumor is assessed using circulating cytokines, peripheral blood correlates, peripheral blood markers.

In one embodiment, the circulating cytokines, peripheral blood correlates, peripheral blood markers are measured at one or more of the following time-points, at baseline (within one week prior to Day 1 of Cycle 1 of treatment); 48 hours +/- 6 hours after dose on Day 1, Cycle 1; and/or Day 28 of Cycle 1 and/or of Cycle 2.

In one embodiment, the peripheral blood markers can be examined at the following time-points for subjects not doing DCE-MRI: (1) at baseline (within two weeks prior to Day 1 of Cycle 1 of treatment), (2) Day 6-8, Cycle 1, and (3) Day 27-28 of Cycle 1 and/or of Cycle 2.

Other embodiments are disclosed *infra.*

### Brief Description of the Drawings

Figure 1 is a bar chart illustrating disease progression in cancer subjects who were administered methylnaltrexone or a placebo.
Figure 2 is a bar chart illustrating disease progression and responsiveness to methylnaltrexone in cancer subjects.
Figure 3 is a table that summarizes three separate efficacy endpoints in a clinical study ("Study 2") involving methylnaltrexone for treatment of opioid-induced constipation in cancer subjects.
Figure 4 is a table that summarizes three separate efficacy endpoints in a clinical study ("Study 1") involving methylnaltrexone for treatment of opioid-induced constipation in cancer subjects.
Figure 5 is a table that summarizes three separate efficacy endpoints in clinical studies (Study 1 and Study 2) involving methylnaltrexone for treatment of opioid-induced constipation in cancer subjects.
Figure 6 is a plot of a time to laxation for cancer subjects with and without adverse events of disease progression after treatment with methylnaltrexone or placebo.
Figure 7 is a plot that illustrates the difference in overall survival between the placebo group and the methylnaltrexone-treated group during the double-blind and open label extension phases in clinical studies (Study 1 and Study 2).
Figure 8 is a plot that illustrates the difference in overall survival within the methylnaltrexone-treated group between the responders and non-responders during the double-blind and open label extension phases in clinical studies (Study 1 and Study 2).
Figure 9 is a plot that illustrates the difference in overall survival between the placebo group plus the methylnaltrexone non-responsive group and the methylnaltrexone-responsive group during the double-blind and open label extension phases in clinical studies (Study 1 and Study 2).
Figure 10 is a plot of updated data that illustrates the difference in overall survival between the placebo group plus the methylnaltrexone non-responsive group and the methylnaltrexone-responsive group during the double-blind and open label extension phases in clinical studies (Study 1 and Study 2).
Figure 11 is a plot that illustrates the difference in overall survival within the placebo group between the methylnaltrexone non-responders and the methylnaltrexone responders after methylnaltrexone was administered to the placebo group during the open label extension phase of clinical studies (Study 1 and Study 2).
Figure 12 is a plot that illustrates the difference in overall survival for subjects with pancreatic cancer between the placebo group plus the methylnaltrexone non-responsive group and the methylnaltrexone-responsive group in clinical studies (Study 1 and Study 2).

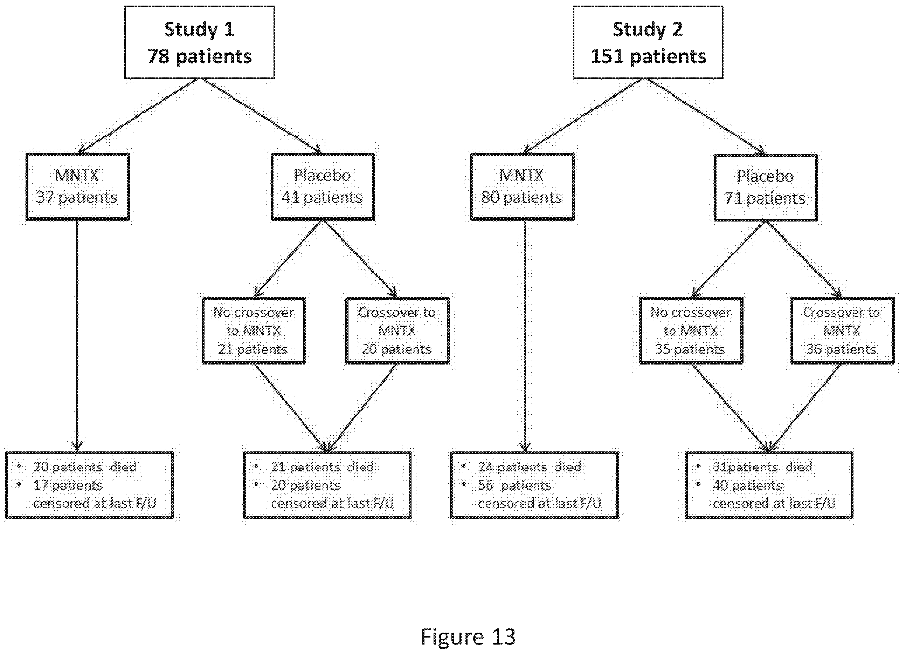

Figure 13 is a diagram that illustrates how patients diagnosed with cancer were distributed among treatment groups in clinical studies (Study 1 and Study 2).
Figure 14 is a plot that illustrates the difference in overall survival between the methylnaltrexone-treated group and the placebo group without subsequent crossover to methylnaltrexone treatment in clinical studies (Study 1 and Study 2).
Figure 15 is a plot that illustrates the difference in overall survival between the methylnaltrexone responder group and the combined cohort containing methylnaltrexone non-responders plus placebo patients without subsequent crossover to methylnaltrexone treatment in clinical studies (Study 1 and Study 2).
Figure 16 is a plot that illustrates the difference in overall survival within the placebo group between the methylnaltrexone crossover cohort and the non-crossover cohort in clinical studies (Study 1 and Study 2).
Figure 17 is a plot that illustrates the difference in overall survival between the placebo group with crossover to methylnaltrexone and the methylnaltrexone non-responders plus the placebo non-crossover cohort in clinical studies (Study 1 and Study 2).
Figure 18 is a plot that illustrates the difference in overall survival between the methylnaltrexone responsive group and the placebo plus methylnaltrexone non-responsive group for patients suffering lung cancer.
Figure 19 is a plot that illustrates the difference in overall survival between the methylnaltrexone treatment group and the placebo group for patients suffering lung cancer.
Figure 20 is a plot that illustrates the difference in overall survival between the methylnaltrexone responsive group and the placebo plus methylnaltrexone non-responsive group for patients suffering prostate cancer.
Figure 21 is a plot that illustrates the difference in overall survival between the methylnaltrexone treatment group and the placebo group for patients suffering prostate cancer.
Figure 22 is a plot that illustrates the difference in overall survival between the methylnaltrexone responsive group and the placebo plus methylnaltrexone non-responsive group for patients suffering breast cancer.
Figure 23 is a plot that illustrates the difference in overall survival between the methylnaltrexone treatment group and the placebo group for patients suffering breast cancer.

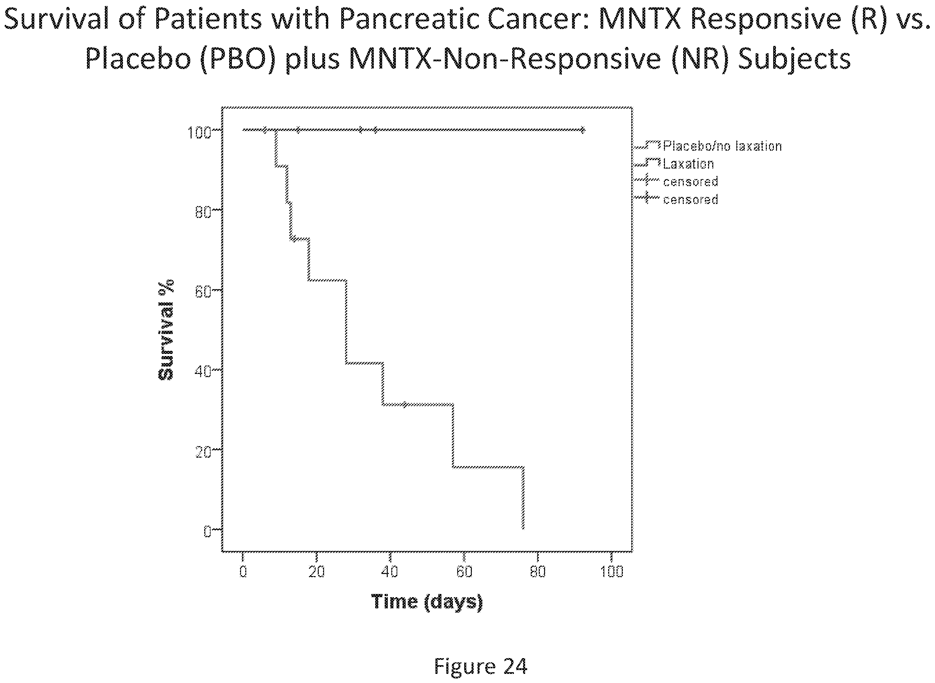

Figure 24 is a plot that illustrates the difference in overall survival between the methylnaltrexone responsive group and the placebo plus methylnaltrexone non-responsive group for patients suffering pancreatic cancer.
Figure 25 is a plot that illustrates the difference in overall survival between the methylnaltrexone treatment group and the placebo group for patients suffering pancreatic cancer.
Figure 26 is a plot that illustrates the survival of non-cancer patients in both the placebo and the methylnaltrexone-treated group in clinical studies (Study 1 and Study 2).

### Detailed Description

Provided herein are methods for treating cancer in a subject who is a responder to administration of a *mu* opioid receptor antagonist to alleviate constipation, comprising administering a therapeutically effective amount of the *mu* opioid receptor antagonist (also herein referred to as "opioid antagonist") to the subject. A responder is, for example, a subject who has a bowel movement or laxation response within about 1 hour, 2 hours, 3 hours, 4 hours, 5 hours, 6 hours, 7 hours, 8 hours, 9 hours, 10 hours, 11 hours, 12 hours, 18 hours or 24 hours after administration of the *mu* opioid receptor antagonist. In some embodiments, the responder is a fast responder, such as, for example, a subject who has a bowel movement or laxation response within about 4 hours after administration of the *mu* opioid receptor antagonist. In some embodiments, the bowel movement or laxation response is rescue-free.

Provided herein are methods of treating a subject suffering from cancer, comprising identifying or determining if the subject is a candidate for *mu* opioid receptor antagonist therapy to prolong survival from cancer, and administering a composition comprising a *mu* opioid receptor antagonist to the subject. In some embodiments, identifying or determining if a subject is a candidate comprises diagnosing the subject as suffering from constipation, administering a composition comprising the *mu* opioid receptor antagonist to the subject, and determining the time to a first bowel movement, wherein if the time to a first bowel movement is within 1 hour, 2 hours, 3 hours, 4 hours, 5 hours, 6 hours, 7 hours, 8 hours, 9 hours, 10 hours, 11 hours, 12 hours, 18 hours or 24 hours of administering the composition to the subject, the subject is a candidate.

Provided herein are methods for preventing and treating tumor growth and/or metastasis in a subject who is a fast responder to administration of a *mu* opioid receptor antagonist to alleviate constipation, comprising administering a therapeutically effective amount of the *mu* opioid receptor antagonist (also herein referred to as "opioid antagonist") to the subject. Embodiments are also directed to methods of attenuating abnormal proliferation of cells in a subject who is a fast responder to administration of a *mu* opioid receptor antagonist to alleviate constipation,, comprising administering a therapeutically effective amount of the *mu* opioid receptor antagonist. A responder can be a subject as described above.

Exemplary *mu* opioid receptor antagonists include, but are not limited to, tertiary derivatives of noroxymorphone, quaternary derivatives of noroxymorphone, quaternary derivatives of benzomorphans and N-substituted piperidines. For example, the *mu* opioid receptor antagonist can be naloxone, naltrexone, alvimopan or methylnaltrexone.

In one embodiment, the *mu* opioid receptor antagonist includes a tertiary opioid antagonist. Exemplary tertiary opioid antagonists include, but are not limited to, naloxone and naltrexone.

In some embodiments, the *mu* opioid receptor antagonist includes a peripherally acting *mu* opioid receptor antagonist (PAMORA). In some embodiments, the PAMORA is selected from the group of: naloxegol, alvimopan, axelopran and methylnaltrexone.

In some embodiments, the *mu* opioid receptor antagonist includes a tertiary opioid antagonist and/or a PAMORA. In some embodiments, the tertiary opioid antagonist is selected from naltrexone and naloxone. In some embodiments, the PAMORA is selected from the group of: naloxegol, alvimopan, axelopran and methylnaltrexone.

In some embodiments, the *mu* opioid receptor antagonist includes at least one of: naloxone, naltrexone and a PAMORA. In some embodiments, the PAMORA is selected from the group of: naloxegol, alvimopan, axelopran and methylnaltrexone. In some embodiments, the peripherally acting *mu* opioid receptor antagonist is a quaternary derivative of noroxymorphone. Quaternary derivatives of noroxymorphone are described in full in Goldberg et al., U.S. Pat. No. 4,176,186.

In general, these derivatives are represented by Formula **I:** 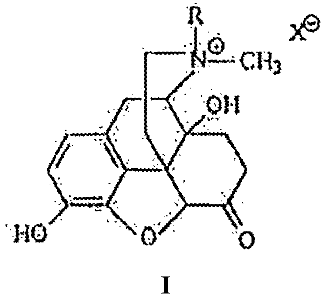 wherein R is allyl or a related radical such as chlorallyl, cyclopropyl-methyl or propargyl, and X⁻ is the anion of a suitable Brønsted acid. Exemplary Brønsted acids include hydrogen halides, carboxylic acids, sulfonic acids, sulfuric acid and phosphoric acid. In some embodiments, X⁻ is a chloride, bromide, iodide, fluoride, sulfate, bisulfate, tartrate, nitrate, citrate, bitartrate, carbonate, phosphate, malate, maleate, fumarate, sulfonate, methylsulfonate, formate, carboxylate, sulfate, methylsulfate or succinate anion. In some embodiments, X⁻ is a bromide anion. In some embodiments, the quaternary derivative of noroxymorphone is methylnaltrexone.

In some embodiments, the peripheral *mu* opioid receptor antagonist comprises a compound of Formula **II:** 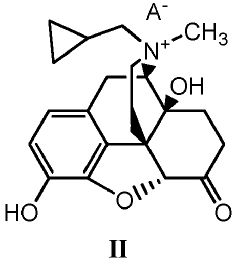 wherein A⁻ is an anion of an amphiphilic pharmaceutically acceptable excipient. In some embodiments, the amphiphilic pharmaceutically acceptable excipient is acidic. In some embodiments, the amphiphilic pharmaceutically acceptable excipient has a pKa of about 3 or less. For example, the amphiphilic pharmaceutically acceptable excipient can include a sulfate, sulfonate, nitrate, nitrite, phosphate, or phosphonate moiety. In some embodiments, the pharmaceutically acceptable excipient comprises an (-OSO₃⁻) group. Without wishing to be bound by a particular theory, such chemical functional groups with pKa values at or below about 3 allow for the ion pair to remain bound together at the acidic pH found in the stomach. The pharmaceutically acceptable excipient also includes a hydrophobic portion. In some embodiments, the hydrophobic portion is a branched or unbranched, saturated or unsaturated, cyclic or acyclic C₄₋₃₀ aliphatic chain, which can be optionally substituted. In some embodiments the pharmaceutically acceptable excipient is, for example, a saturated or unsaturated, branched or unbranched, cyclic or acyclic C₄₋₃₀ aliphatic group that is optionally substituted. In some embodiments it is a saturated, unbranched, acyclic, unsubstituted C₄₋₃₀ alkyl group. In some embodiments, it is a saturated, unbranched, acyclic, unsubstituted C₇₋₁₅ alkyl group. In some embodiments it is a C₁₂ *n*-alkyl group. In some embodiments, it is dodecyl (lauryl) sulfate. Without wishing to be bound by any theory, it is believed that the aliphatic chain makes the excipients amphiphilic and surface active in nature, which helps transport of the ion pair through the unstirred diffusion layer lining the inner surface of the gastrointestinal (GI) tract, thus increasing availability of the compound to the GI membrane for local effects on receptor sites and/or absorption across lipophilic barriers such as the lining of the GI tract, e.g., the stomach and upper duodenum. In some embodiments, the compound of Formula **II** is a salt that is solid at room temperature.

Accordingly, provided herein is a method of preventing and/or treating tumor growth or tumor metastasis in a subject who is a fast responder to administration of a *mu* opioid receptor antagonist to alleviate constipation,, comprising administering a composition comprising the *mu* opioid receptor antagonist to the subject. In some embodiments, the composition is administered subcutaneously. In some embodiments, the composition is administered orally. In some embodiments, the subject is receiving opioids chronically. In some embodiments, administration of the composition also alleviates opioid induced constipation in the subject.

Also presented herein is a method of preventing and/or treating abnormal cell proliferation in a subject who is a fast responder to administration of a *mu* opioid receptor antagonist to alleviate constipation,, comprising administering a composition comprising a *mu* opioid receptor antagonist to the subject. In some embodiments, the composition is administered subcutaneously. In some embodiments, the composition is administered orally. In some embodiments, the subject is receiving opioids chronically. In some embodiments, administration of the composition also alleviates opioid induced constipation in the subject.

A fast responder can be one who has a bowel movement or laxation response within about 1 hour, 2 hours, 3 hours, 4 hours, 5 hours, 6 hours, 7 hours, 8 hours, 9 hours, 10 hours, 11 hours, 12 hours, 18 hours or 24 hours after administration of the composition. In some embodiments, a fast responder is one who has a bowel movement or laxation response within about 0-1 hour, 0-4 hours, 30 minutes to 4 hours, 0-8 hours, 0-12 hours or 0-24 hours after administration of the composition. In some embodiments, a fast responder is a 0-1 hour responder who has a bowel movement or laxation response within about 0-1 hour after administration of the composition. In some embodiments, a fast responder is a 0-4 hour responder who has a bowel movement or laxation response within about 0-4 hours after administration of the composition. In some embodiments, a fast responder is a 0.5-4 hour responder who has a bowel movement or laxation response within about 30 minutes to 4 hours after administration of the composition. In some embodiments, a fast responder is a 0-8 hour responder who has a bowel movement or laxation response within about 0-8 hours after administration of the composition. In some embodiments, a fast responder is a 0-12 hour responder who has a bowel movement or laxation response within about 0-12 hours after administration of the composition. In some embodiments, a fast responder is a 0-24 hour responder who has a bowel movement or laxation response within about 0-24 hours after administration of the composition. In some embodiments, the bowel movement or laxation response is rescue-free.

Provided herein are methods of slowing or stopping the progression of the growth of a tumor in a subject, comprising selecting a subject who is a suitable candidate for treatment with a *mu* opioid receptor antagonist and administering a composition comprising the *mu* opioid receptor (MOR) antagonist to the subject, wherein administration of the composition results in slowing or stopping progression of tumor growth in the subject. A suitable subject can be one that is a fast responder to treatment with the *mu* opioid receptor (MOR) antagonist for a gastrointestinal disorder, such as, for example, constipation or opioid-induced constipation. In some embodiments, a suitable subject is one who has a bowel movement or laxation response within about 1 hour, 2 hours, 3 hours, 4 hours, 5 hours, 6 hours, 7 hours, 8 hours, 9 hours, 10 hours, 11 hours, 12 hours, 18 hours or 24 hours after administration of the composition. In some embodiments, a suitable subject is one who has a bowel movement or laxation response within about 0-1 hour, 0-4 hours, 30 minutes to 4 hours, 0-8 hours, 0-12 hours or 0-24 hours after administration of the composition. In some embodiments, a suitable subject is a 0-1 hour responder who has a bowel movement or laxation response within about 0-1 hour after administration of the composition. In some embodiments, a suitable subject is a 0-4 hour responder who has a bowel movement or laxation response within about 0-4 hours after administration of the composition. In some embodiments, a suitable subject is a 0.5-4 hour responder who has a bowel movement or laxation response within about 30 minutes to 4 hours after administration of the composition. In some embodiments, a suitable subject is a 0-8 hour responder who has a bowel movement or laxation response within about 0-8 hours after administration of the composition. In some embodiments, a suitable subject is a 0-12 hour responder who has a bowel movement or laxation response within about 0-12 hours after administration of the composition. In some embodiments, a suitable subject is a 0-24 hour responder who has a bowel movement or laxation response within about 0-24 hours after administration of the composition. In some embodiments, the bowel movement or laxation response is rescue-free.

Provided herein are diagnostic tests on a subject diagnosed with cancer to determine if the subject is suitable for therapy with a *mu* opioid receptor antagonist. The diagnostic test can include, for example, administering a composition containing a *mu* opioid receptor antagonist (*e.g*., a PAMORA, naloxone, or naltrexone) to the subject and assessing the time to first bowel movement or laxation response after administration of the composition. In some embodiments, the subject is considered suitable for *mu* opioid receptor antagonist therapy if the subject experiences a bowel movement or laxation response within about 0 - 1, 0 - 4, 0.5 - 4, 0 - 8, 0 - 12 or 0 - 24 hours after administration of at least one dose of the composition. In some embodiments, the subject is considered suitable for *mu* opioid receptor antagonist therapy if the subject experiences a bowel movement or laxation response within about 0 - 1, 0 - 4, 0.5 - 4, 0 - 8, 0 - 12 or 0 - 24 hours after administration of at least two out of four doses of the composition. In some embodiments, the subject is considered suitable for *mu* opioid receptor antagonist therapy if the subject experiences a bowel movement or laxation response within about 0 - 1, 0 - 4, 0.5 - 4, 0 - 8, 0 - 12 or 0 - 24 hours after administration of at least four out of seven doses of the composition. In some embodiments, the bowel movement or laxation response is rescue-free.

Determination of the subject for therapy with a *mu* opioid receptor antagonist, or diagnostic tests to determine the suitability of the subject for therapy with a *mu* opioid receptor antagonist, can be carried out with a specific *mu* opioid receptor antagonist and have applicability to *mu* opioid receptor antagonists other than the specific antagonist used in the determination. For example, it can be determined whether a subject is a responder by administration of methylnaltrexone. If the subject is determined to be a responder via administration of methylnaltrexone, the subject is considered to be a suitable candidate for *mu* opioid receptor antagonist therapy to prolong survival from cancer for all *mu* opioid receptor antagonists contemplated herein, including, *e.g.,* a PAMORA, naloxone and naltrexone.

Rescue-free, as used herein includes, for example, the non-use of rescue-laxatives, enemas or manual disimpactions. To be considered rescue-free, none of these are administered, for example, to the subject within 4 hours before and/or after each dose of MNTX. In some embodiments, to be considered rescue-free, none of these are administered, for example, to the subject within 1 hour before and/or after each dose of MNTX. In some embodiments, to be considered rescue-free, none of these are administered, for example, to the subject within 30 minutes to 6 hours before and/or after each dose of MNTX.

In some embodiments, a responder or a suitable subject is one who has at least three laxations per week after at least one administration of the composition. In some embodiments, a responder or a suitable subject is one who experiences less difficulty in laxation response after at least one administration of the composition. For example, a responder or a suitable subject can be one who experiences less straining, has a shorter time to laxation response, has softer stool consistency, or experiences less pain while having a bowel movement after at least one administration of the composition. In some embodiments, a responder or a suitable subject is one who experiences improvement in constipation distress (e.g., improvement in constipation distress score of ≥ 1) after at least one administration of the composition. In some embodiments, a responder or a suitable subject is one who experiences an improvement in bowel status (*e.g*., improvement in bowel status score of ≥ 1) after at least one administration of the composition. In some embodiments, a responder or a suitable subject is one who reduces or eliminates use of a rescue laxative after at least one administration of the composition.

Also provided herein is a method of inhibiting or slowing the proliferation of tumor cells in a subject, comprising determining if the subject is a suitable candidate for treatment with a *mu* opioid receptor antagonist and administering a composition comprising the *mu* opioid receptor antagonist to the subject, wherein administration of the composition results in the inhibition or attenuation of tumor cell proliferation.

In some embodiments, determining if the subject is a suitable candidate comprises determining that the subject is a responder to treatment with a *mu* opioid receptor antagonist. A responder is one who experiences a bowel movement or laxation response within 1 hour, 2 hours, 3 hours, 4 hours, 5 hours, 6 hours, 7 hours, 8 hours, 9 hours, 10 hours, 11 hours, 12 hours, 18 hours, 24 hours, 0-1 hour, 0-4 hours, 30 minutes to 4 hours, 0-8 hours, 0-12 hours or 0-24 hours after administration of the composition comprising the *mu* opioid receptor antagonist. In some embodiments, the responder is a fast responder who experiences a bowel movement or laxation response within about 1 hour after administration of the composition. In some embodiments, the responder is a fast responder who experiences a bowel movement or laxation response within about 2 hours after administration of the composition. In some embodiments, the responder is a fast responder who experiences a bowel movement or laxation response within about 4 hours after administration of the composition. In some embodiments, a responder is one who experiences a bowel movement or laxation response within about 8 hours after administration of the composition. In some embodiments, a responder is one who experiences a bowel movement or laxation response within about 12 hours after administration of the composition. In some embodiments, a responder is one who experiences a bowel movement or laxation response within about 24 hours after administration of the composition. In some embodiments, the bowel movement or laxation response is rescue-free.

In some embodiments, the determining step comprises evaluation of the time to first rescue-free bowel movement after administration of the composition. For example, the subject is a suitable candidate if s/he has a rescue-free bowel movement or rescue-free laxation response within about 1 hour, 2 hours, 3 hours, 4 hours, 5 hours, 6 hours, 7 hours, 8 hours, 9 hours, 10 hours, 11 hours, 12 hours, 18 hours, 24 hours, 0-1 hour, 0-4 hours, 30 minutes to 4 hours, 0-8 hours, 0-12 hours or 0-24 hours after administration of the composition.

In some embodiments, the determining step comprises evaluating the number of rescue-free bowel movements per week following administration of the composition. For example, the subject is a suitable candidate or a responder if s/he has at least three rescue-free bowel movements per week following administration of the composition. In some embodiments, the subject is determined to be a suitable candidate or a responder if s/he has four or more rescue free bowel movements per week following administration of the composition. In some embodiments, the subject is determined to be a suitable candidate or a responder if s/he has four, five or six rescue free bowel movements per week following administration of the composition.

In some embodiments, the determining step comprises evaluating the median time to rescue-free laxation after administration of the composition. In some embodiments, the subject is determined to be a suitable candidate or a responder if the median time to rescue-free laxation after administration of the composition is from about 5 minutes to about 4 hours, or from about 15 minutes to about 2 hours, or from about 30 minutes to 1 hour. For example, the subject can be a suitable candidate or a responder if the median time to rescue-free laxation after administration of the composition is within about 5 minutes, 10 minutes, 15 minutes, 30 minutes, 1 hour, 2 hours, 3 hours or 4 hours.

In some embodiments, the determining step comprises evaluating the subject's difficulty in laxation response relative to baseline after administration of the composition. For example, if difficulty in laxation response is rated on a scale, the subject is determined to be a suitable candidate or a responder if the subject experiences an improvement in laxation difficulty score of ≥ 1 after administration of the compound.

In some embodiments, the determining step comprises evaluating the subject's constipation distress relative to baseline after administration of the composition. In some embodiments, constipation distress is rated on a scale, and the subject is determined to be a suitable candidate or a responder if the subject experiences an improvement in constipation distress score of ≥ 1 after administration of the compound.

In some embodiments, the determining step comprises evaluating the subject's bowel status relative to baseline after administration of the composition. In some embodiments, bowel status is rated on a scale, and the subject is determined to be a suitable subject or a responder if the subject experiences an improvement in bowel status score of ≥ 1 after administration of the composition.

In some embodiments, the determining step comprises evaluating the subject's use of a rescue laxative relative to baseline after administration of the composition. In some embodiments, the subject is determined to be a suitable subject or a responder if the subject reduces or eliminates use of a rescue laxative after administration of the composition.

Unless otherwise defined herein, scientific and technical terms used herein shall have the meanings that are commonly understood by those of ordinary skill in the art. The meaning and scope of the terms should be clear, however, in the event of any latent ambiguity, definitions provided herein take precedent over any dictionary or extrinsic definition. Further, unless otherwise required by context, singular terms shall include pluralities and plural terms shall include the singular. In this application, the use of "or" means "and/or" unless stated otherwise. Furthermore, the use of the term "including," as well as other forms of the term, such as "includes" and "included", is not limiting.

"Peripheral," in reference to opioid antagonists, designates opioid antagonists that act primarily on physiological systems and components external to the central nervous system, *e.g.,* they do not readily cross the blood-brain barrier in an amount effective to inhibit the central effects of opioids. In other words, peripheral opioid antagonists do not effectively inhibit the analgesic effects of opioids when administered peripherally, e.g., they do not reduce the analgesic effect of the opioids. For example, the peripheral opioid antagonist compounds disclosed herein exhibit high levels of activity with respect to gastrointestinal tissue, while exhibiting reduced or substantially no central nervous system (CNS) activity. The peripheral opioid antagonist compounds disclosed herein suitably exhibit less than about 5-15% of their pharmacological activity in the CNS, with about 0% (e.g., no CNS activity) being most suitable. The non-central acting characteristic of a peripheral opioid antagonist is often related to charge, polarity and/or size of the molecule. For example, peripherally-acting quaternary amine opioid antagonists are positively charged while the central-acting tertiary amine opioid antagonists are neutral molecules. The peripheral opioid antagonists useful in the methods disclosed herein are *mu* and/or *kappa* opioid antagonists.

The term "abnormal cell proliferation" refers to abnormal, pathological, dysregulated and/or undesirable or inappropriate proliferation, division, growth or migration of cells that is not part of normal cell turnover, metabolism, growth or propagation, and generally is occurring more rapidly or to a significantly greater extent than typically occurs in a normally functioning cell of the same type and does not serve normal function. Abnormal cell proliferation and unwanted migration is manifest in disorders that are hyperproliferative in nature and include, but are not limited to, cancers, such as melanoma, lung cancer, breast cancer, pancreatic cancer, prostate cancer, colon cancer, ovarian cancer, head and neck cancer, leukemia, myeloma and/or solid tumor cancers. For example, the cancer can be one or more of a carcinoma, sarcoma, lymphoma, leukemia or blastoma. Solid tumor cancers, include, for example, adrenal cortical carcinoma, tumors of the bladder: squamous cell carcinoma, urothelial carcinomas; tumors of the bone (*e.g*., adamantinoma, aneurysmal bone cysts, chondroblastoma, chondroma, chondromyxoid fibroma, chondrosarcoma, fibrous dysplasia of the bone, giant cell tumour, osteochondroma, osteosarcoma); breast tumors (*e.g*., secretory ductal carcinoma, chordoma); colon tumors (*e.g*, colorectal adenocarcinoma); eye tumors (*e.g*., posterior uveal melanoma, fibrogenesis imperfecta ossium, head and neck squamous cell carcinoma); kidney tumors (*e.g*., chromophobe renal cell carcinoma, clear cell renal cell carcinoma, nephroblastoma (Wilms tumor), papillary renal cell carcinoma, primary renal ASPSCR1 -TFE3 tumor, renal cell carcinoma); liver tumors (*e.g*., hepatoblastoma, hepatocellular carcinoma); lung tumors (*e.g*., non-small cell carcinoma, small cell cancer; malignant melanoma of soft parts); nervous system tumors (*e.g*., medulloblastoma, meningioma, neuroblastoma, astrocytic tumors, ependymomas, peripheral nerve sheath tumors, phaeochromocytoma); ovarian tumors (*e.g.*, epithelial tumors, germ cell tumors, sex cord-stromal tumors, pericytoma; pituitary adenomas); rhabdoid tumor; skin tumors (*e.g.,* cutaneous benign fibrous histiocytomas); smooth muscle tumors (*e.g.*, intravenous leiomyomatosis); soft tissue tumors (*e.g.*, liposarcoma, myxoid liposarcoma, low grade fibromyxoid sarcoma, leiomyosarcoma, alveolar soft part sarcoma, angiomatoid fibrous histiocytoma (AFH), clear cell sarcoma, desmoplastic small round cell tumor, elastofibroma, Ewing's tumors, extraskeletal myxoid chondrosarcoma, inflammatory myofibroblastic tumor, lipoblastoma, lipoma / benign lipomatous tumors, liposarcoma / malignant lipomatous tumors, malignant myoepithelioma, rhabdomyosarcoma, synovial sarcoma, squamous cell cancer); tumors of the testis (*e.g*., germ cell tumors, spermatocyte seminoma); thyroid tumors (*e.g*., anaplastic (undifferentiated) carcinoma, oncocytic tumors, papillary carcinoma); and uterus tumors (*e.g.*, carcinoma of the cervix, endometrial carcinoma, leiomyoma). Abnormal cell proliferation and unwanted migration also play a role in disorders such as, for example, psoriasis, rheumatoid arthritis, epidermolytic by perkeratosis, restratosis, restenosis, endometriosis and abnormal wound healing. In some embodiments, the tumor can include one or more of a breast, liver, breast, head and neck, liver, esophageal, stomach, small intestine, colon, rectal, anal, skin, glandular, circulatory, prostate, pancreas, hematopoietic, bone marrow, bone, cartilage, fat, nerve, or lymph tumor.

The term "constipation" as used herein, refers to a condition in which a subject suffers from infrequent bowel movements or bowel movements that are painful and/or hard to pass. A subject experiencing constipation often suffers from straining during bowel movements and/or a sensation of incomplete evacuation following bowel movements. In some embodiments, constipation refers to a subject who experiences less than three (3) rescue free bowel movements (RFBMs) per week on average, wherein "rescue free bowel movement" refers to the passage and evacuation of feces, or laxation. In some embodiments, constipation is caused by opioid use or administration of an opioid to the subject (*e.g*., opioid induced constipation).

As used herein, the term "opioid induced constipation" (OIC) refers to a subject who suffers from constipation resulting from opioid therapy. For example, a subject can suffer from opioid induced constipation arising from opioid therapy with alfentanil, anileridine, asimadoline, bremazocine, burprenorphine, butorphanol, codeine, dezocine, diacetylmorphine (heroin), dihydrocodeine, diphenoxylate, fedotozine, fentanyl, funaltrexamine, hydrocodone, hydromorphone, levallorphan, levomethadyl acetate, levorphanol, loperamide, meperidine (pethidine), methadone, morphine, morphine-6-glucoronide, nalbuphine, nalorphine, opium, oxycodone, oxymorphone, pentazocine, propiram, propoxyphene, remifentanyl, sufentanil, tilidine, trimebutine, and/ or tramadol.

As used herein, progression-free survival refers to the length of time during and after the treatment of a cancer or solid tumor that a subject lives with the disease wherein the disease does not get worse or the solid tumor does not metastasize. According to embodiments disclosed herein, survival or progression-free survival can be increased by at least 10 days, two weeks, 30 days, 60 days, 90 days, 6 months or 1 year.

As used herein, an "effective amount" of a composition as disclosed herein refers to the level required to prevent, treat, inhibit the growth of, or decrease the size of a tumor. In some embodiments, an "effective amount" is at least a minimal amount of a composition which is sufficient for preventing or treating tumor metastasis. In some embodiments, the term "effective amount," as used in connection with an amount of a *mu* opioid receptor antagonist, or a salt thereof, or composition comprising the same, refers to an amount of *mu* opioid receptor antagonist, salt thereof, or composition thereof sufficient to achieve arrest or attenuation of the abnormal proliferation of cells in a subject. In some embodiments, the term "effective amount," as used in connection with an amount of a *mu* opioid receptor antagonist, or a salt thereof, or composition comprising the same, refers to an amount of *mu* opioid receptor antagonist, salt thereof, or composition thereof sufficient to achieve remission of cancer in a subject. In some embodiments, the term "effective amount," as used in connection with an amount of a *mu* opioid receptor antagonist, or a salt thereof, or composition comprising the same, refers to an amount of *mu* opioid receptor antagonist, salt thereof, or composition thereof sufficient to result in prolonged or extended survival of a subject.

The terms "treat" or "treating," as used herein, refers to partially or completely inhibiting the growth of, or reducing the size of, a cancerous mass of cells. In some embodiments, "treat" or "treating" refers to partially or completely inhibiting the growth of, or reducing the size of, an abnormally proliferating mass of tumor cells. In some embodiments, "treat" or "treating" refers to partially or completely arresting the disease progression of cancer in a subject. In some embodiments, "treat" or "treating" refers to achieving remission of cancer in a subject.

The expression "unit dosage form" as used herein refers to a physically discrete unit of a composition or formulation comprising a *mu* opioid receptor antagonist that is appropriate for the subject to be treated. It will be understood, however, that the total daily usage of provided formulation will be decided by the attending physician within the scope of sound medical judgment. The specific effective dose level for any particular subject will depend upon a variety of factors including the severity or stage of cancer and/or tumor progression; nature and activity of the composition; specific formulation employed; age, body weight, general health, sex and diet of the subject; time of administration, and rate of excretion of the specific active agent employed; duration of the treatment; drugs and/or additional therapies used in combination or coincidental with specific compound(s) employed, and like factors well known in the medical arts.

As used herein, the term "non-malignant pain" refers to pain originating from a non-malignant source such as cancer.

The term "subject", as used herein, means a mammal and includes human and animal subjects, such as domesticated animals (*e.g.,* horses, dogs, cats, *etc.*) and experimental animals (*e.g.,* mice, rats, dogs, chimpanzees, apes, *etc.*)*.* In a particular embodiment, the subject is human.

The term "amphiphilic" as used herein to describe a molecule refers to the molecule's dual hydrophobic and hydrophilic properties. Amphiphilic molecules have a polar, water soluble group (*e.g.,* a phosphate, carboxylic acid, sulfate) attached to a nonpolar, water-insoluble group (*e.g.,* a hydrocarbon). The term amphiphilic is synonymous with amphipathic. Examples of amphiphilic molecules include sodium dodecyl (lauryl) sulfate, fatty acids, phospholipids, and bile acids. Amphiphilic molecules can be uncharged, cationic, or anionic.

As used herein, the term "liphophilicity" refers to a compound's ability to associate with or dissolve in a fat, lipid, oil, or non-polar solvent. Lipophilicity and hydrophobicity can be used to describe the same tendency of a molecule to dissolve in fats, oils, lipids, and non-polar solvents.

Compositions useful in the methods disclosed herein can include a compound of formula **II** and/or **II'** 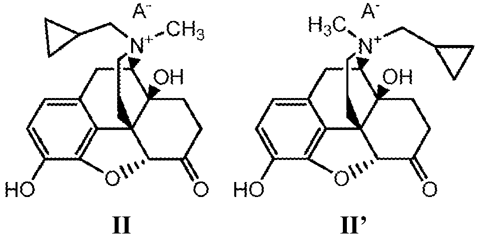 wherein A⁻ is a suitable anion.

In some embodiments, A⁻ is the anion of a suitable Brønsted acid. Exemplary Brønsted acids include, but are not limited to, hydrogen halides, carboxylic acids, sulfonic acids, sulfuric acid and phosphoric acid. In some embodiments, A⁻ is chloride, bromide, iodide, fluoride, sulfate, bisulfate, tartrate, nitrate, citrate, bitartrate, carbonate, phosphate, malate, maleate, fumarate, sulfonate, methylsulfonate, formate, carboxylate, methylsulfate or succinate salt. In some embodiments, A⁻ is trifluoroacetate. In some embodiments, A⁻ is bromide.

In some embodiments, compositions comprising a *mu* opioid receptor (*e.g.,* methylnaltrexone) are formulated in a liquid formulation. Liquid formulations and compositions of methylnaltrexone are described, for example, in International Publications No. WO 2004/091623, WO 2008/019115 and WO 2010/039851. In some embodiments, the liquid formulation or composition is provided in a packaged composition that is substantially free of tungsten, as described, for example in WO 2010/039851. For example, a packaged composition that is substantially free from tungsten can be provided, comprising a unit dosage of a liquid composition comprising methylnaltrexone, a calcium chelating agent, a buffering agent and an isotonicity agent. In some embodiments, the packaged composition can comprise a unit dosage of a liquid composition that comprises methylnaltrexone bromide, edetate calcium disodium and glycine hydrochloride. In some embodiments, the packaged composition can comprise a unit dosage of a liquid composition that comprises methylnaltrexone bromide, edetate calcium disodium and glycine hydrochloride and sodium chloride.

In some embodiments, the liquid formulation or composition comprises a compound of Formula **II** and/or **II'.**

A packaged composition can include, for example, vials, ampoules, prefilled syringes or sachets containing liquids.

In some embodiments, the liquid composition comprising the *mu* opioid receptor has a pH of from about pH 2.0 to about pH 6.0. In some embodiments, the pH of the formulation is from about pH 2.5 to about pH 5.0. In some embodiments, the pH of the formulation is from about pH 3.0 to about pH 4.0. In some embodiments, the pH of the formulation is from about pH 3.4 to about pH 3.6. In some embodiments, the pH of the formulation is about pH 3.5.

In some embodiments, the liquid composition comprises methylnaltrexone and has a pH of from about pH 2.0 to about pH 6.0. In some embodiments, the pH of the formulation comprising methylnaltrexone is from about pH 2.5 to about pH 5.0. In some embodiments, the pH of the formulation comprising methylnaltrexone is from about pH 3.0 to about pH 4.0. In some embodiments, the pH of the formulation comprising methylnaltrexone is from about pH 3.4 to about pH 3.6. In some embodiments, the pH of the formulation comprising methylnaltrexone is about pH 3.5.

In some embodiments, the packaged composition comprises a *mu* opioid receptor in an amount from about 0.5 mg to about 200 mg, about 1 mg to about 80 mg, from about 5 mg to about 40 mg. In some embodiments, the packaged composition comprises a *mu* opioid receptor in an amount of about 8 mg, about 12 mg, about 16 mg, about 18 mg, or about 24 mg.

In some embodiments, the packaged composition comprises methylnaltrexone in an amount from about 0.5 mg to about 200 mg, about 1 mg to about 80 mg, from about 5 mg to about 40 mg. In some embodiments, the packaged composition comprises methylnaltrexone bromide in an amount of about 8 mg, about 12 mg, about 16 mg, about 18 mg, or about 24 mg.

In some embodiments, the packaged composition comprises a liquid composition comprising a *mu* opioid receptor in an amount from about 0.01 mg/mL to about 25 mg/mL, or from about 0.1 mg/mL to about 20 mg/mL in the formulation, or from about 0.2 mg/mL to about 10 mg/mL of the formulation. In some embodiments, the packaged composition comprises a liquid composition comprising a *mu* opioid receptor in an amount about 20 mg/mL.

In some embodiments, the packaged composition comprises a liquid composition comprising methylnaltrexone bromide in an amount from about 0.01 mg/mL to about 25 mg/mL, or from about 0.1 mg/mL to about 20 mg/mL in the formulation, or from about 0.2 mg/mL to about 10 mg/mL of the formulation. In some embodiments, the packaged composition comprises a liquid composition comprising methylnaltrexone bromide in an amount about 20 mg/mL.

In some embodiments, a chelating agent can be present in the liquid composition an amount of from about 0.1 mg/mL to about 1 mg/mL. In some embodiments, the chelating agent is present in the liquid composition in an amount of about 0.1 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL or about 1.0 mg/mL.

Exemplary chelating agents include ethylenediaminetetraacetic acid (also synonymous with EDTA, edetic acid, versene acid, and sequestrene), and EDTA derivatives, such as sodium EDTA, and potassium EDTA, diammonium EDTA, dipotassium EDTA, disodium EDTA, TEA-EDTA, tetrasodium EDTA, tripotassium EDTA, trisodium EDTA, HEDTA, and trisodium HEDTA, and related salts thereof. Other chelating agents include niacinamide and derivatives thereof and sodium desoxycholate and derivatives thereof, ethylene glycol-bis-(2-aminoethyl)-N,N,N', N'-tetraacetic acid (EGTA) and derivatives thereof, diethylenetriaminepentaacetic acid (DTPA) and derivatives thereof, N,N-bis(carboxymethyl)glycine (NTA) and derivatives thereof, nitrilotriacetic acid and derivatives thereof. Additional chelating agents that are contemplated include citric acid and derivatives thereof. Citric acid also is known as citric acid monohydrate. Derivatives of citric acid include anhydrous citric acid and trisodiumcitrate-dihydrate. In some embodiments, the chelating agent is at least one selected from the group consisting of EDTA, an EDTA derivative, EGTA and an EGTA derivative. In some embodiments, the chelating agent comprises EDTA disodium such as, for example, EDTA disodium hydrate.

In some embodiments, a calcium salt is present in the liquid composition an amount of from about 0.1 mg/mL to about 1 mg/mL. In some embodiments, the calcium salt is present in the liquid composition in an amount of about 0.1 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL or about 1.0 mg/mL.

Exemplary calcium salts include, but are not limited to calcium chloride, calcium acetate, calcium citrate, calcium sulfate, and the like.

In some embodiments, a calcium salt chelating agent is present in the liquid composition an amount of from about 0.1 mg/mL to about 1 mg/mL. In some embodiments, the calcium salt chelating agent is present in the liquid composition in an amount of about 0.1 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL or about 1.0 mg/mL.

Calcium salt chelating agents include, but are not limited to, calcium ethylenediaminetetraacetic acid (EDTA) and calcium salt EDTA derivatives, calcium ethylene glycol-bis-(2-aminoethyl)-N,N,N', N'-tetraacetic acid (EGTA) and calcium salt EGTA derivatives, calcium diethylenetriaminepentaacetic acid (DTPA) and calcium salt DTPA derivatives, calcium N,N-bis(carboxymethyl)glycine (NTA) and calcium salt NTA derivatives, and calcium citrate and derivatives thereof. In some embodiments, the calcium salt chelating agent is at least one selected from the group of calcium EDTA, a calcium salt EDTA derivative, calcium EGTA and a calcium salt EGTA derivative. In some embodiments, the calcium salt chelating agent comprises calcium EDTA disodium such as, for example, calcium EDTA disodium hydrate.

In some embodiments, an isotonic agent is present in the liquid composition. Isotonic agents include, for example, agents selected from the group consisting of sodium chloride, mannitol, lactose, dextrose (hydrous or anhydrous), sucrose, glycerol, and sorbitol, and solutions thereof.

In some embodiments, a stabilizing agent is present in the liquid composition in an amount of from about 0.01 mg/mL to about 2 mg/mL, or from about 0.05 mg/mL to about 1 mg/mL, or from about 0.1 mg/mL to about 0.8 mg/mL. In some embodiments, the stabilizing agent can be present in an amount of about 0.10 mg/mL, about 0.15 mg/mL, about 0.2 mg/mL, about 0.25 mg/mL, about 0.3 mg/mL, about 0.35 mg/mL, about 0.4 mg/mL, about 0.45 mg/mL, about 0.5 mg/mL, about 0.6 mg/mL, about 0.7 mg/mL or about 0.8 mg/mL.

Exemplary stabilizing agents include glycine, benzoic acid, citric, glycolic, lactic, malic, and maleic acid. In some embodiments, the liquid formulation comprises glycine. In some embodiments, the glycine comprises glycine-HCl.

For intravenous or intramuscular administration, methylnaltrexone (from, e.g., Mallinckrodt Pharmaceuticals, St. Louis, Mo.) can be formulated with saline or other physiologically acceptable carriers. For transmucosal administration, methylnaltrexone can be formulated with a sugar and cellulose mix or other pharmacologically acceptable carriers known in the art.

In some embodiments, the methods presented herein involve administration of oral compositions of a *mu* opioid receptor antagonist. The compositions for oral administration can comprise at least one pharmaceutically acceptable excipient. For example, the oral composition can comprise a pharmacologically acceptable binder to make a tablet or capsule containing the *mu* opioid receptor antagonist.

In some embodiments, the compositions for oral administration are enterically coated. The enteric coating can be made of any suitable composition. Suitable enteric coatings are described, for example, in U.S. Patent No. 4,311,833 to Namikoshi, et al.; U.S. Patent No. 4,377,568 to Chopra; U.S. Patent No. 4,385,078 to Onda, et al.; U.S. Patent No. 4,457,907 to Porter; U.S. Patent No. 4,462,839 to McGinley, et al.; U.S. Patent No. 4,518,433 to McGinley, et al.; U.S. Patent No. 4,556,552 to Porter, et al.; U.S. Patent No. 4,606,909 to Bechgaard et al.; U.S. Patent No. 4,615,885 to Nakagame, et al.; U.S. Patent No. 4,670,287 to Tsuji; U.S. Patent No. 5,536,507 TO Abramowitz, et al.; U.S. Patent No. 5,567,423 to Ying, et al.; U.S. Patent No. 5,591,433 to Michael, et al.; U.S. Patent No. 5,597,564 to Ying, et al.; U.S. Patent No. 5,609,871 to Michael, et al.; U.S. Patent No. 5,614,222 to Kaplan; U.S. Patent No. 5,626,875 to Rodes, et al.; and U.S. Patent No. 5,629,001 to Michael, et al.,

In some embodiments, the enteric coating compositions include alkyl and hydroxyalkyl celluloses and their aliphatic esters, *e.g*., methylcellulose, ethylcellulose, hydro xyethylcellulo se, hydro xypropylcellulo se, hydro xybutylcellulo se, hydro xyethylethylcellulo se, hydro xyprophymethylcellulo se, hydro xybutylmethylcellulo se, hydroxypropylcellulose phthalate, hydroxypropylmethylcellulose phthalate and hydroxypropylmethylcellulose acetate succincate; carboxyalkylcelluloses and their salts, *e.g.,* carboxymethylethylcellulose; cellulose acetate phthalate; cellulose acetate trimellitate, polycarboxymethylene and its salts and derivatives; polyvinylalcohol and its esters: polyvinyl acetate phthalate; polycarboxymethylene copolymer with sodium formaldehyde carboxylate; acrylic polymers and copolymers, *e.g*., methacrylic acid-methyl methacrylic acid copolymer and methacrylic acid-methyl acrylate copolymer; edible oils such as peanut oil, palm oil, olive oil and hydrogenated vegetable oils; polyvinylpyrrolidone; polyethyleneglycol and its esters: natural products such as shellac, and zein.

Other suitable enteric coatings include polyvinylacetate esters, *e.g*., polyvinyl acetate phthalate; alkyleneglycolether esters of copolymers such as partial ethylene glycol monomethylether ester of ethylacrylate-maleic anhydride copolymer or diethyleneglycol monomethylether ester of methylacrylate-maleic anhydride copolymer, N-butylacrylate-maleic anhydride copolymer, isobutylacrylate-maleic anhydride copolymer or ethylacrylate-maleic anhydride copolymer; and polypeptides resistant to degradation in the gastric environment, *e.g*., polyarginine and polylysine.

Mixtures of two or more of the above compounds can be used as desired. In some embodiments, the enteric coating comprises cellulose acetate phthalate.

The enteric coating material can be mixed with various excipients including plasticizers such as triethyl citrate, acetyl triethyl citrate, diethyl phthalate, dibutyl phthalate, dibutyl subacute, dibutyl tartrate, dibutyl maleate, dibutyl succinate and diethyl succinate and inert fillers such as chalk or pigments.

The composition and thickness of the enteric coating can be selected to dissolve immediately upon coated with the digestive juice of the intestine. In some embodiments, the composition and thickness of the anterior coating can be selected to be a time-release coating which dissolves over a selected period of time, as is well known in the art.

The amount of enteric coating depends on the particular enteric coating composition used and is sufficient to substantially prevent the absorption of the *mu* opioid receptor antagonist in the stomach.

Hydroxyalkyl celluloses and their aliphatic esters, carboxyalkyl celluloses and their salts, polycarboxymethylene and its salts and derivatives, polyvinyl alcohol and its esters, polycarboxymethylene copolymer with sodium formaldehyde carboxylates, polyvinylpyrrolidone, and polyethylene glycol and its esters can be applied as enteric coatings by first dissolving the compound in a minimum amount of water. Alcohol is then added to the point of incipient cloudiness. The mixture can then be applied by known techniques.

Application of cellulose acetate phthalate can be accomplished by dissolving the cellulose acetate phthalate in a minimum amount of alcohol and then applying by techniques known in the art. Hydrogenated vegetable oils can be applied by first dissolving the oil in a minimal amount of a non-polymer solvent, such as methylene chloride, chloroform or carbon tetrachloride, then adding alcohol to the point of incipient cloudiness and then applying by techniques known in the art.

In some embodiments, the methods presented herein involve administration of oral compositions of methylnaltrexone comprising ion pairs of methylnaltrexone and an amphiphilic pharmaceutically acceptable excipient. For example, the composition for use in the methods presented herein can be a salt of methylnaltrexone of the formula: 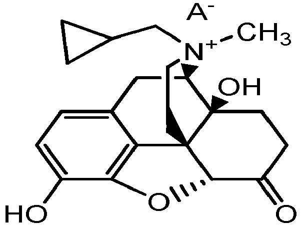 wherein methylnaltrexone is the cation of the salt, and A⁻ is an anion of an amphiphilic pharmaceutically acceptable excipient, as described in International Publication No. WO2011/112816. In certain embodiments, the methylnaltrexone is (*R*)-N-methylnaltrexone, a peripherally acting µ opioid receptor antagonist, as shown in the formula above. It will be understood that the (*R*)-N-methylnaltrexone cation and the anion of the amphiphilic pharmaceutically acceptable excipient can exist in the composition as an ion pair or can exist as separate salts paired with other counter ions such as bromide and sodium, or mixtures thereof.

The compositions for oral administration further include an anion of an amphiphilic pharmaceutically acceptable excipient (A⁻). The amphiphilic pharmaceutically acceptable excipient increases the lipophilicity of the composition thereby allowing for increased transport through the unstirred diffusion layer in the GI tract, resulting in increased permeation through biological membranes. In certain embodiments, the excipient increases the lipophilicity of the drug.

In some embodiments, the amphiphilic pharmaceutically acceptable excipient can include a sulfate, sulfonate, nitrate, nitrite, phosphate, or phosphonate moiety. In some embodiments, the pharmaceutically acceptable excipient comprises an (-OSO₃⁻ ) group. In some embodiments, the anion is butyl sulfate, pentyl sulfate, hexyl sulfate, heptyl sulfate, octyl sulfate, nonyl sulfate, decyl sulfate, undecyl sulfate, dodecyl sulfate, tridecyl sulphate, tetradecyl sulfate, pentadecyl sulfate, hexadecyl sulfate, heptadecyl sulfate, octadecyl sulfate, eicosyl sulfate, docosyl sulfate, tetracosyl sulfate, hexacosyl sulfate, octacosyl sulfate, and triacontyl sulphate.

In some embodiments, A⁻ is the anion of a Brønsted acid. Exemplary Brønsted acids include hydrogen halides, carboxylic acids, sulfonic acids, sulfuric acid, and phosphoric acid. In some embodiments, A⁻ is chloride, bromide, iodide, fluoride, sulfate, bisulfate, tartrate, nitrate, citrate, bitartrate, carbonate, phosphate, malate, maleate, fumarate sulfonate, methylsulfonate, formate, carboxylate, methylsulfate or succinate salt. In some embodiments, A⁻ is trifluoroacetate.

In some embodiments, the methylnaltrexone in the composition can have multiple anions (*e.g*., bromide and dodecyl (lauryl) sulfate) associating therewith.

In some embodiments, A⁻ is bromide, such that the compositions, and formulations thereof, comprise (*R*)-N-methylnaltrexone bromide. (*R*)-*N*-methylnaltrexone bromide, which is also known as "MNTX" and is described in international PCT patent application publication number, WO2006/12789, which is incorporated herein by reference. The chemical name for (*R*)-*N*-methylnaltrexone bromide is (*R*)-*N*-(cyclopropylmethyl) noroxymorphone methobromide. (*R*)-*N*-methylnaltrexone bromide has the molecular formula C₂₁H₂₆NO₄Br and a molecular weight of 436.36 g/mol. (*R*)-*N*-methylnaltrexone bromide has the following structure: 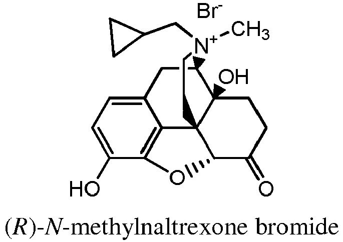 where the compound is in the (*R*) configuration with respect to the quaternary nitrogen. In certain embodiments presented herein, at least about 99.6%, 99.7%, 99.8%, 99.85%, 99.9%, or 99.95% of the compound is in the (*R*) configuration with respect to nitrogen. Methods for determining the amount of (*R*)-*N*-methylnaltrexone bromide, present in a sample as compared to the amount of (*S*)-*N*-methylnaltrexone bromide present in that same sample, are described in detail in WO2006/127899. In other embodiments, the methylnaltrexone contains 0.15%, 0.10%, or less (*S*)-*N-*methylnaltrexone bromide.

In certain embodiments, A⁻ is an acidic amphiphilic pharmaceutically acceptable excipient. In certain embodiments, the pharmaceutically acceptable excipient has a pKₐ of about 3 or less. In certain embodiments, the pharmaceutically acceptable excipient has a pKₐ of about 2 or less. In certain embodiments, the pharmaceutically acceptable excipient has a pKₐ between about 1 and about 2. In certain embodiments, the pharmaceutically acceptable excipient has a pKₐ of about 1 or less.

In some embodiments, the compositions for oral administration are tablet formulations. In some embodiments, the compositions for oral administration are capsule formulations. Methylnaltrexone for use in such compositions and formulations can be in any of a variety of forms. For example, forms of methylnaltrexone suitable for use in the inventive compositions and formulations include pharmaceutically acceptable salts, prodrugs, polymorphs (i.e., crystal forms), co-crystals, hydrates, solvates, and the like. Any form of methylnaltrexone can be used in the compositions or formulations, but the form should allow for ion pairing with the amphiphilic pharmaceutically acceptable excipient. In certain embodiments, the methylnaltrexone ion pair is a salt that is solid at room temperature. In some embodiments, the composition is a pharmaceutical composition.

In general, formulations for oral administration comprise methylnaltrexone, an amphiphilic pharmaceutically acceptable excipient as described above, and a disintegrant, and further, optionally, comprise one or more other components, such as, for example, binders, carriers, chelating agents, antioxidants, fillers, lubricants, wetting agents, or combinations thereof, as set forth in International Publication No. WO2011/112816, the entire contents of which are hereby incorporated by reference herein.

In a particular embodiment, the composition, for example, pharmaceutical composition, for oral administration comprises methylnaltrexone bromide and sodium dodecyl (lauryl) sulfate (also known as SDS or SLS). In certain embodiments, the composition further includes sodium bicarbonate as a disintegrant. Additional excipients, as set forth above, can be incorporated, including, but not limited to, at least one of microcrystalline cellulose, crospovidone, polysorbate 80, edetate calcium disodium dehydrate, silicified microcrystalline cellulose, talc, colloidal silicon dioxide and magnesium stearate. In one embodiment, the composition for oral administration comprises each of methylnaltrexone bromide, sodium lauryl sulfate, sodium bicarbonate, microcrystalline cellulose, crospovidone, polysorbate 80, edetate calcium disodium dehydrate, silicified microcrystalline cellulose, talc, colloidal silicon dioxide and magnesium stearate.

Compositions and formulations thereof for use as described herein can be generated as set forth, for example, in U.S. Patent Publication No. 2012/0190702; U.S. Patent No. 8,552,025; U.S. Patent Publication No. 2008/0070975; U.S. Patent No. 8,420,663; and International Publication No. WO 2011/112816.

In some embodiments, the opioid receptor antagonist is provided as a formulation comprising the opioid receptor antagonist and phosphatidylcholine (PC). In some embodiments, the formulation comprises the opioid receptor antagonist and PC in a molar ratio of from about 2:1 to about 1:10, or from about 1:1 to about 1:5 or about 1:2 (opioid receptor antagonist : PC). In some embodiments, the opioid receptor antagonist is methylnaltrexone. Formulations containing an opioid receptor antagonist and PC are described, for example, in International Publication No. WO 2013/165577.

The particular mode of administration of the opioid antagonist, generally speaking, can be conducted using any mode of administration that is medically acceptable, e.g., any mode that produces effective levels of the active compounds without causing clinically unacceptable adverse effects. Such modes of administration include oral, rectal, sublingual, intramuscular, infusion, intravenous, intracavity or subcutaneous. Direct injection could also be used for local delivery. Oral or subcutaneous administration can be suitable for prophylactic or long term treatment because of the convenience of the subject as well as the dosing schedule.

In some embodiments, the opioid antagonists can be administered as an enterically coated tablet or capsule. In some embodiments, the opioid antagonist is administered by a slow infusion method or by a time-release or controlled-release method or as a lyophilized powder.

When administered, the compounds and compositions as disclosed herein are provided in pharmaceutically acceptable amounts and in pharmaceutically acceptable compositions or preparations. Such preparations can routinely contain salts, buffering agents, preservatives, and optionally other therapeutic ingredients. When used in medicine, the salts can be pharmaceutically acceptable salts, as known to those of skill in the art, although non-pharmaceutically acceptable salts can also be used. Such pharmacologically and pharmaceutically acceptable salts include, but are not limited to, those prepared from the following acids: hydrochloric, hydrobromic, sulfuric, nitric, phosphoric, maleic, acetic, salicylic, p-toluenesulfonic, tartaric, citric, methanesulfonic, formic, succinic, naphthalene-2-sulfonic, pamoic, 3-hydroxy-2-naphthalenecarboxylic, and benzene sulfonic. Suitable buffering agents include, but are not limited to, acetic acid and salts thereof (1-2% WN); citric acid and salts thereof (1-3% WN); boric acid and salts thereof (0.5-2.5% WN); and phosphoric acid and salts thereof (0.8-2% WN).

Suitable preservatives include, but are not limited to, benzalkonium chloride (0.003-0.03% WN); chlorobutanol (0.3-0.9% WIN); parabens (0,01-0.25% WN) and thimerosal (0.004-0.02% WN).

For ease of administration, a pharmaceutical composition of the peripheral opioid antagonist can also contain one or more pharmaceutically acceptable excipients, such as lubricants, diluents, binders, carriers, and disintegrants. Other auxiliary agents can include, e.g., stabilizers, wetting agents, emulsifiers, salts for influencing osmotic pressure, coloring, flavoring and/or aromatic active compounds.

A pharmaceutically acceptable carrier or excipient refers to a non-toxic solid, semisolid or liquid filler, diluent, encapsulating material or formulation auxiliary of any type. For example, suitable pharmaceutically acceptable carriers, diluents, solvents or vehicles include, ,but are not limited to, water, salt (buffer) solutions, alcohols, gum arabic, mineral and vegetable oils, benzyl alcohols, polyethylene glycols, gelatin, carbohydrates such as lactose, amylose or starch, magnesium stearate, talc, silicic acid, viscous paraffin, vegetable oils, fatty acid monoglycerides and diglycerides, pentaerythritol fatty acid esters, hydroxy methylcellulose, polyvinyl pyrrolidone, etc. Proper fluidity can be maintained, for example, by the use of coating materials such as lecithin, by the maintenance of the required particle size in the case of dispersions and by the use of surfactants. Prevention of the action of microorganisms can be ensured by the inclusion of various antibacterial and antifungal agents such as paraben, chlorobutanol, phenol, sorbic acid and the like.

If a pharmaceutically acceptable solid carrier is used, the dosage form of the analogs can be in the form of, for example, tablets, capsules, powders, suppositories, or lozenges. If a liquid carrier is used, exemplary forms such as soft gelatin capsules, syrups or liquid suspensions, emulsions or solutions can be the dosage form.

For parental application, particularly suitable are injectable, sterile solutions, preferably nonaqueous or aqueous solutions, as well as dispersions, suspensions, emulsions, or implants, including suppositories. Ampoules are often convenient unit dosages. Injectable depot forms can also be suitable and can be made by forming microencapsule matrices of the drug in biodegradable polymers such as polylactide-polyglycolide, poly(orthoesters) and poly(anhydrides). Depending upon the ratio of drug to polymer and the nature of the particular polymer employed, the rate of drug release can be controlled.

Depot injectable formulations can also be prepared by entrapping the drug in liposomes or microemulsions which are compatible with body tissues. The injectable formulations can be sterilized, for example, by filtration through a bacterial-retaining filter or by incorporating sterilizing agents in the form of sterile solid compositions which can be dissolved or dispersed in sterile water or other sterile injectable media just prior to use.

For enteral application, particularly suitable are tablets, dragees, liquids, drops, suppositories, or capsules such as soft gelatin capsules. A syrup, elixir, or the like can be used wherein a sweetened vehicle is employed.

Other delivery systems can include, for example, time-release, delayed-release or sustained-release delivery systems. Such systems can avoid repeated administrations of the compounds disclosed herein, increasing convenience to the subject and the physician and maintain sustained plasma levels of compounds. Many types of controlled-release delivery system are available and known to those of ordinary skill in the art. Sustained- or controlled-release compositions can be formulated, e.g., as liposomes or those wherein the active compound is protected with differentially degradable coatings, such as by microencapsulation, multiple coatings, and the like.

For example, the compounds and compositions as disclosed herein can be combined with pharmaceutically acceptable sustained-release matrices, such as biodegradable polymers, to form therapeutic compositions. An exemplary sustained-release matrix is a matrix made of materials, usually polymers, which are degradable by enzymatic or acid-base hydrolysis or by dissolution. Once inserted into the body, the matrix is acted upon by enzymes and body fluids. A sustained-release matrix can be desirably chosen from biocompatible materials such as liposomes, polymer-based system such as polylactides (polylactic acid), polyglycolide (polymer of glycolic acid), polylactide co-glycolide (copolymers of lactic acid and glycolic acid), polyanhydrides, poly (ortho)esters, polysaccharides, polyamino acids, hyaluronic acid, collagen, chondroitin sulfate, polynucleotides, polyvinyl propylene, polyvinyl pyrrolidone, and silicone; nonpolymer system such as carboxylic acids, fatty acids, phospholipids, amino acids, lipids such as sterols, hydrogel release system; silastic system; peptide-based system; implants and the like. Specific examples include, but are not limited to: (a) erosional system in which the polysaccharide is contained in a form within a matrix, described, for example, in U.S. Pat. Nos. 4,452,775, 4,675,189, and 5,736,152 (each of which is incorporated herein by reference in its entirety), and (b) diffusional system in which an active component permeates at a controlled rate from a polymer, such as that described in U.S. Pat. Nos. 3,854,480,5,133,974 and 5,407,686. In addition, pump-based hard-wired delivery system can be used, some of which are adapted for implantation. Suitable enteric coatings are described in, for example, International Publication No. WO 1998/025613 and U.S. Pat. No. 6,274,591.

Use of a long-term sustained-release implant can be suitable for treatment of chronic conditions. The implant can be constructed and arranged to deliver therapeutic levels of the active ingredient for at least 7 days, and optionally for from about 30 to 60 days. Long-term sustained-release implants are well-known to those of ordinary skill in the art and include some of the release system described above.

With respect to methylnaltrexone, aqueous formulations can include chelating agent, a buffering agent, an anti-oxidant and, optionally, an isotonicity agent, preferably pH adjusted to between 3.0 and 3.5. Preferred such formulations that are stable to autoclaving and long term storage are described, for example, in U.S. Patent No. 8,552,025.

In some embodiments, compounds are administered in a dosing regimen which provides a continuous dosing regimen of the compound to a subject, e.g., a regimen that maintains minimum plasma levels of the opioid antagonist, and preferably eliminates the spikes and troughs of a drug level with other regimens. Suitably, a continuous dose can be achieved by administering the compound to a subject on a daily basis using any of the delivery methods disclosed herein. In some embodiments, the continuous dose can be achieved using continuous infusion to the subject, or via a mechanism that facilitates the release of the compound over time, for example, a sustained release formulation. Suitably, compounds can be continuously released to the subject in amounts sufficient to maintain a concentration of the compound in the plasma of the subject effective to reduce or inhibit opioid-induced side effects. The compounds and compositions as disclosed herein, whether provided alone or in combination with other therapeutic agents, are provided in a therapeutically effective amount. It will be understood, however, that the total daily usage of the compounds and compositions disclosed herein will be decided by the attending physician within the scope of sound medical judgment. The specific therapeutically effective dose level for any particular subject will depend upon a variety of factors including the disorder being treated and the severity of the disorder; activity of the specific compound employed; the specific composition employed; the age, body weight, general health, sex and diet of the subject; the time of administration; the route of administration; the rate of excretion of the specific compound employed; the duration of the treatment; drugs used in combination or coincidental with the specific compound employed and like factors well known in the medical arts.

If desired, the effective daily dose can be divided into multiple doses for purposes of administration. Consequently, single dose compositions can contain such amounts or submultiples thereof to make up the daily dose. Those of ordinary skill in the art can readily determine effective doses and co-administration regimens (as described herein) as determined by good medical practice and the clinical condition of the individual subject.

In some embodiments, the opioid antagonists are co-administered with the opioid. The term "co-administration" is meant to refer to a combination therapy by any administration route in which two or more agents are administered to a subject or subject. Co-administration of agents can also be referred to as combination therapy or combination treatment. The agents can be in the same dosage formulations or separate formulations. For combination treatment with more than one active agent, where the active agents are in separate dosage formulations, the active agents can be administered concurrently, or they each can be administered at separately staggered times. The agents can be administered simultaneously or sequentially (e.g., one agent can directly follow administration of the other or the agents can be give episodically, e.g., one can be given at one time followed by the other at a later time, e.g., within a week), as long as they are given in a manner sufficient to allow both agents to achieve effective concentrations in the body. The agents can also be administered by different routes, e.g., one agent can be administered intravenously while a second agent is administered intramuscularly, intravenously or orally. In other words, the co-administration of the opioid antagonist compound in accordance with the present methods with an opioid is suitably considered a combined pharmaceutical preparation which contains an opioid antagonist and a opioid agent, the preparation being adapted for the administration of the peripheral opioid antagonist on a daily or intermittent basis, and the administration of opioid agent on a daily or intermittent basis. Thus, the opioid antagonists can be administered prior to, concomitant with, or after administration of the opioids. In some embodiments, the opioid is administered prior to administration of the *mu* opioid receptor antagonist. Prior administration can involve administration of the opioid from about 15 minutes up to about 30 days prior to administration of the *mu* opioid receptor antagonist. For example, the opioid can be administered from about 15 minutes up to about 4 hours prior to administration of the *mu* opioid receptor antagonist. In some embodiments, the opioid is administered about 4-12 hours prior to administration of the *mu* opioid receptor antagonist. In some embodiments, the opioid is administered about 12-24 hours prior to administration of the *mu* opioid receptor antagonist. In some embodiments, the opioid is administered about 24-72 hours prior to administration of the *mu* opioid receptor antagonist. In some embodiments, the opioid is administered from about 72 hours up to about 7 days prior to administration of the *mu* opioid receptor antagonist. In some embodiments, the opioid is administered from about 7 days up to about 30 days prior to administration of the *mu* opioid receptor antagonist. Co-administrable agents also can be formulated as an admixture, as, for example, in a single formulation or single tablet. These formulations can be parenteral or oral, such as the formulations described, e.g., in U.S. Pat. Nos. 6,277,384; 6,261,599; 5,958,452 and International Publication No. WO 98/25613.

The compounds and compositions disclosed herein are useful in preventing or inhibiting the growth of, or reducing the size of, tumors and/or cancerous growths. In some embodiments, the compounds and compositions can be used to attenuate or arrest the abnormal proliferation of cells (*e.g.,* tumor cells). A wide variety of tumors can be treated using the compositions and methods disclosed herein, including cancers of the brain, lung, liver, spleen, kidney, lymph node, pancreas, small intestine, blood cells, colon, stomach, breast, endometrium, prostate, testicle, ovary, skin, head and neck, esophagus, bone marrow, blood or other tissue. For example, such tumors include, but are not limited to adrenal cortical carcinoma, tumors of the bladder: squamous cell carcinoma, urothelial carcinomas; tumors of the bone: adamantinoma, aneurysmal bone cysts, chondroblastoma, chondroma, chondromyxoid fibroma, chondrosarcoma, fibrous dysplasia of the bone, giant cell tumour, osteochondroma, osteosarcoma; breast tumors: secretory ductal carcinoma, chordoma; colon tumors: colorectal adenocarcinoma; eye tumors: posterior uveal melanoma, fibrogenesis imperfecta ossium, head and neck squamous ceil carcinoma; kidney tumors: chromophobe renal cell carcinoma, clear cell renal cell carcinoma, nephroblastoma (Wilms tumor), kidney: papillary renal cell carcinoma, primary renal ASPSCR.1-TFE3 tumor, renal cell carcinoma; liver tumors: hepatoblastoma, hepatocellular carcinoma; lung tumors: non-small cell carcinoma, small cell cancer; malignant melanoma of soft parts; nervous system tumors: medulloblastoma, meningioma, neuroblastoma, astrocytic tumors, ependymomas, peripheral nerve sheath tumors, phaeochromocytoma; ovarian tumors: epithelial tumors, germ cell tumors, sex cord-stromal tumors, pericytoma; pituitary adenomas; rhabdoid tumor; skin tumors: cutaneous benign fibrous histiocytomas; smooth muscle tumors: intravenous leiomyomatosis; soft tissue tumors: liposarcoma, myxoid liposarcoma, low grade fibromyxoid sarcoma, leiomyosarcoma, alveolar soft part sarcoma, angiomatoid fibrous histiocytoma (AFH), clear cell sarcoma, desmoplastic small round cell tumor, elastofibroma, Ewing's tumors, extraskeletal myxoid chondrosarcoma, inflammatory myofibroblastic tumor, lipoblastoma, lipoma/benign lipomatous tumors, liposarcoma/malignant lipomatous tumors, malignant myoepithelioma, rhabdomyosarcoma, synovial sarcoma, squamous ceil cancer; tumors of the testis: germ cell tumors, spermatocyte seminoma; thyroid tumors: anaplastic (undifferentiated) carcinoma, oncocytic tumors, papillary carcinoma; uterus tumors: carcinoma of the cervix, endometrial carcinoma, leiomyoma, and the like. Embodiments are also directed to the provision of a method of treating abnormal tumors, comprising administering to a subject in need of such treatment, an effective amount of an opioid antagonist.

In some embodiments, the compounds and compositions disclosed herein are also useful in antagonizing undesirable side effects of opioid analgesic therapy (e.g., gastrointestinal effects (e.g., delayed gastric emptying, altered GI tract motility), etc.). Furthermore, a provided compound or composition can be used as to treat subjects having disease states that are ameliorated by binding µ opioid receptors, or in any treatment wherein temporary suppression of the µ opioid receptor system is desired (e.g., ileus, etc.).

Accordingly, administration of the compounds and compositions disclosed herein can be advantageous for treatment, prevention, amelioration, delay or reduction of side effects of opioid use, such as, for example, gastrointestinal dysfunction (e.g., inhibition of intestinal motility, constipation, GI sphincter constriction, nausea, emesis (vomiting), biliary spasm, opioid bowel dysfunction, colic, dysphoria, pruritus, urinary retention, depression of respiration, papillary constriction, cardiovascular effects, chest wall rigidity and cough suppression, depression of stress response, and immune suppression associated with use of narcotic analgesia, etc., or combinations thereof. Use of a provided compound or composition as disclosed herein can thus be beneficial from a quality of life standpoint for subjects receiving opioids, as well as to reduce complications arising from chronic constipation, such as hemorrhoids, appetite suppression, mucosal breakdown, sepsis, colon cancer risk, and myocardial infarction.

In some embodiments, a provided compound or composition as disclosed herein is useful for administration to a subject receiving acute opioid administration. In some embodiments, a provided compound or composition is useful for administration to subjects suffering from postoperative gastrointestinal dysfunction.

In some embodiments, a provided compound or composition as disclosed herein is useful for administration to subjects receiving chronic opioid administration (e.g., terminally ill subjects receiving opioid therapy such as an AIDS subject, a cancer subject, a cardiovascular subject; subjects receiving chronic opioid therapy for pain management; subjects receiving opioid therapy for maintenance of opioid withdrawal). In some embodiments, the subject is a subject using opioid for chronic pain management. In some embodiments, the subject is a terminally ill subject. In other embodiments the subject is a person receiving opioid withdrawal maintenance therapy. Chronic opioid administration can refer to, or can be characterized by, the need for substantially higher levels of opioid to produce the therapeutic benefit as a result of prior opioid use. Chronic opioid administration can include, for example, daily opioid treatment for a week or more, or intermittent opioid use for at least two weeks.

In some embodiments, a provided compound or composition as disclosed herein can be useful in treating, reducing, inhibiting, or preventing the effects of opioid use including, e.g., aberrant migration or proliferation of endothelial cells (e.g., vascular endothelial cells), increased angiogenesis, and increase in lethal factor production from opportunistic infectious agents (e.g., *Pseudomonas aeruginosa*)*.* Additional advantageous uses of a provided compound or composition include treatment of opioid-induced immune suppression, inhibition of angiogenesis, inhibition of vascular proliferation, treatment of pain, treatment of inflammatory conditions such as inflammatory bowel syndrome, treatment of infectious diseases and diseases of the musculokeletal system such as osteoporosis, arthritis, osteitis, periostitis, myopathies, and treatment of autoimmune diseases.

In some embodiments, a provided compound or composition as disclosed herein can be used in methods for preventing, inhibiting, reducing, delaying, diminishing or treating gastrointestinal dysfunction, including, but not limited to, irritable bowel syndrome, opioid-induced bowel dysfunction, colitis, post-operative or postpartum ileus, nausea and/or vomiting, decreased gastric motility and emptying, inhibition of the stomach, and small and/or large intestinal propulsion, increased amplitude of non-propulsive segmental contractions, constriction of sphincter of Oddi, increased anal sphincter tone, impaired reflex relaxation with rectal distention, diminished gastric, biliary, pancreatic or intestinal secretions, increased absorption of water from bowel contents, gastro-esophageal reflux, gastroparesis, cramping, bloating, abdominal or epigastric pam and discomfort, constipation, idiopathic constipation, post-operative gastrointestinal dysfunction following abdominal surgery (e.g., colectomy (e.g., right hemicolectomy, left hemicolectomy, transverse hemicolectomy, colectomy takedown, low anterior resection», and delayed absorption of orally administered medications or nutritive substances.

Provided forms of a provided compound or composition as disclosed herein are also useful in treatment of conditions including cancers involving angiogenesis, immune suppression, sickle cell anemia, vascular wounds, and retinopathy, treatment of inflammation associated disorders (e.g., irritable bowel syndrome), immune suppression, chronic inflammation.

In still further embodiments, veterinary applications (e.g., treatment of domestic animals, *e.g*., horse, dogs, cats, etc.) of use of a provided compound or composition are provided. Thus, use of provided formulations in veterinary applications analogous to those discussed above for human subjects is contemplated. For example, inhibition of equine gastrointestinal motility, such as colic and constipation, can be fatal to a horse. Resulting pain suffered by the horse with colic can result in a death-inducing shock, while a long-term case of constipation can also cause a horse's death. Treatment of equines with peripheral opioid receptor antagonists has been described, e.g., in U.S. Patent Publication No. 20050124657 published January 20, 2005.

In other embodiments, a provided compound or composition and unit dose forms are useful in preparation of medicaments, including, but not limited to medicaments useful in the treatment of side effects of opioid use (e.g., gastrointestinal side effects (e.g., inhibition of intestinal motility, 01 sphincter constriction, constipation) nausea, emesis, vomiting, dysphoria, pruritus, etc.) or a combination thereof. Compounds and methods of use described herein, and pharmaceutically acceptable compositions and formulations thereof, are useful for preparations of medicaments, useful in treatment of subjects receiving acute opioid therapy (e.g., subjects suffering from post-operative gastrointestinal dysfunction receiving acute opioid administration) or subjects using opioids chronically (e.g., terminally ill subjects receiving opioid therapy such as an AIDS subject, a cancer subject, a cardiovascular subject; subjects receiving chronic opioid therapy for pain management; or subjects receiving opioid therapy for maintenance of opioid withdrawal). Still further, preparation of medicaments useful in the treatment of pain, treatment of inflammatory conditions such as inflammatory bowel syndrome, treatment of infectious diseases, treatment of diseases of the musculokeletal system such as osteoporosis, arthritis, osteitis, periostitis, myopathies, treatment of autoimmune diseases and immune suppression, therapy of post-operative gastrointestinal dysfunction following abdominal surgery (e.g., colectomy (e.g., right hemicolectomy, left hemicolectomy, transverse hemicolectomy, colectomy take down, low anterior resection), idiopathic constipation, and ileus (e.g., post-operative ileus, post-partum ileus), and treatment of disorders such as cancers involving angiogenesis, chronic inflammation and/or chronic pain, sickle cell anemia, vascular wounds, and retinopathy.

In some embodiments, certain subjects having advanced illness while suffering from opioid induced constipation are selected for treatment with compositions of methylnaltrexone.

As used herein, a subject suffering from opioid induced constipation refers to a subject who suffers from constipation resulting from opioid activity, for example, exogenous opioid therapy or endogenous opioid activity. "Constipation" refers to a condition in which a subject suffers from infrequent bowel movements or bowel movements that are painful and/or hard to pass. A subject experiencing constipation often suffers from hard or lumpy stools, straining during bowel movements and/or a sensation of incomplete evacuation following bowel movements. In a particular embodiment, constipation refers to a subject who experiences less than three (3) rescue free bowel movements (RFBMs) per week on average, for example, over the course of the last four consecutive weeks, wherein "rescue free bowel movement" refers to the passage and evacuation of feces, or laxation.

In certain embodiments, the subject does not have a history of chronic constipation prior to the initiation of opioid therapy.

Subjects who are on opioid therapy, who have recently been on opioid therapy or who intend to be on opioid therapy, can be administered the oral compositions of methylnaltrexone. In one embodiment, the subject, at the time of the screening, is on an opioid therapeutic regimen and has been on such regimen for at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 70, 75, 80 85, 90, 95 or 100 days. In a particular embodiment, the subject has been taking opioids for at least one month. In another embodiment, the subject, at the time of the screening, will begin an opioid therapeutic regimen at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 70, 75, 80 85, 90, 95 or 100 days after the screening. In yet another embodiment, the subject, at the time of the screening, will have discontinued opioid therapeutic regimen less than 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 70, 75, 80 85, 90, 95 or 100 days prior to the screening.

The subject can be on an opioid regimen for a variety of purposes. For example, the subject can be a cancer or surgical subject, an immunosuppressed or immuno-compromised subject (including HIV infected subject), a subject with advanced medical illness, a terminally ill subject, a subject with neuropathies, a subject with rheumatoid arthritis, a subject with osteoarthritis, a subject with chronic back pain, a subject with spinal cord injury, a subject with chronic abdominal pain, a subject with chronic pancreatic pain, a subject with pelvic perineal pain, a subject with fibromyalgia, a subject with chronic fatigue syndrome, a subject with migraine or tension headaches, a subject on hemodialysis, or a subject with sickle cell anemia.

In various embodiments, the subject is receiving opioids for alleviation of pain. In a particular embodiment, the subject is receiving opioids for alleviation of chronic non-malignant pain. As used herein, the term "non-malignant pain" refers to pain originating from a non-malignant source such as cancer. In particular embodiments, non-malignant pain includes to back pain, cervical pain, neck pain, fibromyalgia, low extremity pain, hip pain, migraines, headaches, neuropathic pain, or osteoarthritis.

As used herein, the term "chronic" refers to a condition that persists for an extended period of time. In various embodiments, chronic can refer to a condition that lasts at least 1, 2, 3 or 4 weeks. Alternatively, chronic can refer to a condition that lasts at least 1, 2, 3, 4, 5, 6, 7, 8, 9, 10, 11, 12, 18, 24, 30 or 36 months. In a particular embodiment, the subject is receiving opioids for alleviation of chronic non-malignant pain that has persisted for at least 2 months.

In various embodiments, the subject can be on opioid therapy including, but not limited to, alfentanil, anileridine, asimadoline, bremazocine, burprenorphine, butorphanol, codeine, dezocine, diacetylmorphine (heroin), dihydrocodeine, diphenoxylate, ethylmorphine, fedotozine, fentanyl, funaltrexamine, hydrocodone, hydromorphone, levallorphan, levomethadyl acetate, levorphanol, loperamide, meperidine (pethidine), methadone, morphine, morphine-6-glucoronide, nalbuphine, nalorphine, nicomorphine, opium, oxycodone, oxymorphone, papaveretum, pentazocine, propiram, propoxyphene, remifentanyl, sufentanil, tilidine, trimebutine, and/ or tramadol.

Opioids can be administered at a morphine equivalent dosage of: 0.005 to 0.15 mg/kg body weight for intrathecal administration; 0.05 to 1.0 mg/kg body weight for intravenous administration; 0.05 to 1.0 mg/kg body weight for intramuscular administration; 0.05 to 1.0 mg/kg body weight/hour for transmucosal administration. By "morphine equivalent dosage" is meant representative doses of other opioids which equal one milligram of morphine, for example 10 mg meperidine, 1 mg methadone, and 80 µg fentanyl.

In some embodiments, the subject is receiving a daily opioid dose of from about 10 to 300 mg of oral morphine equivalents, or from about 15 to 250 mg of oral morphine equivalents, or from about 20 to 200 mg of oral morphine equivalent, or from about 25 to 100 mg of oral morphine equivalents. In some embodiments, the subject is receiving a daily opioid dose of at least 10, 20, 30, 40, 50, 60, 70, 80, 90, 100, 110, 120, 130, 140, 150, 160, 170, 180, 190, 200, 210, 220, 230, 240, 250, 260, 270, 280, 290 or 300 mg of oral morphine equivalents. In a particular embodiment, the subject is receiving at least 50 mg of oral morphine equivalents. Calculation of oral morphine equivalents is well known in the art. Table A provides a morphine oral equivalence table for known opioids.

**Table A: Morphine Oral Equivalence Table**

| **Drug** | **Route** | **Units** | **Factor for Morphine Equivalents in mgs** |
|---|---|---|---|
| ALFENTANIL | IV | mcg | 0.6 |
| CODEINE | PO | mg | 0.3 |
| CODEINE CONTIN | PO | mg | 0.3 |
| FIORICET WITH CODEINE CAPSULES | PO | mg | 0.3 |
| PANADEINE FORTE | PO | mg | 0.3 |
| PHENERGAN WITH CODEINE | PO | mg | 0.3 |
| TYLENOL W/CODEINE NO. 2 | PO | mg | 0.3 |
| TYLENOL W/CODEINE NO. 3 | PO | mg | 0.3 |
| TYLENOL WITH CODEINE | PO | mg | 0.3 |
| DEMEROL | IM | mg | 1.25 |
| DEMEROL | IV | mg | 1.25 |
| DEMEROL | PO | mg | 0.2 |
| DURAGESIC | TD | mcg/hr | 3.6 |
| FENTANYL | IV | mcg | 0.6 |
| FENTANYL | IV | mg | 600 |
| FENTANYL | PO | mcg | 0.076 |
| FENTANYL CITRATE | PO | mg | 75 |
| FENTANYL CITRATE | PO | mcg | 0.076 |
| FENTANYL | TD | mcg/hr | 3.6 |
| ACETAMINOPHEN W/HYDROCODONE BITARTRATE | PO | mg | 1.8 |
| APAP WITH HYDROCODONE | PO | mg | 1.8 |
| HYCODAN | PO | mg | 1.8 |
| HYDROCODONE | PO | mg | 1.8 |
| LORCET | PO | mg | 1.8 |
| LORTAB | PO | mg | 1.8 |
| TUSSIONEX | PO | mg | 1.8 |
| VICODIN | PO | mg | 1.8 |
| VICODIN ES | PO | mg | 1.8 |
| VICOPROFEN | PO | mg | 1.8 |
| ZYDONE | PO | mg | 1.8 |
| DILAUDID | IV | mg | 40 |
| DILAUDID | PO | mg | 8 |
| HYDROMORPH CONTIN | PO | mg | 8 |
| HYDROMORPHONE | PO | mg | 8 |
| HYDROMORPHONE HYDROCHLORIDE | PO | mg | 8 |
| METHADONE | PO | mg | 3 |
| METHADONE HYDROCHLORIDE | PO | mg | 3 |
| METHADOSE | PO | mg | 3 |
| MORPHINE | IV | mg | 6 |
| MORPHINE | PO | mg | 1 |
| MORPHINE HYDROCHLORIDE | PO | mg | 1 |
| MORPHINE SULFATE | PO | mg | 1 |
| MS CONTIN | PO | mg | 1 |
| MSIR | PO | mg | 1 |
| MSIR | PR | mg | 1 |
| ORAMORPH | PO | mg | 1 |
| STATEX | PO | mg | 1 |
| ACETAMINOPHEN W/OXYCODONE | PO | mg | 2 |
| ENDONE | PO | mg | 2 |
| OXYCOCET | PO | mg | 2 |
| OXYCODONE | PO | mg | 2 |
| OXYCODONE HYDROCHLORIDE | PO | mg | 2 |
| PERCOCET | PO | mg | 2 |
| SUPEUDOL | PO | mg | 2 |
| TYLOX | PO | mg | 2 |
| OXYMORPHONE | IV | mg | 60 |
| OXYMORPHONE | PO | mg | 3 |
| OXYMORPHONE HYDROCHLORIDE | PO | mg | 3 |
| DARVOCET | PO | mg | 0.234 |
| DARVOCET-N | PO | mg | 0.15 |
| DARVON | PO | mg | 0.234 |
| DARVON-N | PO | mg | 0.15 |
| PROPOXYPHENE | PO | mg | 0.234 |
| REMIFENT ANIL | IV | mcg | 0.6 |
| ROXICET | PO | mg | 2 |
| SUFENTANIL | IV | mg | 6000 |
| SUFENTANIL | IV | mcg | 6 |
| TRAMADOL | PO | mg | 0.2 |
| TRAMADOL HYDROCHLORIDE | PO | mg | 0.2 |
| TRAMAL | PO | mg | 0.2 |
| ULTRACET | PO | mg | 0.2 |
| TAPENTADOL | PO | mg | 0.33 |

| | | | |
|---|---|---|---|
| Foley KM. The treatment of cancer pain. N Engl J Med. 1985 Jul, 313(2):84-95 | | | |

The subject's opioid therapeutic regimen can be by any mode of administration. For example, the subject can be taking opioids orally, transdermally, intravenously, or subcutaneously. An example is administration of fentanyl by a transdermal patch, such as, e.g., Duragesic®.

In some embodiments, the subject is not on an opioid regimen. In such embodiments, the subject can be one who has higher than normal systemic baseline levels of endogenous opioids. For example, a cancer patient who does not receive administration of exogenous opioids can benefit from administration of a *mu* opioid receptor antagonist (*e.g.,* prolongation of overall survival). As long as the subject can be identified as a responder by experiencing a bowel movement after administration of a *mu* opioid receptor antagonist as described herein, the subject can receive the benefits of such administration regardless of whether the subject is receives exogenous opioid therapy or experiences higher than normal baseline levels of endogenous opioid activity.

Generally, oral doses of the opioid antagonists, particularly peripheral antagonists, will range from about 0.01 to about 80 mg/kg body weight per day. In some embodiments, the oral dose of opioid antagonists range from about 1 to 20 mg/kg body weight.

In some embodiments, the amount of opioid antagonist that is orally administered ranges from about 1 mg to about 1 g. In some embodiments, the amount of opioid antagonist that is orally administered ranges from about 10 mg to about 600 mg. In some embodiments, the amount of opioid antagonist that is orally administered ranges from about 75 mg to about 900 mg. In some embodiments, the amount of opioid antagonist that is orally administered is about 1 mg, about 10 mg, about 25 mg, about 50 mg, about 75 mg, about 100 mg, about 125 mg, about 150 mg, about 175 mg, about 200 mg, about 250 mg, about 300 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg, about 700 mg, about 725 mg, about 750 mg, about 775 mg, about 800 mg, about 825 mg, about 850 mg, about 875 mg, about 900 mg, about 925 mg, about 950 mg, about 975 mg, or about 1000 mg, or any amount included therein. The opioid antagonist can be administered once every other day, once a day, twice a day, three times a day, four times a day or five times day, or as needed. In some embodiments, the opioid antagonist comprises a compound of Formula I or Formula II. In some embodiments, the opioid antagonist includes a tertiary opioid antagonist. In some embodiments, the opioid antagonist includes a peripherally acting *mu* opioid receptor antagonist (PAMORA). In some embodiments, the opioid antagonist includes a tertiary opioid antagonist and a PAMORA. In some embodiments, the tertiary opioid antagonist is naloxone or naltrexone. In some embodiments, the PAMORA is selected from the group of: naloxegol, alvimopan, axelopran and methylnaltrexone. In some embodiments, the opioid antagonist includes at least one of: naloxone, naltrexone and a PAMORA. In some embodiments, the opioid antagonist includes at least one of: naloxone, naltrexone, naloxegol, alvimopan, axelopran and methylnaltrexone. In some embodiments, the opioid antagonist comprises one or more of naloxone, naltrexone or methylnaltrexone. In some embodiments, the opioid antagonist comprises methylnaltrexone.

Generally, parenteral administration of the opioid antagonist, including intravenous and subcutaneous administration, will range from about 0.001 to about 5 mg/kg body weight. In some embodiments, doses administered intravenously or subcutaneously range from about 0.05 to about 0.5 mg/kg body weight. In some embodiments, doses administered intravenously or subcutaneously range from about 0.075 to about 0.6 mg/kg body weight. In some embodiments, doses administered intravenously or subcutaneously range from about 0.05 to about 0.45 mg/kg body weight. In some embodiments, the opioid antagonist comprises a compound of Formula I or Formula II. In some embodiments, the opioid antagonist includes a tertiary opioid antagonist. In some embodiments, the opioid antagonist includes a peripherally acting *mu* opioid receptor antagonist (PAMORA). In some embodiments, the opioid antagonist includes a tertiary opioid antagonist and a PAMORA. In some embodiments, the tertiary opioid antagonist is naloxone or naltrexone. In some embodiments, the PAMORA is selected from the group of: naloxegol, alvimopan, axelopran and methylnaltrexone. In some embodiments, the opioid antagonist includes at least one of: naloxone, naltrexone and a PAMORA. In some embodiments, the opioid antagonist includes at least one of: naloxone, naltrexone, naloxegol, alvimopan, axelopran and methylnaltrexone. In some embodiments, the opioid antagonist comprises one or more of naloxone, naltrexone or methylnaltrexone. In some embodiments, the opioid antagonist comprises methylnaltrexone.

In some embodiments, doses administered intravenously are about 0.05 mg/kg, about 0.10 mg/kg, about 0.15 mg/kg, about 0.20 mg/kg, about 0.25 mg/kg, about 0.30 mg/kg, about 0.35 mg/kg, about 0.40 mg/kg, about 0.45 mg/kg or about 0.50 mg/kg body weight, or any amount that is included therein. The doses administered intravenously can be administered on a continuous basis. In some embodiments, the intravenous doses can be administered every 6 hours, every 12 hours, every 24 hours or every 48 hours for a period of from about 5 days to about 6 months or more. In some embodiments, the intravenous doses are administered intermittently (*e*.*g*., every 6, 12, 24, or 48 hours) for about 5 days, about 6 days, about 7 days, about 8 days, about 9 days, about 10 days, about 11 days, about 12 days, about 13 days, about 14 days or about 15 days. In some embodiments, the intravenous doses are administered intermittently (*e*.*g*., every 6, 12, 24, or 48 hours) for about 2, 4, 6, 8, 12, or 24 or more weeks. In some embodiments, the intravenous doses are administered intermittently (*e.g.,* every 6, 12, 24, or 48 hours) for about a year or longer.

In some embodiments, doses administered subcutaneously are about 0.005 mg/kg, about 0.01 mg/kg, about 0.015 mg/kg, about 0.025 mg/kg, about 0.05 mg/kg, about 0.075 mg/kg, about 0.1 mg/kg, about 0.15 mg/kg, about 0.2 mg/kg, about 0.25 mg/kg, about 0.3 mg/kg, about 0.4 mg/kg or about 0.45 mg/kg body weight, or any amount that is included therein. The subcutaneous dose can be administered every day or every other day. In some embodiments, the subcutaneous dose is not administered more than once in a 24-hour period. In some embodiments, the subcutaneous dose is administered on an as needed basis. In some embodiments, the subcutaneous dose is administered at least once per week.

Dosages can be adjusted appropriately to achieve desired drug levels, local or systemic, depending on the mode of administration. For example, the dosage for oral administration of the opioid antagonists in an enteric ally coated formulation can be from about 10 to 30% of the non-coated oral dose. In the event that the response in a subject is insufficient of such doses, even higher doses (or effectively higher dosages by a different, more localized delivery route) can be employed to the extent that the subject tolerance permits. Multiple doses per day can be administered to achieve appropriate systemic levels of compounds. Appropriate system levels can be determined by, for example, measurement of the subject's plasma level of the drug using routine HPLC methods known to these of skill in the art.

In some embodiments, methylnaltrexone is administered at a dosage of: 0.001 to 1.0 mg/kg body weight for intravenous administration; 0.001 to 1.0 mg/kg body weight for intramuscular administration; 0.001 to 1.0 mg/kg body weight for transmucosal administration and 0.1 to 40.0 mg/kg body weight for oral administration.

The administration of the opioid antagonist can be commenced prior to administration of the opioid to prevent opioid-induced side effects, including constipation. In some embodiments, administration of the opioid antagonist commences about 5 minutes for parenteral administration and 20 minutes for enteral administration prior to administration of opioids in order to prevent these opioid-induced side effects. While the prevention of symptoms is preferred, in some subjects, such as those chronically on opioids, prevention is not possible. However, administration of the opioid antagonist can also be commenced after the administration of the opioid or after the onset of opioid induced symptoms as a treatment for those symptoms. In some embodiments, the opioid antagonist comprises a compound of Formula I or Formula II. In some embodiments, the opioid antagonist includes a tertiary opioid antagonist. In some embodiments, the opioid antagonist includes a peripherally acting *mu* opioid receptor antagonist (PAMORA). In some embodiments, the opioid antagonist includes a tertiary opioid antagonist and a PAMORA. In some embodiments, the tertiary opioid antagonist is naloxone or naltrexone. In some embodiments, the PAMORA is selected from the group of: naloxegol, alvimopan, axelopran and methylnaltrexone. In some embodiments, the opioid antagonist includes at least one of: naloxone, naltrexone and a PAMORA. In some embodiments, the opioid antagonist includes at least one of: naloxone, naltrexone, naloxegol, alvimopan, axelopran and methylnaltrexone. In some embodiments, the opioid antagonist comprises one or more of naloxone, naltrexone or methylnaltrexone. In some embodiments, the opioid antagonist comprises methylnaltrexone.

Methylnaltrexone is rapidly absorbed after oral administration from the stomach and bowel. Initial plasma levels of the drug are seen within 5-10 minutes of the administration of non-enteric coated compound. Addition of an enteric coating which prevents gastric absorption is associated with lower plasma levels of the methylnaltrexone.

In the description above and below, methylnaltrexone is used as an example of a particularly effective QDNM. It is apparent that other QDNMs can be used as desired, and appropriate dosage can readily be determined empirically by those of skill in the art to account for e.g., variable affinity of the QDNM for opiate receptors, different formulations, etc.

Compositions and formulations can be administered to a subject as required to provide an effective amount of an opioid antagonist. As used above, an "effective amount" of a compound or pharmaceutically acceptable composition can achieve a desired therapeutic and/or prophylactic effect. In some embodiments, an "effective amount" is at least a minimal amount of a compound, or composition containing a compound, which is sufficient for inhibiting the growth of, or reducing the size of, a tumor or cancerous growth. In some embodiments, the term "effective amount," as used in connection with an amount of a *mu* opioid receptor antagonist, a salt thereof, or composition comprising the same, refers to an amount of *mu* opioid receptor antagonist, salt thereof, or composition comprising the same that is sufficient to achieve arrest or attenuation of the abnormal proliferation of cells, *e.g.,* tumor cells, in a subject.

In some embodiments, the compositions as described herein are sufficient to reduce the size of a tumor or cancerous growth by about 2% to about 100%. For example, the compositions can be used to reduce the size of a tumor or cancerous growth the compositions can be used to reduce the size of a tumor or cancerous growth by about 2%, 5%, 10%, 15%, 20%, 25%, 30%, 35%, 40%, 45%, 50%, 55%, 60%, 65%, 70%, 75%, 80%, 85%, 90%, 95% or 100%, or any percentage inclusive within this range.

In some embodiments, the compositions described herein can also be useful for treating or preventing one or more symptoms of opioid-induced constipation. For example, the compositions presented herein can be assessed for treating opioid induced constipation by measuring an increase in the number of rescue free bowel movements experienced by a subject. For example, in some embodiments, the compositions as described herein are sufficient to increase the weekly number of rescue free bowel movements experienced by a subject by at least 1, 2, 3, 4, 5, 6, 7, 8, 9 or 10. In particular embodiments, the compositions as described herein are sufficient to increase the weekly number of rescue free bowel movements experienced by a subject by at least 1. In another embodiment, the compositions as described herein are sufficient to increase the weekly number of rescue free bowel movements experienced by a subject by at least 2. In yet another embodiment, the compositions as described herein are sufficient to increase the weekly number of rescue free bowel movements experienced by a subject by at least 3. In certain embodiments, the compositions as described herein are sufficient to increase the weekly number of rescue free bowel movements experienced by a subject during the first 1, 2, 3, 4, 5, 6, 7, 8, 9 or 10 weeks of dosing. In a particular embodiment, the compositions as described herein are sufficient to increase the weekly number of rescue free bowel movements experienced by a subject by at least 1 during the first 4 weeks of dosing.

In another particular embodiment, the compositions as described herein are sufficient to increase the weekly number of rescue free bowel movements by at least one to at least 3 a week. In yet a further embodiment, the compositions as described herein are sufficient to increase the weekly number of rescue free bowel movements by at least one to at least 3 a week for at least 3 of the first 4 weeks following administration.

The efficacy of the compositions presented herein can be further assessed using various assessment tools available to those skilled in the art to assess treatment of constipation.

In some embodiments, the subject or subject is subcutaneously administered a composition of methylnaltrexone about once a day. In some embodiments, the subject or subject is subcutaneously administered a composition of methylnaltrexone about once every other day. In some embodiments, the subject or subject is subcutaneously administered a composition of methylnaltrexone on an as-needed basis. In some embodiments, the subject or subject is subcutaneously administered a composition of methylnaltrexone on an as-needed basis and at least once per week.

In some embodiments, the subject is subcutaneously administered from about 6 mg to about 15 mg of methylnaltrexone, or a salt thereof, daily or every other day. In some embodiments, the subject is subcutaneously administered from about 8 mg to about 12 mg of methylnaltrexone, or a salt thereof, daily or every other day. For example, the subject can be administered about 6 mg, about 6.25 mg, about 6.5 mg, about 6.75 mg, about 7 mg, about 7.25 mg, about 7.5 mg, about 7.75 mg, about 8 mg, about 8.25 mg, about 8.5 mg, about 8.75 mg, about 9 mg, about 9.25 mg, about 9.5 mg, about 9.75 mg, about 10 mg, about 10.25 mg, about 10.5 mg, about 10.75 mg, about 11 mg, about 11.25 mg, about 11.5 mg about 11.75 mg, about 12 mg, about 12.25 mg, about 12.5 mg, about 12.75 mg, about 13 mg, about 13.25 mg, about 13.5 mg, about 13.75 mg, about 14 mg, about 14.25 mg, about 14.5 mg, about 14.75 mg, about 15 mg, or any other amount included therein, of methylnaltrexone, or salt thereof, daily or every other day. In some embodiments, the subject is subcutaneously administered about 8 mg of methylnaltrexone, or a salt thereof, daily or every other day. In some embodiments, the subject is subcutaneously administered about 12 mg of methylnaltrexone, or a salt thereof, daily or every other day. In some embodiments, the subject is subcutaneously administered 8 mg or 12 mg of methylnaltrexone, or a salt thereof, every other day, as needed, but not more frequently than once in a 24-hour period.

In some embodiments, the subject is subcutaneously administered methylnaltrexone or a salt thereof, at a dose of between about 0.05 mg/kg to about 0.45 mg/kg body weight daily or every other day. In some embodiments, the subject is subcutaneously administered methylnaltrexone or a salt thereof, at a dose of between about 0.10 mg/kg to about 0.30 mg/kg body weight daily or every other day. For example, the subject can be administered methylnaltrexone, or a salt thereof, at a dose of about 0.05 mg/kg, about 0.10 mg/kg, about 0.15 mg/kg, about 0.20 mg/kg, about 0.25 mg/kg, about 0.30 mg/kg, about 0.35 mg/kg, about 0.40 mg/kg, about 0.45 mg/kg body weight, or any amount included therein, daily or every other day. In some embodiments, the subject is administered methylnaltrexone, or a salt thereof, at a dose of about 0.15 mg/kg body weight daily or every other day.

In some embodiments, the subject is subcutaneously administered methylnaltrexone, or a salt thereof, at a dose of between about 0.05 mg/kg to about 0.45 mg/kg body weight every other day, as needed, but not more frequently than once in a 24-hour period. In some embodiments, the subject is subcutaneously administered methylnaltrexone, or a salt thereof, methylnaltrexone or a salt thereof, at a dose of between about 0.10 mg/kg to about 0.30 mg/kg body weight every other day, as needed, but not more frequently than once in a 24-hour period. In some embodiments, the subject is subcutaneously administered methylnaltrexone, or a salt thereof, at a dose of 0.15 mg/kg body weight every other day, as needed, but not more frequently than once in a 24-hour period.

In some embodiments, the subject is intravenously administered methylnaltrexone, or a salt thereof, at a dose of from about 0.05 mg/kg to about 0.50 mg/kg body weight. For example, the subject can be administered an intravenous dose of methylnaltrexone, or a salt thereof, of about 0.05 mg/kg, about 0.10 mg/kg, about 0.15 mg/kg, about 0.20 mg/kg, about 0.25 mg/kg, about 0.30 mg/kg, about 0.35 mg/kg, about 0.40 mg/kg, about 0.45 mg/kg or about 0.50 mg/kg body weight, or any amount that is included therein. The intravenous dose can be administered on a continuous or intermittent basis. In some embodiments, the intravenous dose is administered continuously over a period of from about 1 minute to about 30 minutes. For example, the intravenous dose can be administered continuously over a period of about 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 minutes, and the dose can be administered once a day or once every other day, or on an as-needed basis (PRN). In some embodiments, the intravenous dose can be administered every 6 hours, every 12 hours, every 24 hours or every 48 hours for a period of from about 5 days up to about 6 months or more. In some embodiments, the intravenous dose is administered every 6 hours, every 12 hours, every 24 hours or every 48 hours for a period of about 5 days, about 7 days, about 10 days, about 14 days, about 21 days, about 28 days, about 4 weeks, about 6 weeks, about 8 weeks, about 12 weeks, about 16 weeks, about 20 weeks or about 24 weeks or longer. In some embodiments, the intravenous dose is administered for a period of a year or more.

In some embodiments, the subject or subject is orally administered a composition of methylnaltrexone at least once a day. In some embodiments, the subject or subject is administered an oral composition of methylnaltrexone at least once, twice, three, four or five times a day. In some embodiments, the subject or subject is administered an oral composition of methylnaltrexone three times a day.

In some embodiments, the subject is orally administered a total daily dose of from about 25 mg to about 600 mg, from about 50 mg to about 450 mg, or from about 100 mg to about 300 mg of methylnaltrexone, or a salt thereof. For example, the subject can be orally administered a total daily dose of methylnaltrexone, or a salt thereof, of about 25 mg, 30 mg, 35 mg, 40 mg, 45 mg, 50 mg, 55 mg, 60 mg, 65 mg, 70 mg, 75 mg, 80 mg, 85 mg, 90 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, or any amount inclusive within this range.

In some embodiments, the subject is orally administered a total dose of from about 25 mg to about 600 mg, from about 50 mg to about 450 mg, or from about 100 mg to about 300 mg of methylnaltrexone, or a salt thereof, wherein the total dose is administered every other day or on an as needed basis (PRN). For example, the subject can be orally administered a total dose of methylnaltrexone, or a salt thereof, of about 25 mg, 30 mg, 35 mg, 40 mg, 45 mg, 50 mg, 55 mg, 60 mg, 65 mg, 70 mg, 75 mg, 80 mg, 85 mg, 90 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, or any amount inclusive within this range, wherein the total dose is administered every other day or on an as needed basis.

In various embodiments, the subject is orally administered 150 mg of methylnaltrexone, or a salt thereof, daily. For example, the subject can be administered a tablet comprising 150 mg of methylnaltrexone or a salt thereof, daily. In another embodiment, the subject is orally administered 300 mg of methylnaltrexone or a salt thereof, daily. For example, the subject can be administered two tablets, each comprising 150 mg of methylnaltrexone or a salt thereof, daily. In yet another embodiment, the subject is orally administered 450 mg of methylnaltrexone or a salt thereof, daily. For example, the subject can be administered three tablets, each comprising 150 mg of methylnaltrexone or a salt thereof, daily.

In some embodiments, the subject is also administered an anti-tumor or anti-cancer therapy that does not comprise a *mu* opioid receptor antagonist. The anti-tumor or anti-cancer therapy can include, for example, a chemotherapeutic agent, radiation therapy (or radiotherapy), an anti-angiogenic agent and/or surgery. The anti-tumor or anti-cancer therapy can be administered simultaneously, sequentially or separately in combination with the compositions disclosed herein.

The chemotherapeutic agent can be a taxane or platinum drug known for use in treating cancer. In some embodiments, the chemotherapeutic drug is at least one selected from the group consisting of paclitaxel, gemcitabine, nab-paclitaxel, 5-fluorouracil, oxaliplatin, irinotecan, dasatinib, bevacizumab and combinations thereof. In some embodiments, the chemotherapeutic drug includes an MEK inhibitor, a PI3K inhibitor, a Hedgehog inhibitor, a Wnt inhibitor, or a combination thereof. In some embodiments, the drug includes an agent that interferes with the mTOR or NfKb pathways. The chemotherapeutic drug can be selected from new classes of therapies, such as siRNA-Alnylam type therapies. In some embodiments, the chemotherapeutic agent can be at least one selected from the group of: cisplatin, carboplatin, nedaplatin, oxaliplatin, satraplatin, triplatin tetranitrate, gemcitabine, topotecan hydrochloride, doxorubicin, pegylated doxorubicin, and taxol.

In some embodiments, the chemotherapeutic agent is at least one selected from the group of: a cytotoxic and/or cytostatic agents, an immunological modifier (e.g. interferons and interleukins), an MEK inhibitor, an anti-progestogen, a cytokine, folic acid and a vitamin. The MEK inhibitor can be any MEK inhibitor, such as, but not limited to PD184325 (CI-1040, N-(3-chloro-4-fluorophenyl)-7-methoxy-6-(3-morpholin-4-ylpropoxy)quinazol- in-4-amine), PD0325901 (N-[(2R)-2,3-dihydroxypropoxy]-3,4-difluoro-2-[(2-fluoro-4-iodophenyl)ami- no]-benzamide), PD98059 (2'-amino-3'-methoxyflavone) and U0126 (1,4-diamino-2,3-dicyano-1,4-bis(aminophenylthio) butadiene). Ccytotoxic agents encompass any agent used for the treatment of abnormal and uncontrolled progressive cellular growth. Non-limiting examples of cytotoxic agents include the alkylating agents cyclophosphamide (CTX)(Bristol-Meyers Squibb), ifosfamide (Bristol-Meyers Squibb), chlorambucil (Glaxo Wellcome), and carmustine (Bristol-Meyers Squibb); the anti-metabolites cytarabine (Pharmacia & Upjohn), 6-mercaptopurine (Glaxo Wellcome), 6-thioguanine (Glaxo Wellcome), and methotrexate (Immunex); the antibiotics doxorubicin (Pharmacia & Upjohn), daunorubicin (NeXstar), and mitoxantrone (Immunex); and miscellaneous agents such as vincristine (Lilly), vinblastine (Lilly), and paclitaxel (Bristol-Meyers Squibb) or their pharmaceutically acceptable salts.

In some embodiments, the anti-tumor or anti-cancer therapy comprises administration of one or more agents are selected from an alkylating agent, including, but not limited to, adozelesin, altretamine, bendamustine, bizelesin, busulfan, carboplatin, carboquone, carmofur, carmustine, chlorambucil, cisplatin, cyclophosphamide, dacarbazine, estramustine, etoglucid, fotemustine, hepsulfam, ifosfamide, improsulfan, irofulven, lomustine, mannosulfan, mechlorethamine, melphalan, mitobronitol, nedaplatin, nimustine, oxaliplatin, piposulfan, prednimustine, procarbazine, ranimustinc, satraplatin, semustine, streptozocin, temozolomide, thiotepa, treosulfan, triaziquone, triethylenemelamine, triplatin tetranitrate, trofosphamide, and uramustine; an antibiotic, including, but not limited to, aclarubicin, amrubicin, bleomycin, dactinomycin, daunorubicin, doxorubicin, elsamitrucin, epirubicin, idarubicin, menogaril, mitomycin, neocarzinostatin, pentostatin, pirarubicin, plicamycin, valrubicin, and zorubicin; an antimetabolite, including, but not limited to, aminopterin, azacitidine, azathioprine, capecitabine, cladribine, clofarabine, cytarabine, decitabine, floxuridine, fludarabine, 5-fluorouracil, gemcitabine, hydroxyurea, mercaptopurine, methotrexate, nelarabine, pemetrexed, azathioprine, raltitrexed, tegafur-uracil, thioguanine, trimethoprim, trimetrexate, and vidarabine; an immunotherapy, including, but not limited to, alemtuzumab, bevacizumab, cetuximab, galiximab, gemtuzumab, panitumumab, pertuzumab, rituximab, tositumomab, trastuzumab, 90 Y ibritumomab tiuxetan, ipilimumab, and tremelimumab; a hormone or hormone antagonist, including, but not limited to, anastrozole, androgens, buserelin, diethylstilbestrol, exemestane, flutamide, fulvestrant, goserelin, idoxifene, letrozole, leuprolide, magestrol, raloxifene, tamoxifen, and toremifene; a taxane, including, but not limited to, DJ-927, docetaxel, TPI 287, larotaxel, ortataxel, paclitaxel, DHA-paclitaxel, and tesetaxel; a retinoid, including, but not limited to, alitretinoin, bexarotene, fenretinide, isotretinoin, and tretinoin; an alkaloid, including, but not limited to, demecolcine, homoharringtonine, vinblastine, vincristine, vindesine, vinflunine, and vinorelbine; an antiangiogenic agent, including, but not limited to, AE-941 (GW786034, Neovastat), ABT-510, 2-methoxyestradiol, lenalidomide, and thalidomide; a topoisomerase inhibitor, including, but not limited to, amsacrine, belotecan, edotecarin, etoposide, etoposide phosphate, exatecan, irinotecan (also active metabolite SN-38 (7-ethyl-10-hydroxy-camptothecin)), lucanthone, mitoxantrone, pixantrone, rubitecan, teniposide, topotecan, and 9-aminocamptothecin; a kinase inhibitor, including, but not limited to, axitinib (AG 013736), dasatinib (BMS 354825), erlotinib, gefitinib, flavopiridol, imatinib mesylate, lapatinib, motesanib diphosphate (AMG 706), nilotinib (AMN107), seliciclib, sorafenib, sunitinib malate, AEE-788, BMS-599626, UCN-01 (7-hydroxystaurosporine), and vatalanib; a targeted signal transduction inhibitor including, but not limited to bortezomib, geldanamycin, and rapamycin; a biological response modifier, including, but not limited to, imiquimod, interferon-.alpha., and interleukin-2; and other chemotherapeutics, including, but not limited to 3-AP (3-amino-2-carboxyaldehyde thiosemicarbazone), altrasentan, aminoglutethimide, anagrelide, asparaginase, bryostatin-1, cilengitide, elesclomol, eribulin mesylate (E7389), ixabepilone, lonidamine, masoprocol, mitoguanazone, oblimersen, sulindac, testolactone, tiazofurin, mTOR inhibitors (e.g. temsirolimus, everolimus, deforolimus), PI3K inhibitors (e.g. BEZ235, GDC-0941, XL147, XL765), Cdk4 inhibitors (e.g. PD-332991), Akt inhibitors, Hsp90 inhibitors (e.g. tanespimycin) and farnesyltransferase inhibitors (e.g. tipifarnib); MEK inhibitors (e.g., AS703026, AZD6244 (selumetinib), AZD8330, BIX02188, C11040 (PD184352), D-87503, GSK1120212 (JTP-74057), PD0325901, PD318088, PD98059, PDEA119 (BAY 869766), TAK-733). Preferably, the method of treating a cancer involves administering to the subject an effective amount of a composition including any one or more compound(s) as described herein in combination with a chemotherapeutic agent selected from capecitabine, 5-fluorouracil, carboplatin, dacarbazine, gefitinib, oxaliplatin, paclitaxel, SN-38, temozolomide, vinblastine, bevacizumab, cetuximab, interferon-.alpha., interleukin-2, or erlotinib.

### EXAMPLES

### EXAMPLE 1: STUDY 1

A double-blind, randomized, placebo-controlled study of subcutaneous methylnaltrexone (MNTX) was conducted in subjects with advanced medical illness and a current history of opioid-induced constipation. To satisfy eligibility criteria for enrollment, subjects were required to be on stable doses of laxatives and opioid analgesics. In the context of the study, a stable dose of opioid analgesics was defined as no reduction in the dose of opioid analgesic of ≥ 50% for at least three days before the first dose of MNTX. A stable dose of laxatives was defined as having a standing laxative order (and not PRN, or as-needed) for at least three days before the first dose of MNTX. A diagnosis of constipation was provided if the subject had fewer than 3 bowel movements during the previous week by history and no clinically notable laxation in the 24 hours before the first dose of MNTX, or no clinical notable laxation in the 48 hours before the first dose of MNTX.

Eligible subjects were randomized to one of two treatment groups: MNTX 0.15 mg/kg *(e.g.,* 8 mg for patients < 62 kg body weight and 12 mg for patients ≥ 62 kg body weight) or placebo. Each treatment was administered every other day (QOD) subcutaneously for two weeks. Injections were administered subcutaneously in either the shoulder area, buttocks, abdomen, thighs or extremities. Rescue-laxatives, enemas or manual disimpactions were not administered within 4 hours before or after each dose of MNTX. Endpoints included: (1) the proportion of subjects with rescue-free laxation within 4 hours of the first dose of study drug, and (2) the proportion of subjects with ≥ 2 rescue-free laxations within 4 hours post dosing over 4 doses of study drug (the first week of the two-week treatment period). One hundred and thirty-four (134) subjects were enrolled and evaluated, including 78 patients with advanced cancers and 56 patients with other advanced illness, including COPD, cardiovascular disease and severe neurologic disease. Seventy-one (71) of the 134 subjects were randomized to the placebo group, and sixty-two (62) of the 134 subjects were randomized to MNTX treatment group. The co-primary efficacy endpoints of this study were: 1) the proportion of patients with laxation within 4 hours after the first dose of study drug, and 2) the proportion of patients with laxation within 4 hours after 2 of the first 4 doses (the first week of double-blind treatment). The primary endpoint was laxation within 4 hours. Secondary endpoints included the use of rescue medication and frequency of symptoms within 24 hours.

Rescue-free laxation occurred within 4 hours after the first dose in 30 (48.4%) MNTX-treated subjects and 11 (15.5%) placebo-treated subjects (p<0.0001). Thirty-two (51.6%) MNTX-treated subjects and 6 (8.5%) placebo-treated subjects had a rescue-free laxation within 4 hours after at least 2 of the first 4 doses (p<0.0001).

In addition, twenty-four (38.7%) MNTX-treated subjects and 4 (5.6%) placebo-treated subjects had a rescue-free laxation within 4 hours after at least 4 of the maximum 7 doses (p<0.0001). After administration of any individual dose, higher percentages of subjects in the MNTX group (range, 37.3-48.4%) than in the placebo group (range, 6.8-15.5%) had rescue-free laxations within 4 hours. Likewise, higher percentages of subjects in the MNTX group (range, 55.4-66.0%) than in the placebo group (range, 28.6-39.2%) had rescue-free laxations within 24 hours after administration of any individual dose.

The median time to rescue-free laxation after the first dose was 1.0 hour in the MNTX group and 11.2 hours in the placebo group, based on the subjects who had laxation. Following subsequent doses, the median time to laxation ranged from 1.1 to 2.6 hours in the MNTX group and 7.2 to 22.0 hours in the placebo group.

At least three rescue-free laxations per week, regardless of the time after dose administration, occurred in 42 (67.7%) MNTX-treated subjects and 32 (45.1%) placebo-treated subjects (p=0.0087). Higher percentages of MNTX-treated subjects than placebo-treated subjects had at least one rescue-free laxation at some time during the first week of treatment (95.2% and 83.1%, respectively) and during the second week of treatment (91.2% and 81.0%, respectively).

Focusing on the rescue-free laxations that occurred within 4 hours after any dose, a higher percentage were rated as having moderate, considerable, or great difficulty in the placebo group (50.0%) than in the MNTX group (33.0%). Similar percentages in the two groups (16.7% and 15.9%, respectively) experienced watery bowel movements. Higher percentages of subjects in the MNTX group than in the placebo group had improvement in constipation distress on Day 1 (53% versus 30%), Day 7 (64% versus 52%), and Day 14 (60% versus 54%).

Global rating scales showed that higher percentages of subjects in the MNTX group than in the placebo group rated their bowel status improved on Day 7 (73.5% versus 35.1%) and Day 14 (67.9% versus 44.6%). Likewise, the bowel status was improved for higher percentages of subjects in the MNTX group than in the placebo group on Day 7 (69.4% versus 35.1%) and Day 14 (67.9% versus 50.0%).

Baseline use of laxatives was comparable in the groups. The percentage of subjects using enemas increased during double-blind treatment in both groups, but the magnitude of the increase was greater in the placebo group (from 14.1% at baseline to 35.2% during the study) than in the MNTX group (from 12.7% to 23.8%). There was also a larger increase in the use of osmotic agents in the placebo group (from 33.8% at baseline to 40.8% during the study) than in the MNTX group (from 30.2% to 33.3%).

Of the patients who completed the double-blind phase of the study, 80 (placebo 39, MNTX 41) elected to proceed with a 12-week open-label phase, during which they could receive one dose of MNTX (0.15 mg/kg subcutaneously, which could be reduced to 0.075 mg/kg or increased to 0.3 mg/kg) as often as every 24 hours PRN. Patients received 30-days follow-up after the last study dose.

### EXAMPLE 2: STUDY 2

A double-blind, randomized, placebo-controlled study of subcutaneous MNTX was conducted in subjects with advanced medical illness and a current history of opioid-induced constipation. To satisfy eligibility criteria for enrollment, subjects were required to be on stable doses of laxatives and opioid analgesics. In the context of the study, a stable dose of opioid analgesics was defined as no reduction in the dose of opioid analgesic of ≥ 50% for at least three days before the first dose of MNTX. A stable dose of laxatives was defined as having a standing laxative order (and not PRN, or as-needed) for at least three days before the first dose of MNTX. A diagnosis of constipation was provided if the subject had (a) fewer than 3 bowel movements during the previous week by history and no clinically notable laxation in the 24 hours before the first dose of MNTX, or (b) no clinical notable laxation in the 48 hours before the first dose of MNTX.

Eligible subjects were randomized to receive MNTX or placebo every other day during a 14-day, double-blind period. Subjects weighing 38 kg to < 62 kg received 0.4 mL SC MNTX (8 mg) or equal volume of placebo. Subjects weighing ≥ 62 kg received 0.6 mL SC MNTX (12 mg) or equal volume of placebo. Injections were administered subcutaneously in either the shoulder area, buttocks, abdomen, thighs or extremities, and rescue-laxatives, enemas or manual disimpactions were not administered within 4 hours before or after each dose of test article. Subjects who did not continue into an open-label extension phase of the study had a follow-up visit 15 to 21 days after their last dose of test article.

The endpoint assessed was the proportion of subjects with rescue-free laxation response within 4 hours after at least two of the first four doses. Two hundred and twenty-nine (229) subjects were enrolled and evaluated, including 151 patients with advanced cancers and 78 patients with other advanced illness (*e*.*g*., cardiovascular, pulmonary or neurologic disorders). Of which 229 subjects which enrolled, 113 were randomized to the placebo group and 116 were randomized to the MNTX treatment group. Of the patients who completed the double-blind phase, 142 (placebo 69, MNTX 73) elected to proceed with a 10-week open label phase of subcutaneous MNTX at PRN dosing.

The results indicated that fixed doses of MNTX SC demonstrated robust efficacy in the treatment of OIC in subjects with advanced illness. Methylnaltrexone was statistically superior to placebo for the primary and all secondary efficacy endpoints.

For the endpoint, the proportion of subjects who had a rescue-free laxation response (RFLR) within 4 hours after at least 2 of the first 4 doses, highly statistically significant improvements were observed for the MNTX group compared with the placebo group in the intent-to-treat (ITT) population (p < 0.0001). The mean proportions of subjects with RFLRs within 4 hours of at least 2 of the first 4 doses in the first week of treatment were 62.9% (95% CI = 53.5%, 71.7%) in the MNTX group and 9.6% in the placebo group (95% CI = 4.9%, 16.6%). The endpoint results showed significant improvements compared with placebo across subgroups defined by demographic and baseline characteristics.

In addition, subjects in the MNTX group had significantly shorter median times to first rescue-free laxation after the first dose than subjects in the placebo group (0.8 hours vs 23.6 hours, p < 0.0001). Significantly larger proportions of MNTX-treated subjects had rescue-free laxations within 4 hours and within 24 hours following the first dose of study drug when compared with placebo-treated subjects. The mean proportions of rescue-free laxations within 4 hours of the first dose were 69.8% (95% CI = 60.6%, 78.0%) in the MNTX group and 17.5% (95% CI = 11.1%, 25.8%) in the placebo group. Furthermore, subjects in the MNTX group had significantly shorter median times to first rescue-free laxation within 24 hours after each dose (up to a maximum of 7 doses) than subjects in the placebo group.

The proportion of subjects with rescue-free laxation within the first 4 hours after the first dose was significantly greater in the MNTX group compared with the placebo group (p < 0.0001; 69.8% (95% CI = 60.6%, 78.0%) in the MNTX group, 17.5% (95% CI = 11.1%, 25.8%) in the placebo group). Statistically significant improvements were also observed for the MNTX group compared with the placebo group with respect to the proportion of subjects with rescue-free laxation within 4 hours or within 24 hours after the each dose in the ITT population. The proportion of subjects with rescue-free laxation within 4 hours after at least 4 of the maximum 7 doses was significantly higher for the MNTX group compared with the placebo group (p < 0.0001, 62.2% (95% CI = 51.4%, 72.2%) in the MNTX group, 4.9% (95% CI = 1.3%, 12.0%) in the placebo group).

Differences in the number of total laxations within 24 hour post dosing per week in the MNTX group versus the placebo group were statistically significant in favor of MNTX during both Weeks 1 (p < 0.0001, MNTX 4.9 [95% CI = 4.3, 5.6], placebo 3.0 [95% CI = 2.3, 3.7]) and 2 (p = 0.0083, MNTX 3.2 [95% CI = 2.7, 3.7], placebo 2.2 [95% CI = 1.7, 2.8]) as were the number of rescue-free laxations (Week 1, p < 0.0001, MNTX 4.9 [95% CI = 4.2, 5.6], placebo 2.7 [95% CI = 2.0, 3.4]; Week 2, p = 0.0024, MNTX 3.2 [95% CI = 2.6, 3.7], placebo 2.0 [95% CI = 1.5, 2.5]).

A significantly smaller number of subjects in the MNTX group used rescue laxatives during the double-blind period compared with subjects in the placebo group (p = 0.0020, 27% (95% CI = 19%, 36%) in the MNTX group and 40% in the placebo group (95% CI = 31%, 50%)). In addition, a significantly smaller number of subjects in the MNTX group used enemas during the double-blind period compared with subjects in the placebo group (p = 0.0003, 17% (95% CI = 11%, 25%) in the MNTX group and 32% in the placebo group (95% CI = 23%, 41%)). Few subjects (≤ 5%) in either group required manual disimpaction during the double-blind period.

Improvement in the stool symptoms domain of the PAC-SYM was more pronounced in the MNTX group compared with the placebo group with a trend toward statistical significance (p = 0.0920).

In conclusion, methylnaltrexone SC administered every other day at a fixed dose demonstrated robust efficacy in the treatment of OIC in subjects with advanced illness. There were statistically superior efficacy findings for MNTX compared with placebo for the primary and all secondary endpoints. The efficacy results in this study using a fixed dose of MNTX were consistent with those observed in the Study 1 (Example 1), which used weight-based (mg/kg) dosing. The effectiveness of MNTX led to shorter median times to rescue-free laxations compared with placebo as well as a larger proportion of subjects with rescue-free laxations compared with placebo. Also, significantly smaller numbers of subjects in the MNTX group used rescue laxatives and enemas compared with subjects in the placebo group.

Opioid use was not affected by MNTX administration, thereby showing clinical evidence of peripheral-restricted opiate receptor antagonism by MNTX without effects on central nervous system opiate receptors.

### EXAMPLE 3

Methylnaltrexone (MNTX) is approved for the treatment of opioid-induced constipation (OIC) in subjects with advanced illness who are receiving palliative care when response to laxative therapy has not been sufficient. Because MNTX has restricted passage through the blood brain barrier, it can be given to subjects with cancer who are receiving opioid therapy without affecting analgesia. Recent cellular, molecular, animal, and human data suggest that the mu opioid receptor (MOR) may be a target for chemotherapeutic agents. It has been suggested that MNTX might attenuate cancer progression. In animal models, mu opiate receptor (MOR) antagonists in clinically relevant doses reduced tumor growth in lung, head and neck, breast, and pancreatic tumors. MOR knockout mice showed decreased tumor growth and metastasis in lung cancer and melanoma. Infusion of MNTX dramatically reduced growth and metastasis in the Lewis lung cancer model. Additionally, polymorphisms in the MOR which confer opioid resistance show a significantly improved survival at all stages of human breast cancer. Further, opioid use has been confirmed as an important co-factor in survival in advanced prostate cancer, and two recent retrospective studies demonstrated that perioperative opiate use is associated with decreased overall survival and increased recurrence in subjects undergoing surgery for stage 1 lung cancer. Herein it is shown that peripheral antagonism of opioid-mediated effects can attenuate tumor disease progression in cancer subjects. Accordingly, pooled data from two randomized, placebo-controlled trials (Examples 1 and 2) were evaluated to identify whether MNTX given at regular clinical doses could influence the progression of cancer.

As part of the routine adverse event (AE) evaluations filled out by all investigators in the studies described in Examples 1 and 2, AEs of tumor progression were tabulated. Both studies were randomized, placebo-controlled trials that involved a 2-week treatment period followed by a 2-week follow-up period. The enrolled subjects were advanced illness (incurable cancer or other end-stage disease) subjects with OIC who were receiving stable doses of laxatives and opioid analgesics. In the context of the study, a stable dose of opioid analgesics was defined as no reduction in the dose of opioid analgesic of ≥ 50% for at least three days before the first dose of MNTX. A stable dose of laxatives was defined as having a standing laxative order (and not PRN, or as-needed) for at least three days before the first dose of MNTX. A diagnosis of constipation was provided if the subject had (a) fewer than 3 bowel movements during the previous week by history and no clinically notable laxation in the 24 hours before the first dose of MNTX, or (b) no clinical notable laxation in the 48 hours before the first dose of MNTX. In Study 1 (Example 1), subjects received subcutaneous MNTX 0.15 mg/kg or placebo (PBO) every other day (QOD), and in Study 2 (Example 2) subjects received subcutaneous MNTX (8 mg or 12 mg based on body weight 38 to <62 kg or ≥62 kg, respectively) or placebo administered QOD. The 12 mg fixed dose was designed to correspond to the 0.15 mg/kg weight-based dose. Rescue-laxatives, enemas or manual disimpactions were not administered within 4 hours before or after each dose of MNTX. For the purpose of this analysis, only subjects with a cancer-related diagnosis were considered. For statistical significance testing, the Fisher's exact test was used (p<0.05).

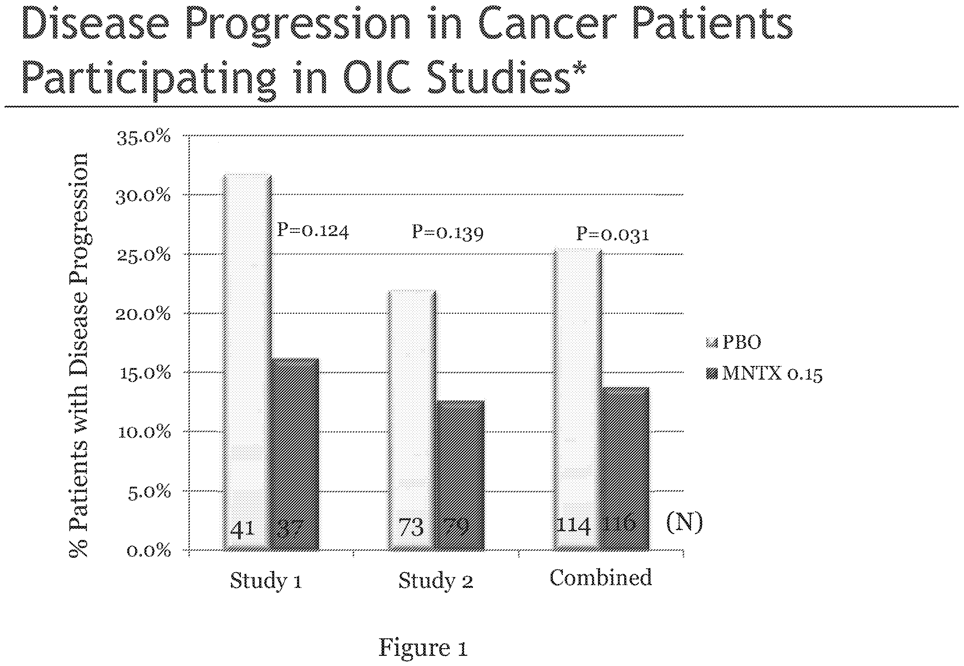

In an initial pooled analysis of the two studies, of 370 subjects randomized across the 2 studies, 230 (62%) had a cancer-related diagnosis, of which 116 and 114 were randomized to MNTX and PBO, respectively. Table 1 and Figure 1 provide AEs of disease progression in the pooled analysis and for each individual study during the double-blind phase of the studies. In the analysis, AEs of disease progression were reported for 16 of 116 (13.8%) MNTX-treated subjects compared to 29 of 114 (25.4%) PBO-treated subjects (p = 0.031), corresponding to an approximate 46% reduction in the incidence of this AE.

**Table 1. Treatment-emergent AEs of Disease Progression (Double-blind Phase)**

| **Adverse Events of Disease Progression, n/N (%)** | | | |
|---|---|---|---|
| **Study** | **PBO** | **MNTX** | **P-Value** |
| Study 1 | 13/41 (31.7%) | 6/37 (16.2%) | 0.1238 |
| Study 2 | 16/73 (21.9%) | 10/79 (12.7%) | 0.1387 |
| Combined (Study 1 & Study 2) | 29/114 (25.4%) | 16/116 (13.8%) | 0.0308 |

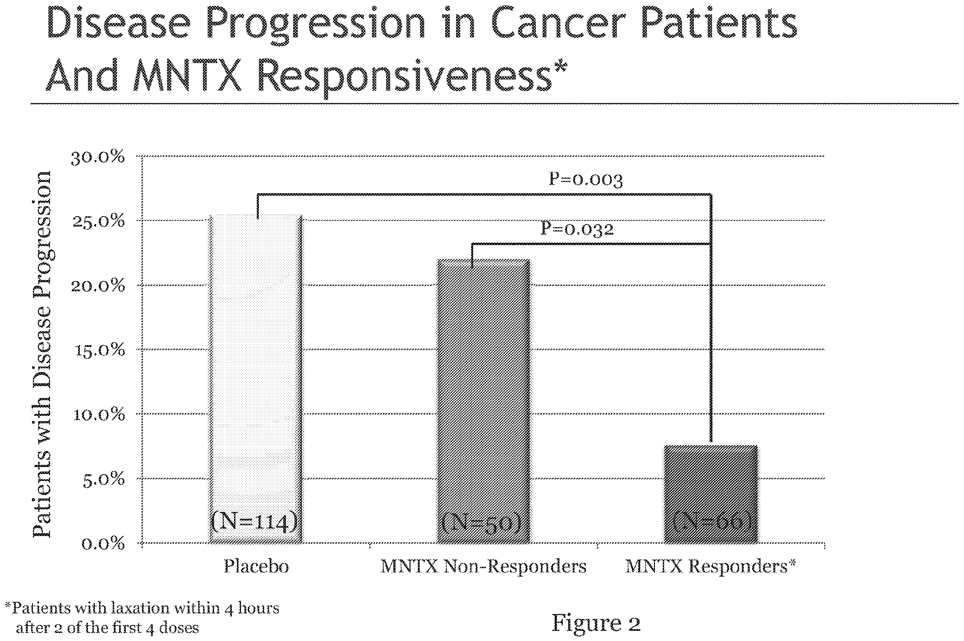

Furthermore, when the pool of MNTX-treated subjects across both studies was examined, there was a significant decrease in the percentage of subjects with disease progression between subjects who responded to MNTX treatment and those who were considered non-responders. A subject with laxation within 4 hours after two of the first four doses of MNTX was considered a responder. As illustrated in Table 2 and Figure 2, disease progression was observed in a significantly lower percentage of subjects in the MNTX responder group relative to subjects in the MNTX non-responder group.

**Table 2. Treatment-emergent AEs of Disease Progression (Double-blind Phase)**

| **Adverse Events of Disease Progression** | | | | |
|---|---|---|---|---|
| | **PBO** | **MNTX Responders** | **MNTX Nom-Responders** | **MNTX Total** |
| Disease Progression | 29 | 5 | 11 | 16 |
| No Disease Progression | 85 | 61 | 39 | 100 |
| Total | 114 | 66 | 50 | 116 |
| % Progression | 25.4% | 7.6% | 22.0% | 13.8% |

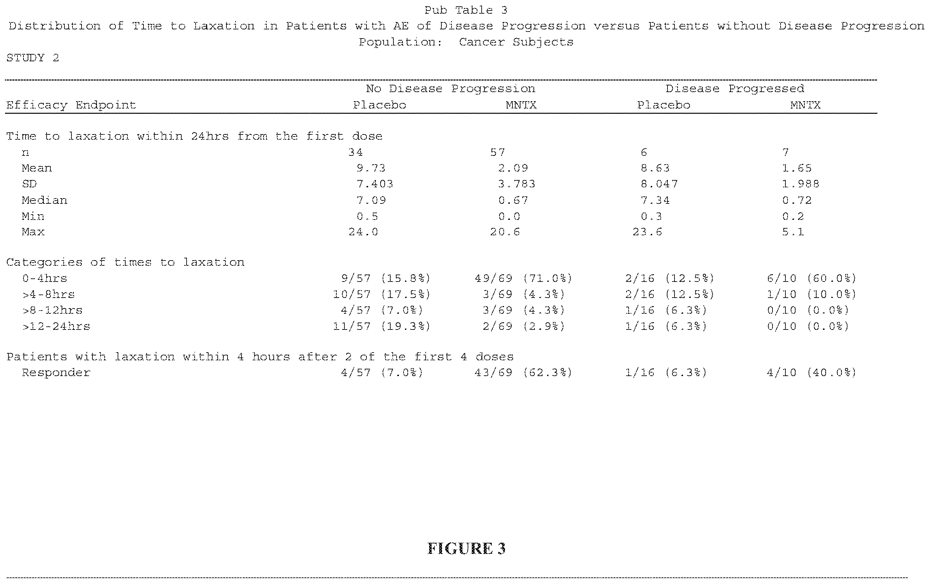

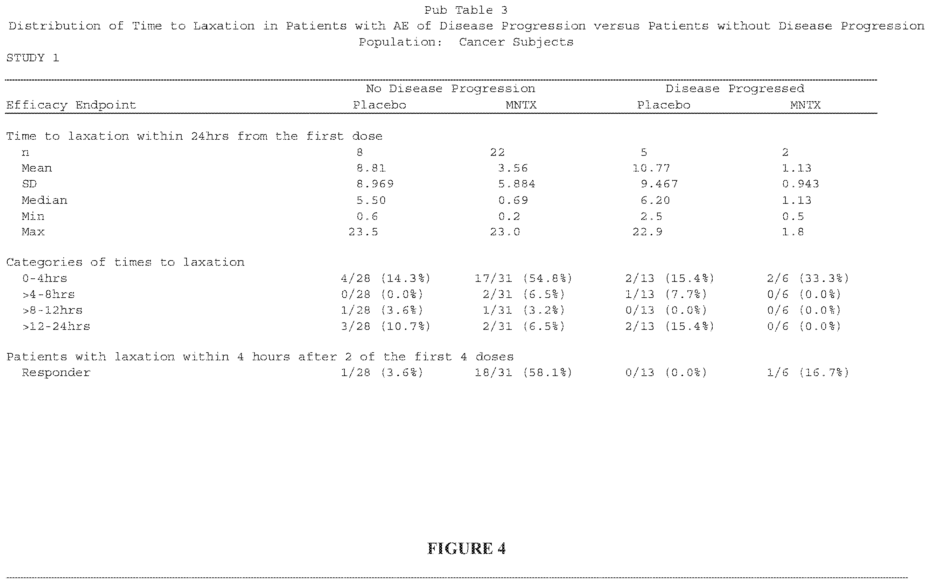

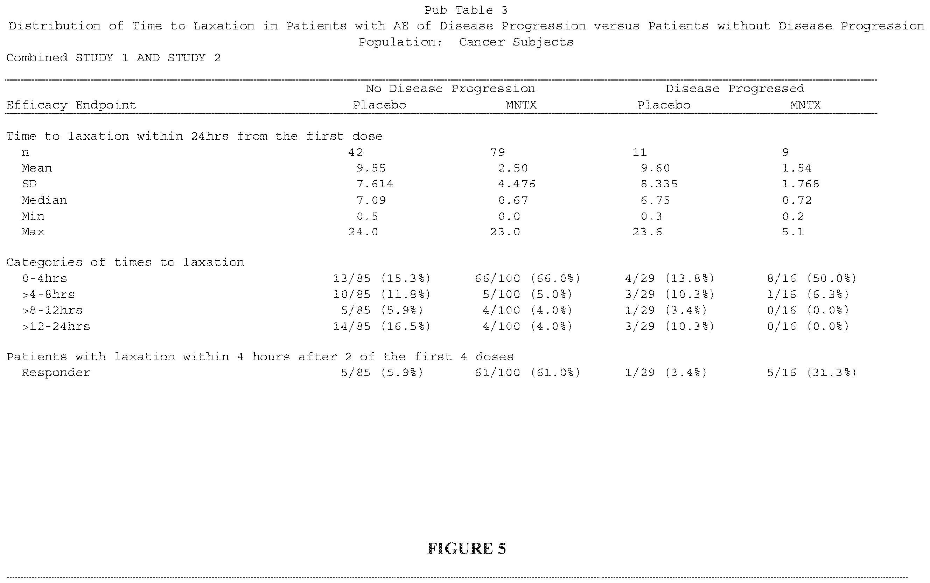

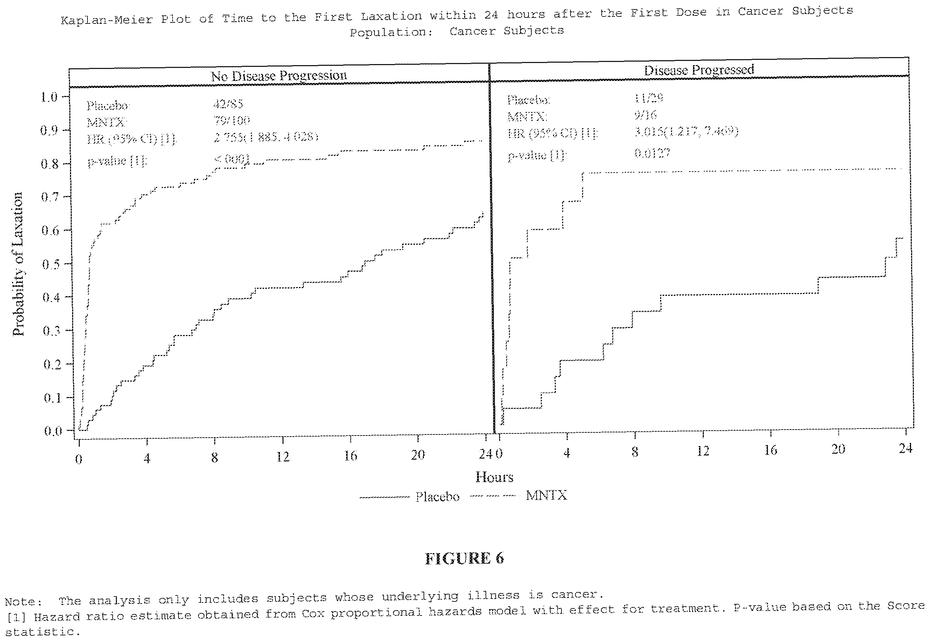

Additional information regarding time to laxation within 24 hours from the first dose as well as categories of time to laxation in both placebo and MNTX-treated subject pools can be found in Figures 3 to 5. Figure 6 provides time to event analysis on time to laxation for subjects with and without AEs of disease progression.

The data indicate a role for MNTX in slowing tumor progression. These are the first placebo-controlled human data demonstrating that MNTX can be an adjunct to therapy and further suggest that the MOR may be a therapeutic target.

### EXAMPLE 4

Routine adverse event (AE) evaluations described in Example 3 were analyzed. AEs of tumor progression in specific cancers and survival analysis of the placebo and methylnaltrexone-treated cohorts in the clinical studies were tabulated. Both studies were randomized, placebo-controlled trials that involved a 2-week treatment period followed by a 2-week follow-up period. The enrolled subjects were advanced illness (incurable cancer or other end-stage disease) subjects with OIC who were receiving stable doses of laxatives and opioid analgesics. In the context of the study, a stable dose of opioid analgesics was defined as no reduction in the dose of opioid analgesic of ≥ 50% for at least three days before the first dose of MNTX. A stable dose of laxatives was defined as having a standing laxative order (and not PRN, or as-needed) for at least three days before the first dose of MNTX. A diagnosis of constipation was provided if the subject had (a) fewer than 3 bowel movements during the previous week by history and no clinically notable laxation in the 24 hours before the first dose of MNTX, or (b) no clinical notable laxation in the 48 hours before the first dose of MNTX. In Study 1 (Example 1), subjects received subcutaneous MNTX 0.15 mg/kg or placebo (PBO) every other day (QOD), and in Study 2 (Example 2) subjects received subcutaneous MNTX (8 mg or 12 mg based on body weight 38 to <62 kg or ≥62 kg, respectively) or placebo administered QOD. The 12 mg fixed dose was designed to correspond to the 0.15 mg/kg weight-based dose. Rescue-laxatives, enemas or manual disimpactions were not administered within 4 hours before or after each dose of MNTX. For the purpose of this analysis, only subjects with a cancer-related diagnosis were considered. For statistical significance testing, the Fisher's exact test was used (p<0.05).

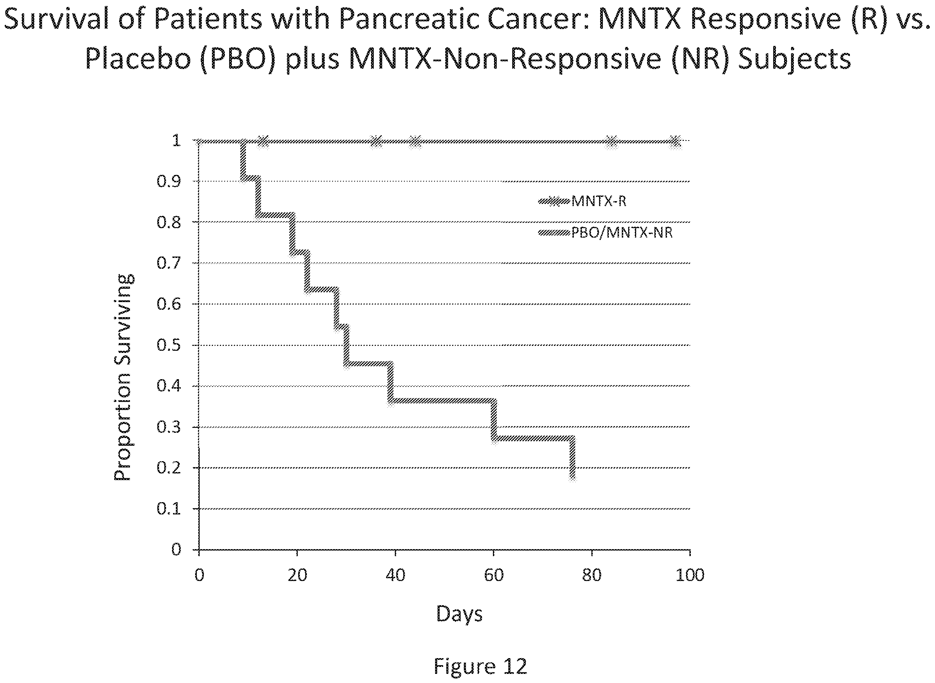

Subsequent to the initial pooled analysis in Example 3, the data from Study 1 and Study 2 was re-analyzed for overall survival (OS) in a post-hoc analysis. Overall survival (OS) was defined as the time interval from the treatment initiation to the date of death or the date of last follow up, whichever occurred first. The subsequent analysis indicated that in Study 1, 78 of the enrolled 134 patients were diagnosed with advanced cancers. In Study 2, 151 of the enrolled 229 patients were diagnosed with advanced cancers. Accordingly, of the 363 subjects randomized across the two studies, 229 (63%) had a cancer-related diagnosis, of which 117 and 112 were randomized to MNTX and PBO, respectively. Figure 12 provides a schematic of the subjects with advanced cancers from the pooled studies that were analyzed in the subsequent analysis. Table 3 provides the patient characteristics from the subsequent pooled analysis of the two studies. Table 4 provides a distribution of the type of cancer diagnosed in the subjects analyzed for survival.

**Table 3. Patient characteristics**

| **Category** | **All patients** | **MNTX** | **Placebo** | **P value** |
|---|---|---|---|---|
| **Cancer Patients** | 229 | 117 | 112 | |
| Female | 105 (46%) | 53 (45%) | 52 (46%) | |
| Median age (range) | 63 (27-91) | 63 (27-91) | 64 (32-90) | 0.82 |
| Albumin < 3.5 g/dL | 94 (41%) | 44 (38%) | 50 (45%) | 0.35 |
| Albumin >/= 3.5 g/dL | 129 (56%) | 69 (59%) | 60 (54%) | |
| Albumin missing | 6 (3%) | 4 (3%) | 2 (2%) | |
| Primary Cancer | | | | |
| Lung | 58 (25%) | 32 (27%) | 26 (23%) | 0.54 |
| Prostate | 30 (13%) | 13 (11%) | 17 (15%) | 0.43 |
| Breast | 23 (10%) | 14 (12%) | 9 (8%) | 0.38 |
| Pancreatic | 16 (7%) | 8 (7%) | 8 (7%) | 1.0 |
| Renal | 12 (5%) | 8 (7%) | 4 (4%) | 0.38 |
| Head and neck | 11 (5%) | 6 (5%) | 5 (4%) | 1.0 |
| Colorectal | 10 (4%) | 4 (3%) | 6 (5%) | 0.53 |
| Uterine | 6 (3%) | 0 (0%) | 6 (5%) | **0.01** |
| All Others (</=4%) | 69 (30%) | 32 (27%) | 37 (33%) | 0.72 |
| | | | | |
| **Non-Cancer Patients** | 134 | 62 | 72 | |
| Femaie | 75 | 34 | 41 | |
| Median age (range) | 71 (34-101) | 72 (34-101) | 69 (35-98) | 0.78 |
| Albumin < 3.5 g/dL | 27 (20%) | 13 (21%) | 14 (19%) | 0.99 |
| Albumin >/= 3.5 g/dL | 104 (78%) | 48 (77%) | 56 (78%) | |
| Albumin missing | 3 (2%) | 1 (2%) | 2 (3%) | |
| Primary Disease | | | | |
| COPD | 38 (28%) | 17 (27%) | 21 (29%) | 0.97 |
| Cardiovascular | 35 (26%) | 20 (32%) | 15 (21%) | 0.19 |
| Neurodegenerative | 16 (12%) | 5 (8%) | 11 (15%) | 0.31 |
| CHF | 15 (11%) | 9 (15%) | 6 (8%) | 0.59 |
| Hepatic Failure | 4 (3%) | 0 (0%) | 4 (6%) | 0.17 |
| Failure to Thrive | 6 (4%) | 2 (3%) | 4 (6%) | 0.82 |
| All Others (</=4%) | 20 (15%) | 9 (15%) | 11 (15%) | 0.90 |

**Table 4. Distribution of primary cancers**

| **Category** | **All patients** | **Placebo** | **MNTX** | **P value** |
|---|---|---|---|---|
| All patients | 229 | 112 | 117 | |
| LUNG | 58 (25%) | 26 (23%) | 32 (27%) | 0.54 |
| PROSTATE | 30 (13%) | 17 (15%) | 13 (11%) | 0.43 |
| BREAST | 23 (10%) | 9 (8%) | 14 (12%) | 0.38 |
| PANCREATIC | 16 (7%) | 8 (7%) | 8 (7%) | 1.00 |
| RENAL | 12 (5%) | 4 (4%) | 8 (7%) | 0.38 |
| HEAD AND NECK | 11 (5%) | 5 (4%) | 6 (5%) | 1.00 |
| COLORECTAL | 10 (4%) | 6 (5%) | 4 (3%) | 0.53 |
| MELANOMA | 8 (3%) | 5 (4%) | 3 (3%) | 0.49 |
| ESOPHAGEAL | 7 (3%) | 4 (4%) | 3 (3%) | 0.72 |
| BRAIN | 6 (3%) | 2 (2%) | 4 (3%) | 0.68 |
| HEPATOCELLULAR | 6 (3%) | 5 (4%) | 1 (1%) | 0.11 |
| UTERINE | 6 (3%) | 6 (5%) | 0 (0%) | 0.01 |
| CERVICAL | 5 (2%) | 2 (2%) | 3 (3%) | 1.00 |
| LYMPHOMA | 4 (2%) | 2 (2%) | 2 (2%) | 1.00 |
| MYELOMA | 4 (2%) | 2 (2%) | 2 (2%) | 1.00 |
| SARCOMA | 3 (1%) | 2 (2%) | 1 (1%) | 0.62 |
| BLADDER | 3 (1%) | 1 (1%) | 2 (2%) | 1.00 |
| BILIARY | 3 (1%) | 1 (1%) | 2 (2%) | 1.00 |
| OTHER | 14 (6%) | 5 (4%) | 9 (8%) | 0.41 |

As shown in Table 4, lung (25%), prostate (13%), breast (10%) and pancreatic (7%) cancers were the most frequent types. Among the non-cancer patients, COPD (29%), cardiovascular disease (27%), neurodegenerative diseases (12%) and congestive heart failure (12%) were the most frequently reported conditions. Non-cancer disease distribution in each of the treatment arms was also well balanced. Treatment arms did not have any major imbalances for gender, age, albumin level or tumor type with the exception of uterine cancer (only enrolled to placebo arm).

Table 5 lists the number of deaths and survivors in the methylnaltrexone-treated group for both studies. The analysis includes the results from the double-blind and open label phases of the studies. When the pool of MNTX-treated subjects was examined, there was a significant difference in the percentage of subject deaths between the MNTX-responsive group and the MNTX non-responsive group. At the time of the most recent analysis, 122 (34%) of the 363 patients had died. The remaining 241 (66%) were alive and censored at the time of the last follow up. Despite equally stringent entry requirements, significantly more cancer patients died during the study than non-cancer patients, irrespective of drug treatment (Placebo, 45.5% cancer vs 20.8% non-cancer, p=0.001; MNTX, 38.5% cancer vs non-cancer 17.7%, p=0.008). Notably, an effect of MNTX response on survival was observed. Among the MNTX-treated cancer patients, significantly more MNTX non-responding patients died during the study than did MNTX responders (51.1% vs 30.6%, P=0.043). No difference was observed between the MNTX responding and non-responding, non-cancer patients (16.7% vs 19.2%, n.s.).

**Table 5. Patient deaths in pooled studies**

| | **Totals** | **Cancer** | **Non-Cancer** | |
|---|---|---|---|---|
| Placebo | 184 | 112 | 72 | P-value |
| Deaths | 66 (35.9%) | 51 (45.5%) | 15 (20.8%)* | 0.001 |
| MNTX | | | | |
| All Patients | 179 | 117 | 62 | |
| Deaths | 56 (31.3%) | 45 (38.5%) | 11 (17.7%)* | 0.008 |
| Responders | 108 | 72 | 36 | |
| Deaths | 28 (25.9%) | 22 (30.6%) | 6 (16.7%) | |
| Non-Responders | 71 | 45 | 26 | |
| Deaths | 28 (39.4%) | 23 (51.1%)** | 5 (19.2%)* | 0.017 |
| P-value | | 0.026 | | |

| | | | | |
|---|---|---|---|---|
| * P-value for cancer vs. non-cancer comparison. ** P-value for MNTX responders vs. non-responders. | | | | |

This difference was statistically significant for MNTX responders who experienced a laxation response within 4 hours, 8 hours and 12 hours of administration of MNTX, and a similar trend is observed for MNTX responders who experienced a laxation response with 24 hours of administration of MNTX (Table 6). The results indicate that an increase in survival is linked to responsiveness to MNTX therapy for constipation in subjects who are receiving opioid therapy.

**Table 6. Subject Deaths in Responder and Non-resonders Groups**

| | Deaths | Survivors | Total | % Deaths | P-Value* |
|---|---|---|---|---|---|
| Non-Responders | 22 | 22 | 44 | 50.0% | |
| Responders < 4hr | 23 | 50 | 73 | 31.5% | 0.0464 |
| Non-Responders | 21 | 18 | 39 | 53.8% | |
| Responders < 8hr | 24 | 54 | 78 | 30.8% | 0.0156 |
| Non-Responders | 18 | 18 | 36 | 50.0% | |
| Responders < 12hr | 27 | 54 | 81 | 33.3% | 0.0872 |
| Non-Responders | 15 | 16 | 31 | 48.4% | |
| Responders ≤ 24hr | 30 | 56 | 86 | 34.9% | 0.1852 |

| | | | | | |
|---|---|---|---|---|---|
| Chi-Square Test w/out Yates correction | | | | | |

Table 7 lists the number of deaths and survivors in both the placebo and the methylnaltrexone-treated groups for subjects diagnosed with lung, breast, prostate, pancreatic and colon cancer. The groups are further categorized into methylnaltrexone responders and methylnaltrexone non-responders alone or in combination with the placebo group. The analysis included the results from the double-blind and open label phases of the studies. When the group of MNTX-treated subjects across both studies was examined, there was a significant difference in the percentage of subject deaths between the MNTX-responsive group and the MNTX non-responsive group, with the number of survivors in the MNTX-responsive group exceeding the number of survivors in the MNTX non-responsive group. This difference was observed for the pooled group of these five cancers *(e.g.,* lung, breast, prostate, pancreatic and colon cancer), and it was particularly striking in subjects diagnosed with pancreatic cancer. For the analysis in Table 7, a subject with laxation within 0-4 hours after two of the first four doses of MNTX was considered a responder. The data in Table 6 may vary slightly from that in Table 4 based on how the subjects were coded as cancer patients during analysis of the data.

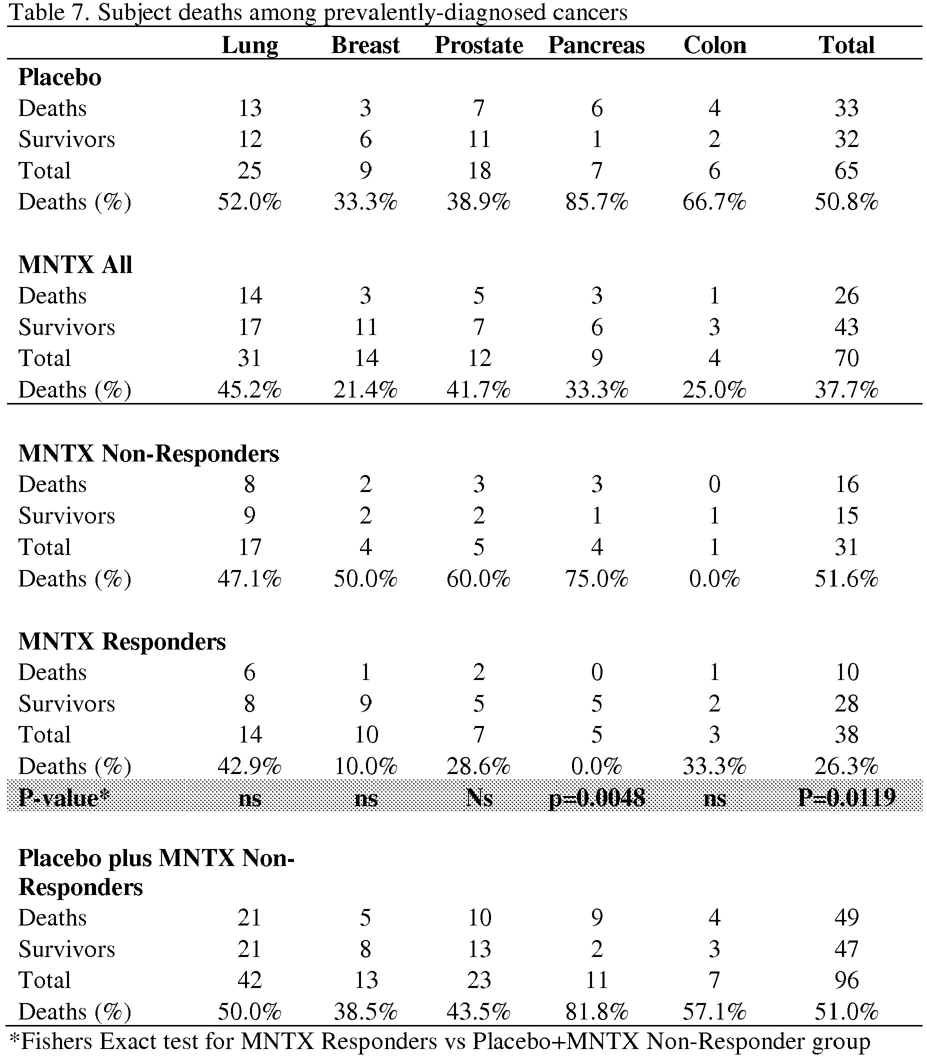

While the sample sizes of each of these cancer types limited the power of the analyses, the trends are consistent in all types examined. For each cancer type, patients treated with MNTX showed an increased survival compared to placebo patients. Furthermore, the difference in survival seen between these two populations was further magnified in patients responding to MNTX compared to patients not responding to MNTX or treated with placebo. The results of multivariable analysis, which included tumor type (lung cancer, prostate cancer, breast cancer, pancreatic cancer, other cancers), indicated that tumor type was not an independent prognostic factors for longer overall survival (data not shown). Accordingly, the effect of response to MNTX on overall survival in cancer patients was not specific for any cancer and was observed as a universal trend across the various types of cancers that were examined.

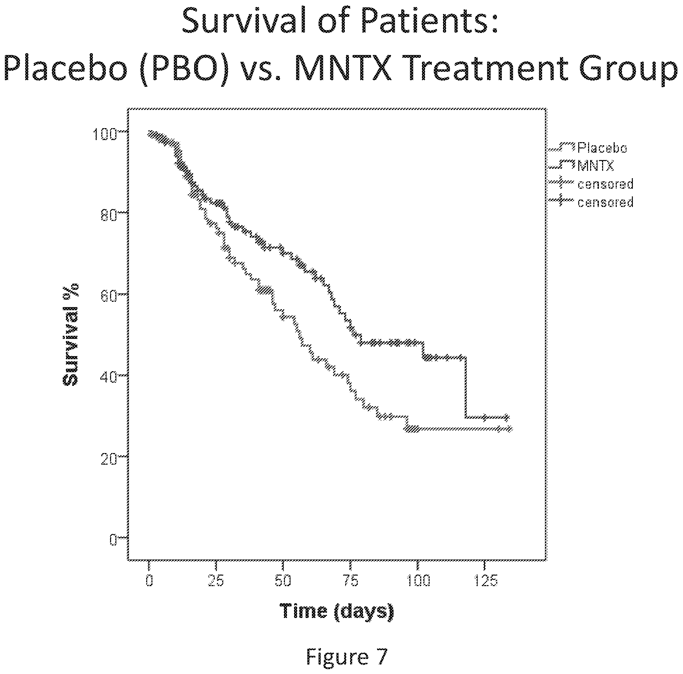

Figure 7 illustrates the difference in survival between the placebo group and the methylnaltrexone-treated group during the double-blind and open label extension phases in a clinical study. There is a clear trend in the difference in survival between the subjects in the placebo group and those in the methylnaltrexone treatment group. The percentage of subject deaths in the treatment group is less than that in the placebo group. In a statistical analysis of the results, it was observed that cancer patients treated with MNTX had a longer median overall survival (OS) compared to the cancer patients treated with placebo (117 subjects vs. 112 subjects, respectively; 76 days (95% CI 43-109) vs. 56 days (95% CI 43-69), respectively; p = 0.033).

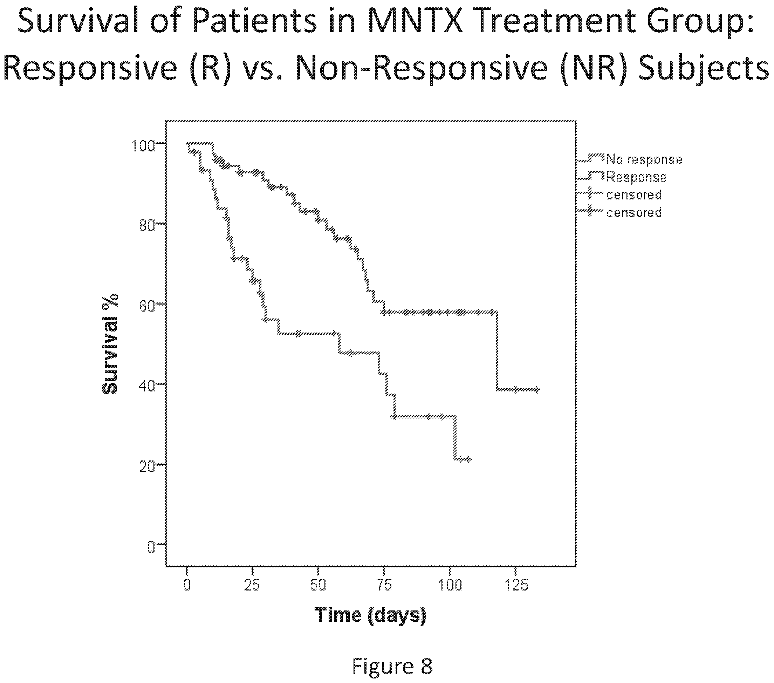

Figure 8 illustrates the difference in survival within the methylnaltrexone-treated group between the MNTX-responsive and MNTX non-responsive subjects during the double-blind and open label extension phases in the same study. The rate of subject death in the responsive group is lower than that in the non-responsive group. In a statistical analysis of the results, it was observed that MNTX-responsive subjects had a longer median overall survival (OS) compared to the MNTX non-responsive group (72 subjects vs. 45 subjects, respectively; 118 days vs. 58 days, respectively; p = 0.001).

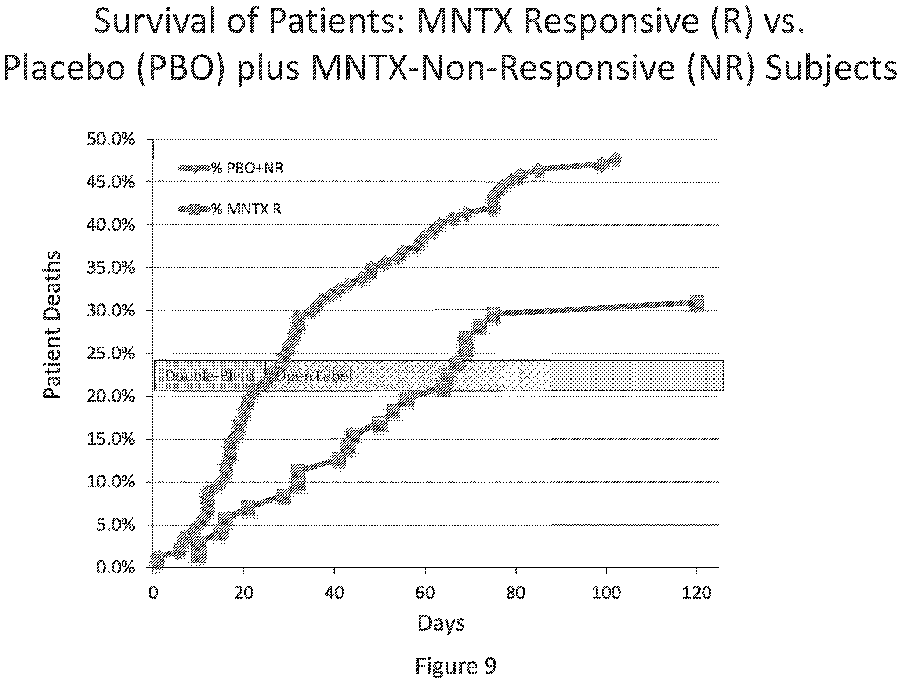

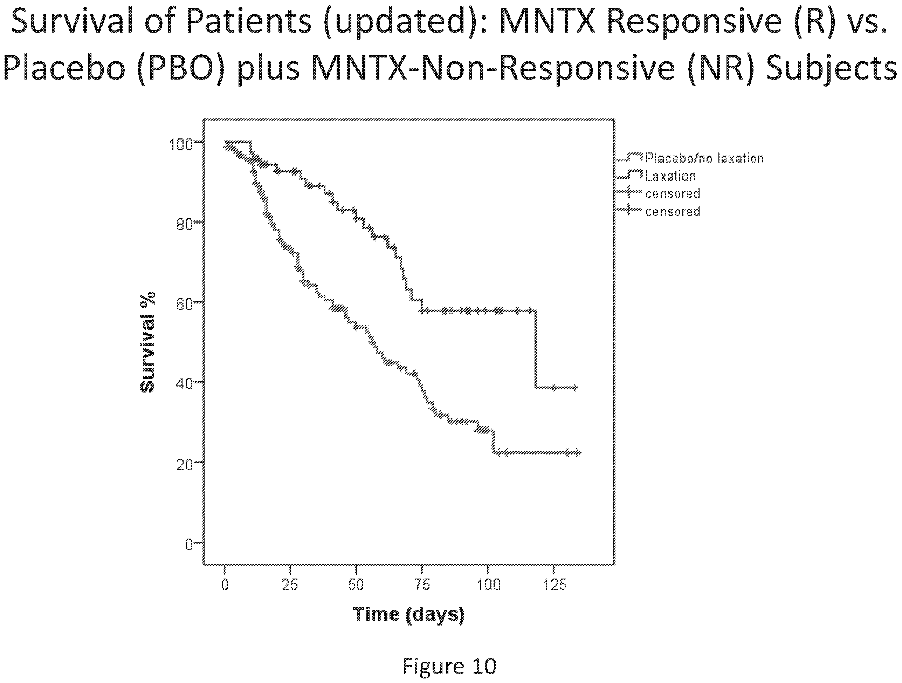

Figure 9 shows the difference in survival between the placebo group plus the methylnaltrexone non-responsive group and the methylnaltrexone-responsive group during the double-blind and open label extension phases. The difference in rate of subject death between the responsive group and the combined placebo plus non-responsive group can be observed, with the responsive group exhibiting a lower subject death rate than that of the combined placebo and non-responsive group. In a statistical analysis of the results, it was observed that MNTX-responsive group had a longer overall survival (OS) compared to the combined placebo and non-responsive group (121 days vs. 58 days, respectively, p < 0.001). In an updated analysis, a similar result was observed. In the updated analysis, 72 cancer patients with response to MNTX had a longer median OS compared to 157 cancer patients without response or treated with placebo (118 days, 95% CI 46-190 vs. 56 days, 95% CI 43-69; p<0.001; Figure 10).

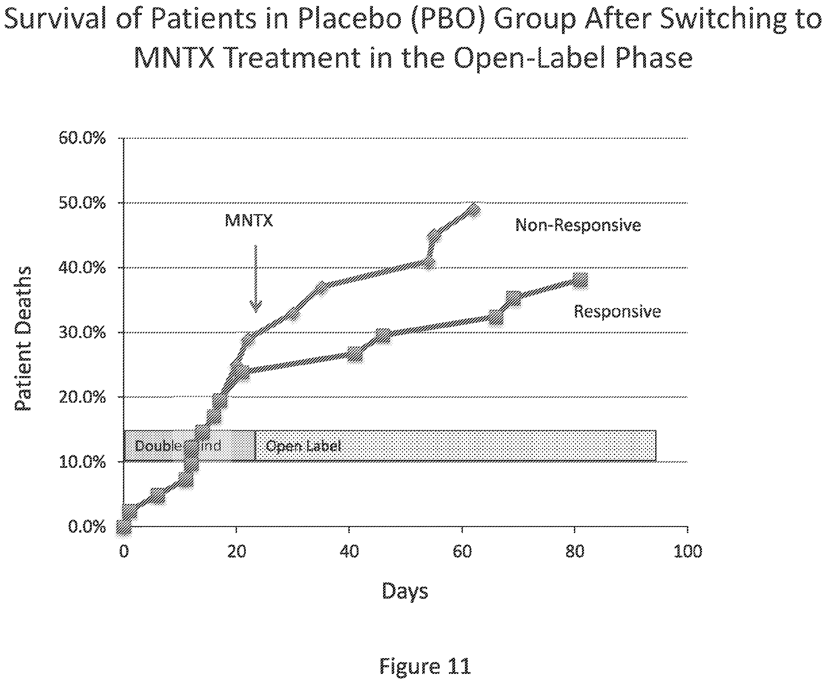

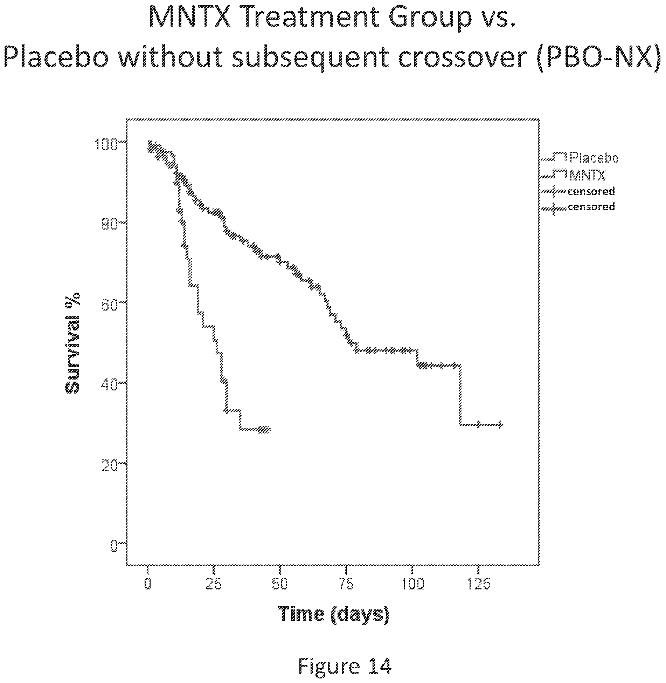

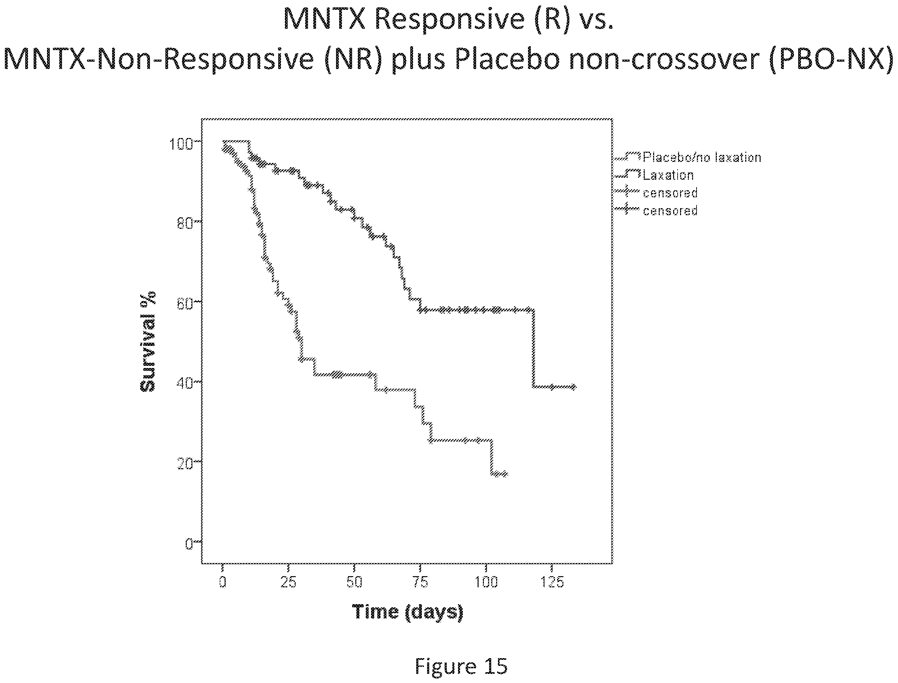

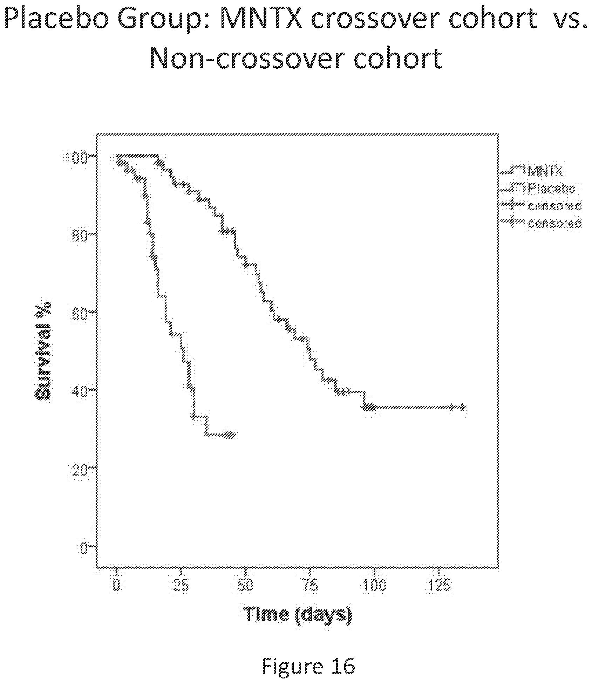

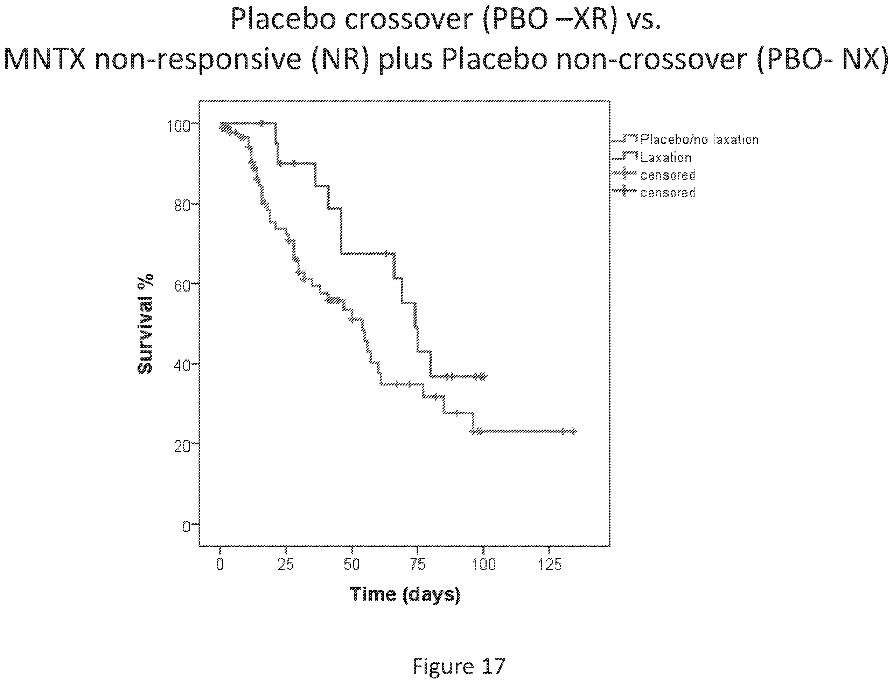

In addition, a difference in overall survival was observed in the placebo group between methylnaltrexone non-responders and the methylnaltrexone responders after methylnaltrexone was administered to the placebo group during the open label extension phase of the study. Figure 11 illustrates the difference in survival between the methylnaltrexone non-responders and the methylnaltrexone responders after crossover to methylnaltrexone treatment occurred. A bifurcation in the subject death rate is observed between the responsive and non-responsive subjects after methylnaltrexone administration was begun in the placebo group, with the responsive subjects exhibiting better overall survival than the non-responsive subjects. In the most updated analysis of the pooled data, it was observed that patients (n=117) treated with MNTX had a longer median overall survival compared to 66 patients treated with placebo without subsequent crossover to MNTX (76 days (95% CI 43-109) vs. 26 days (95% CI 17-35); p<0.001, Figure 14). Furthermore, 72 patients with response to MNTX ("Laxation") also had a longer median OS compared to 111 patients without response ("No Laxation") or treated with placebo without crossover to MNTX (118 days, 95% CI 46-190 vs. 30 days, 95% CI 23-37; p<0.001; Figure 15). Patients (n=56), who crossed over to MNTX had a longer median overall survival compared to 66 patients treated with placebo, who did not (75 days (95% CI 59-91) vs. 26 days (95% CI 17-35); p<0.001, Figure 16). Finally, placebo patients (n=23) with response to MNTX during open-label treatment had a trend towards longer median OS compared to 99 patients without response or treated with placebo without crossover to MNTX (74 days, 95% CI 62-86 vs. 54 days, 95% CI 39-69; p=0.10; Figure 17).

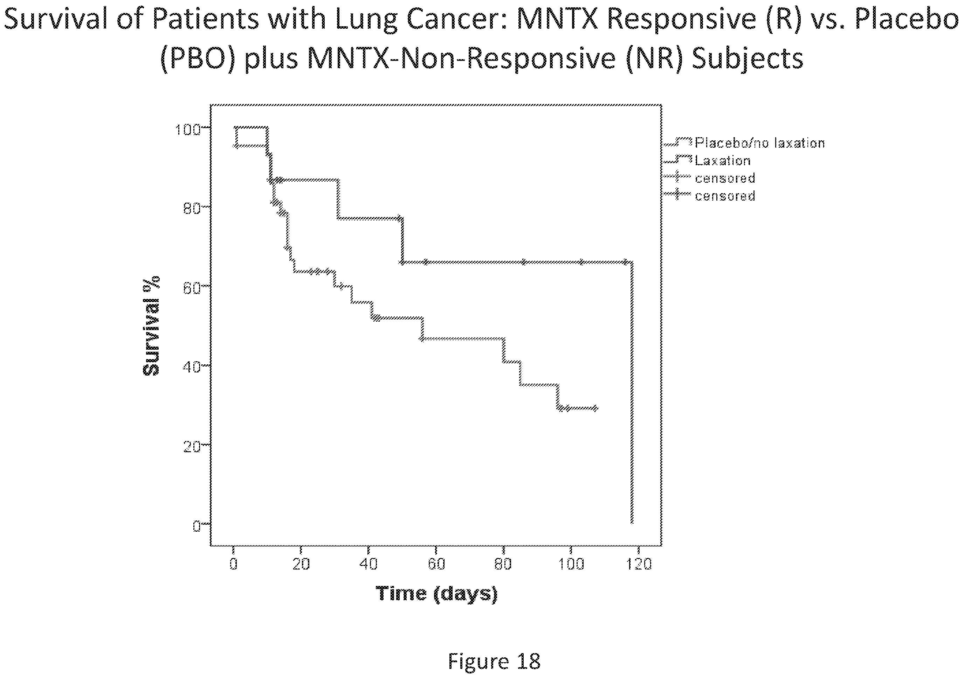

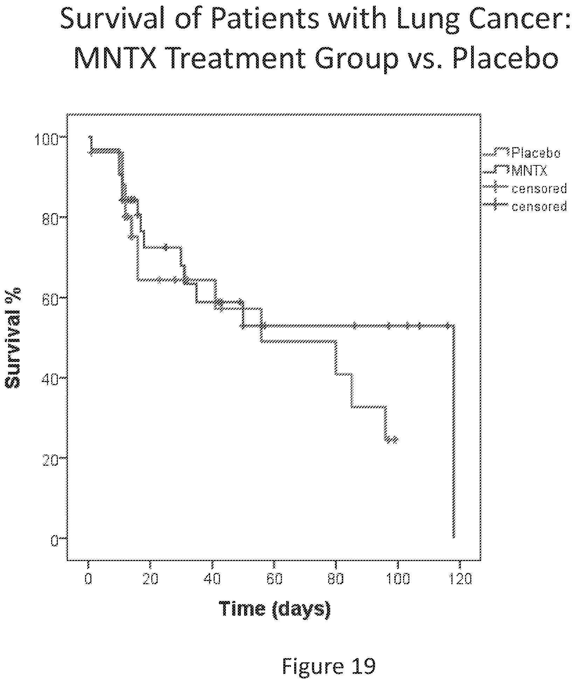

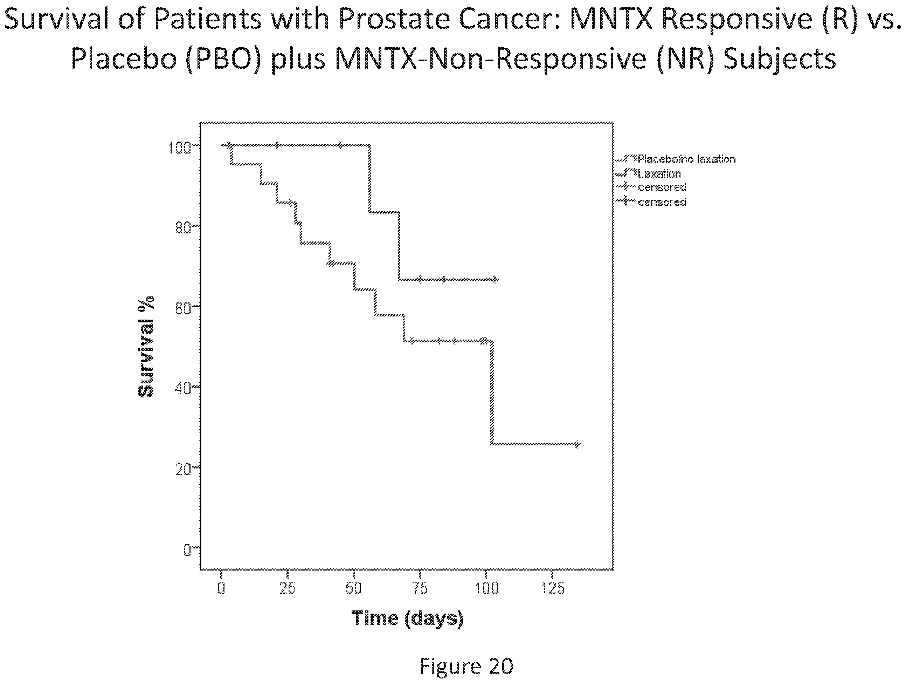

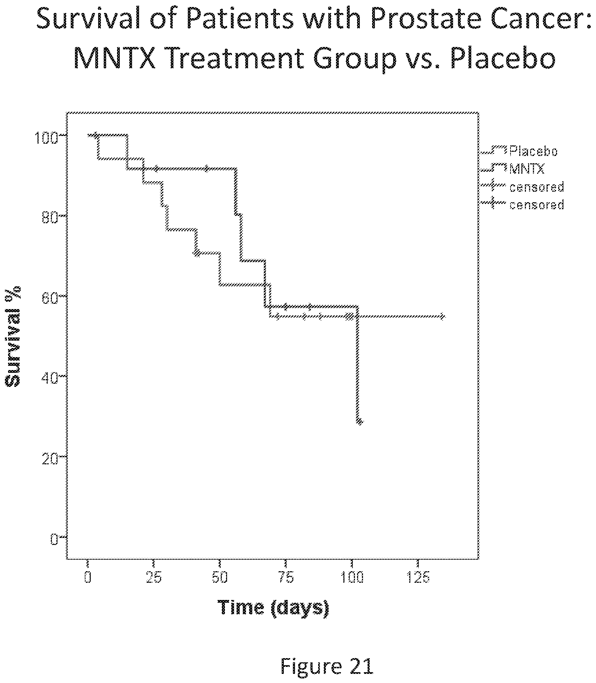

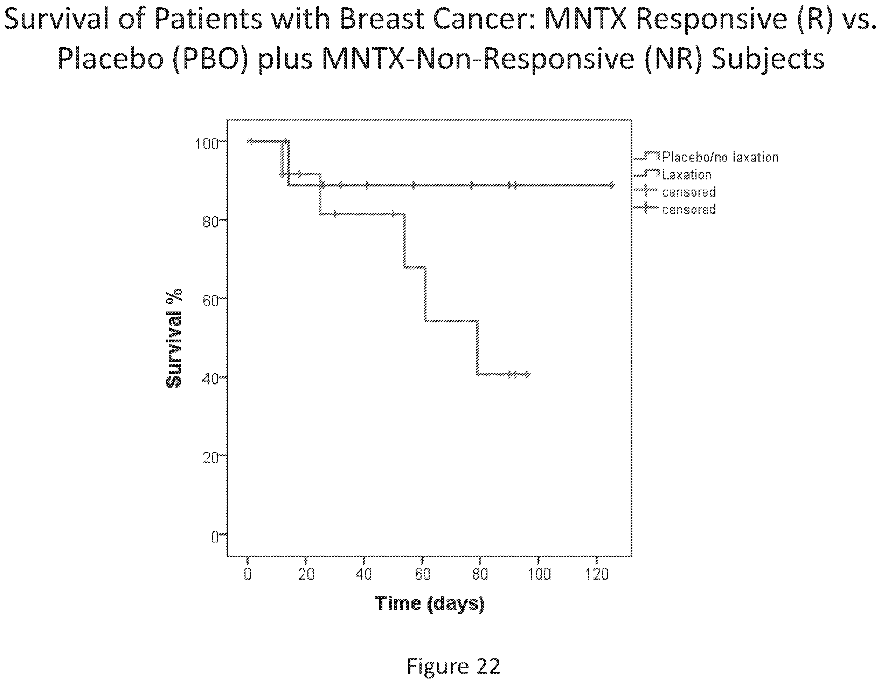

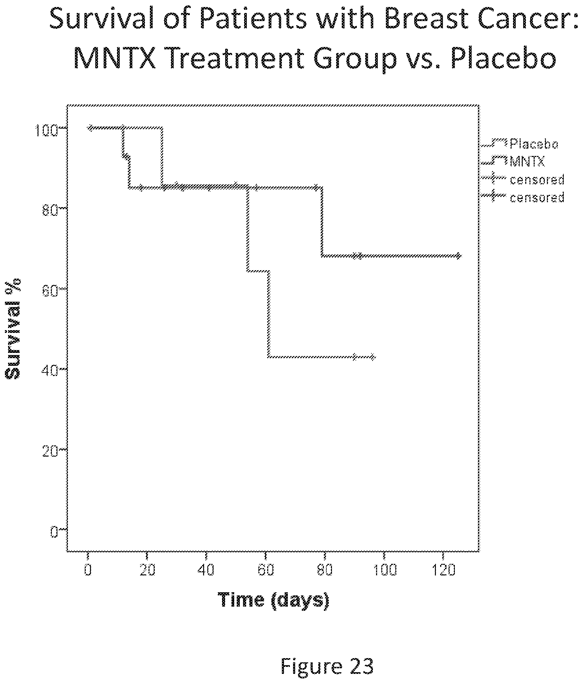

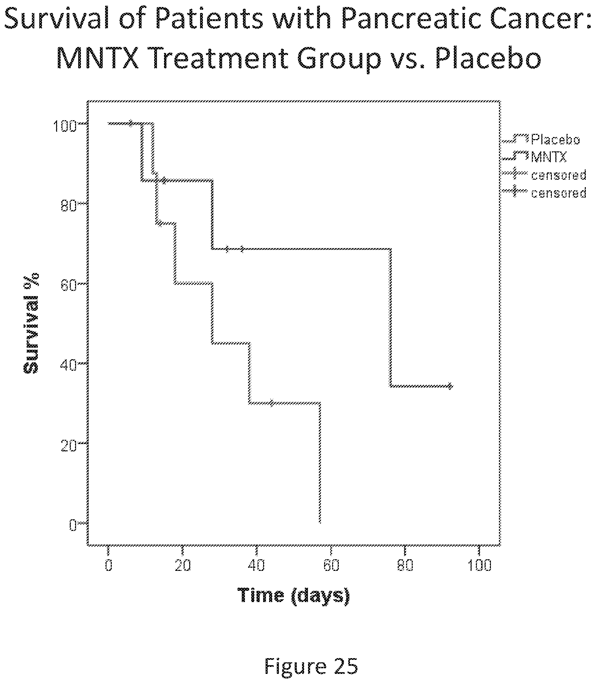

The MNTX effect on survival was also in the four largest tumor subgroups, which included lung, prostate, breast and pancreatic cancers. While the sample sizes of each of these cancer types limited the power of the analyses, the trends are consistent in all types examined. For each cancer type, patients treated with MNTX showed an increased survival compared to placebo patients. Furthermore, the difference in survival seen between these two populations was further magnified in patients responding to MNTX compared to patients not responding to MNTX or treated with placebo. In 58 patients with lung cancer, 32 patients treated with MNTX had longer, though not statistically different median OS compared to 26 patients treated with placebo (118 days vs. 56 days; p=0.34; Figure 19). Lung cancer patients (n=15) with response to MNTX ("Laxation") had a trend towards longer median OS compared to 43 patients without response ("No Laxation") or treated with placebo (118 days vs. 56 days; p=0.13; Figure 18). Similar trends were observed for patients with prostate cancer and breast cancer (Figures 20-23). In 16 patients with pancreatic cancer, 8 patients treated with MNTX had a trend to longer median OS compared to 8 patients treated with placebo (76 days vs. 28 days; p=0.14; Figure 25). There was no death among 4 patients with response to MNTX, which translated to improved OS compared to 12 patients without response or treated with placebo (medians not calculated; p=0.035). In the initial analysis for pancreatic cancer patients, survival of the methylnaltrexone-responsive group was extended for up to almost 100 days after administration of methylnaltrexone during the study (Figure 12). In comparison, the percentage of surviving subjects among the placebo and non-responsive groups fell to below 20% at about 70 days in the study.

The observations regarding MNTX were compared to the effect of albumin in order to assess the clinical significance of the response to MNTX and its effect on overall survival. Levels of albumin <3.5 g/dL have been found to be an independent prognostic factor for survival in cancer patients refractory to standard therapies. Albumin levels were available for 223 of the cancer patients, of which 94 (41%) were determined to have albumin levels <3.5 g/dL. Table 8 provides the results of a statistical analysis of the pooled data using a multicovariate model for overall survival (Cox regression).

**Table 8. Multicovariate model for overall survival (Cox regression)**

| **Variable** | **Hazard Ratio** | **95% CI** | **P value** |
|---|---|---|---|
| All patients (MNTX 117, placebo 112) | | | |
| Albumin: >=3.5 g/dL vs. < 3.5 g/dL | 0.47 | 0.31-0.71 | <0.001 |
| Treatment: MNTX vs. placebo | 0.70 | 0.47-1.06 | -0.009 |
| Response vs. no response | -0.46 | 0.28-0.75 | 0.002 |
| Excluding crossover to MNTX (MNTX 117, placebo 61) | | | |
| Albumin: >=3.5 g/dL vs. < 3.5 g/dL | 0.40 | 0.24-0.65 | <0.001 |
| Treatment: MNTX vs. placebo | 0.32 | 0.18-0.57 | <0.001 |
| Response vs. no response | 0.32 | 0.18-0.55 | <0.001 |
| Patients on placebo (crossover to MNTX 51, placebo 61) | | | |
| Albumin: >=3.5 g/dL vs. < 3.5 g/dL | 0.58 | 0.33-1.01 | 0.06 |
| Treatment: MNTX vs. placebo | 0.15 | 0.07-0.32 | <0.001 |
| Response vs. no response | 0.64 | 0.33-1.27 | 0.20 |

| | | | |
|---|---|---|---|
| MNTX = methylnaltrexone | | | |

In the multivariate analysis, which included albumin (≥3.5g/dL vs. <3.5g/dL), treatment (MNTX vs. placebo) or response to MNTX (response vs. no response or placebo), albumin ≥3.5g/dL ((hazard ratio [HR] 0.47, 95% CI 0.31-0.71, p<0.001), and response to MNTX (HR 0.46, 95% CI 0.28-0.75, p=0.002) were independent prognostic factors for longer overall survival. Treatment with MNTX, regardless of response, had a trend towards a longer overall survival (HR 0.70, 95% CI 0.47-1.06, p=0.09).

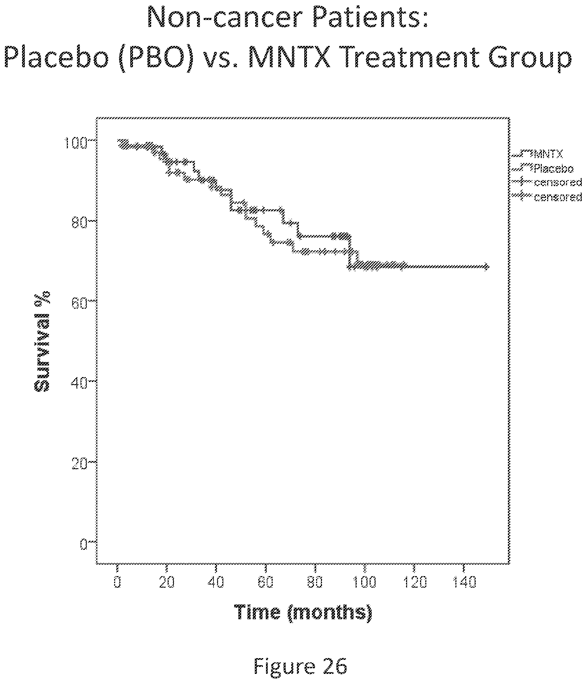

For comparison, there was no effect of MNTX or MNTX response on OS of the non-cancer patients, although the median survival of the non-cancer patients was longer than that of the cancer patients (Figure 15). The patients in Study 1 and Study 2 who did not have a diagnosis of cancer were analyzed to determine if a prolongation of overall survival after treatment of MNTX was observed. Based on the analysis, there was no difference in overall survival between MNTX vs. placebo in the 134 patients with advanced illness other than cancer (p = 0.88, Figure 26).

For the analysis carried out in this Example and in Example 3, a subject with laxation occurring from 0-4 hours after two of the first four doses of MNTX was considered a MNTX responder or responsive subject. Data for placebo patients who elected to participate in the open-label MNTX treatment phase was collected from the first 4 doses of open-label treatment. Data for the MNTX-treated patients was collected from the first 4 doses of the double-blind phase of the study.

Accordingly, in the retrospective post-hoc analysis of the two pooled studies, it was demonstrated that in patients with advanced terminal cancers and OIC, treatment with MNTX (76 days vs. 56 days, p=0.033) and even more so response to MNTX (118 days vs. 56 days, p<0.001) is associated with prolonged OS compared to placebo. The data suggest an effect of MNTX on OS that is as significant as but independent from albumin. Without being bound by theory, it is hypothesized that a direct effect of the drug on tumors can be an explanation for these observations. The improved survival observed with MNTX response can be a direct effect of the drug on cellular targets related to the mu opioid receptor and its pathway. The results provide evidence supporting the possible role of opioids and *mu* opioid receptors (MOR) in cancer progression. Interestingly, the impact on survival appeared to be larger in patients with response on MNTX (laxation), which can be understood as a pharmacodynamic marker. In the study, 50% of patients crossed over from placebo to MNTX, which could have influenced survival outcomes favoring the placebo arm. An analysis was performed in which patients crossing over from placebo to MNTX were excluded; it was found that in this subgroup, patients with response to MNTX had nearly tripled OS compared to placebo or no response (118 days vs. 30 days, p<0.001). Even patients treated on placebo lived longer if they crossed over to MNTX (75 days vs. 26 days, p<0.001).

This is the first human demonstration of improved survival for cancer patients following treatment with peripheral opioid antagonists. While tertiary opioid antagonists have been clinically available for over 70 years, the development of peripheral opioid antagonists that can be administered to cancer patients requiring mu opioids for pain or during surgery without affecting analgesia or precipitating withdrawal may represent an important therapeutic adjunct. Moderate to severe pain, which requires opioids administration, affects up to 70-80% of patients with advanced cancers and are widely used in surgery. There has been a major focus in anesthesia as to whether the type of anesthetic used can influence tumor recurrence. Methylnaltrexone has been safely given to more than 500 colectomy patients without affecting perioperative analgesia in two proposed randomized clinical trials (Yu, et al. Dis Colon Rectum 2011; 54:570-578). The widespread use of opioids in cancer surgery and cancer care underscores the possible clinical relevance of our observations, which if confirmed, could transform patient care.

In conclusion, the data indicate that MNTX use in advanced cancer patients treated with opioids can prolong survival, plausibly through attenuation of opioid-mediated MOR signaling. While the findings are in patients with advanced malignancies, the hypothesis that mu opioid antagonism can have a potential therapeutic value also extends to earlier tumors and to the perioperative period. The data indicate a role for MNTX in increasing the survival of a subject suffering from cancer who is concurrently taking an opioid or a combination of opioids. These are the first placebo-controlled human data demonstrating that MNTX can be a adjunct to therapy and further suggest that the MOR may be a therapeutic target.

### EXAMPLE 5

The acquired ability of a localized tumor to metastasize is a multistep process involving many pathways, including those involved in angiogenesis, focal adhesion, invasion and eventually colonization of a distant site. This ability is accompanied by an epithelial-to-mesenchymal transition (EMT), involving changes in gene expression patterns (Avizienye, E. and M.C. Frame. 2005. Curr Opin Cell Biol 17:542-547). Studies have implicated Src signaling in these processes. Vascular endothelial growth factor (VEGF) is a pivotal component of both normal and malignant angiogenesis, and it has been validated as a clinical target in many tumors (Hurwitz et al. 2004. N Engl J Med. 350:2335-2442; Ellis, L.M. and D.J. Hicklin. 2008. Nat Rev Cancer 8:579-591). Paclitaxel is a microtubule formation inhibiting agent with a broad spectrum of activity in multiple tumor types (Miller et al. 2007. N Engl J Med. 357:2666-2676; Schiller et al. 2002. N Engl J. Med. 346:92-98; Ozols et al. 2003. J Clin Oncol 21:3194-3200). Three-times weekly paclitaxel followed by a week off has been approved in combination with bevacizumab for metastatic cancer treatment. Accordingly, a study is carried out to determine the efficacy as well as the maximum tolerated dose and dose-limiting toxicities of a combination treatment of dasatinib, bevacizumab and paclitaxel with or without methylnaltrexone in subjects with advanced or metastatic cancer that is refractory to standard treatment.

Subjects with advanced or metastatic cancer that is refractory to standard therapy, relapsed after standard therapy, or who have no standard therapy available that improves survival by at least three months are included in the study. Subjects are selected such that s/he is at least three weeks beyond treatment with a cytotoxic chemotherapy regimen, or therapeutic radiation. In addition, subjects for inclusion in the study have normal organ and marrow function, e.g. absolute neutrophil count ≥ 1000/mL, platelets ≥ 90,000/mL, creatinine ≤ 2 x ULN, total bilirubin ≤ 2.0, ALT (SGPT) ≤ 5 x ULN (or total bilirubin ≤ 3 x ULN, ALT (SGPT) ≤ 8 x ULN in subjects with liver metastasis). Subjects receiving opioids within two weeks before the study and subjects who cannot be off opioids until initiating the study are excluded.

Dose escalation proceeds as described in Tables 6 and 7. The protocol utilizes a 3+3 dose escalation design with 3 subjects per cohort. Three subjects are entered at each dose level to obtain adequate safety data. Three subjects are treated at dose level 1 and evaluated for toxicity. If none of the three subjects at dose level I experience a dose-limiting toxicity (DLT), the next cohort of three subjects is treated at the next higher dose level. If the incidence of DLT among the three subjects is 1 in 3, then the cohort is expanded to six subjects. If two or more of six subjects treated at a given dose level experience a DLT, then the maximum tolerated dose (MTD) is considered to have been exceeded. The MTD is accordingly defined as the highest does studied in which the incidence of DLT is less than 33%. Alternatively, the MTD is defined as the highest dose below any dose that has one-third or more subjects with DLT. If two or more subjects experience DLT at dose level 1, then the dose is decreased to dose level -1. The cohort defined as the MTD can be expanded by up to an additional 14 subjects to further evaluate toxicity and correlative data.

**Table 7. Dose-Escalation Schedule for Dasatinib, Bevacizumab and Paclitaxel (28-day cycle)**

| **Dose level** | **Dasatinib, daily p.o.** | **Bevacizumab, i.v. day 1 and 15** | **Paclitaxel, i.v. day 1, 8 and 15** |
|---|---|---|---|
| -1 | 40 mg | 2.5 mg/kg | 30 mg/m2 |
| 1 | 50 mg | 5 mg/kg | 40 mg/m2 |
| 2 | 70 mg | 5 mg/kg | 40 mg/m2 |
| 3 | 70 mg | 5 mg/kg | 60 mg/m2 |
| 4 | 70 mg | 10 mg/kg | 60 mg/m2 |
| 5 | 100 mg | 10 mg/kg | 60 mg/m2 |
| 5A | 100 mg | 10 mg/kg | 75 mg/m2 |
| 6 | 100 mg | 10 mg/kg | 90 mg/m2 |

**Table 7. Dose-escalation Schedule for Dasatinib, Bevacizumab, Paclitaxel and Methylnaltrexone (28-day cycle)**

| **Dose level** | **Dasatinib, daily p.o.** | **Bevacizumab, i.v. day 1 and 15** | **Paclitaxel, i.v. day 1, 8 and 15** | **Methylnaltrexone, s.c.** |
|---|---|---|---|---|
| -1 | 100 mg | 10 mg/kg | 75 mg/m2 | 0.10 mg/kg BID |
| 1 | 100 mg | 10 mg/kg | 75 mg/m2 | 0.15 mg/kd BID |

**Table 8. Regimen Description**

| **Agent** | **Premedication Precautions** | **Dose** | **Route** | **Schedule** | **Cycle Length** |
|---|---|---|---|---|---|
| Dasatinib | | ** | Orally with food with a cup of water | Day 1-28 | 28 Days |
| Bevacizumab | | ** In 100 cc NS | 1^{st} dose infused over 90 minutes, infusion can be shortened to 60 minutes if the initial infusion is well -tolerated and then to 30 minutes if again well-tolerated | Day 1, 15 | 28 Days |
| Paclitaxel | According to institutionalized practice | ** In 250 cc NS | Infusion over 60 minutes, through paclitaxel giving set | Day 1, 8, 15 | 28 days |
| Methylnaltrexone | | ** | Administer subcutaneously into upper arm, abdomen, or thigh. Rotate injection site. Do not use tender, bruised, red or hard areas. | Day 1-28 | |

Intra-patient dose escalation is allowed in subjects, as deemed appropriate by the physician, to the highest dose level already deemed safe for any subjects with Grade 1 or less toxicity at the current dose level. No subjects are enrolled in the next dose level until three subjects enrolled at the previous dose level have completed at least three weeks of therapy. If a DLT is observed in at least one of the first three subjects of a six-patient cohort after one cycle, then dose escalation does not proceed until all six subjects in the cohort are assessed for toxicity after one cycle.

Subjects continue treatment until their disease worsens, their side effects become too severe, or it is deemed by the physician or patient that it is not in the patient's best interest to continue.

If an individual patient experiences a new clinically significant grade 3 or greater toxicity, treatment is held until recover to ≤ grade 1 or to the baseline levels. The drug to which toxicity is attributed by the physician can be reduced by up to 50%. If it is unclear which drug is responsible for the toxicity, then all drugs are reduced by up to 50%. Subjects requiring dose reductions during the first three weeks of therapy are replaced, as long as any toxicity leading to dose reduction is not considered a DLT.

If a response is observed in a particular tumor type with the study combination, then the study is expanded to include a total of 14 participants with that tumor type, and those subjects are administered the highest safe dose yet determined. Responses have been observed in dendritic cell sarcoma, breast, prostate, gastroesophageal and non-small cell lung cancer subjects. For the purpose of adding up to 14 additional participants, a tumor response is defined as one or more of the following: (1) stable disease for more than or equal to 4 months, (2) decrease in measurable tumor (sentinel lesions) by more than or equal to 20% by RECIST criteria, (3) decrease in tumor markers by more than or equal to 25% (*e.g.,* a ≥ 25% decrease in CA-125 for subjects with ovarian cancer), or (4) a partial response according to the Choi criteria (*i.e.,* a decrease in size by 10% or more, or a decrease in the tumor density, as measured in Hounsfield units (HU), by more than or equal to 15%.

Laboratory studies *(e.g.,* CBC with differential, sodium, potassium, chloride, bicarbonate, BUN, creatinine, glucose, calcium, magnesium, albumin, alkaline phosphatase, total bilirubin, SGPT [ALT], serum, pregnancy test) are carried out, with the first study carried out within two weeks before the first dose, then about once a week for 4 weeks and then about every 4 weeks, preferably before the next cycle.

Appropriate scans and markers for tumor measurement are conducted at baseline and at approximately every other cycle. Subjects can be staged using computed tomography (CT), although magnetic resonance imaging (MRI) can also be performed. Optional Dynamic Contrast-Enhanced Magnetic Resonance Imaging (DCE-MRI) can be performed to predict response in terms of anti-angiogenesis. DCE-MRI can be conducted at the following time points: (a) at baseline (within one week before day 1 of treatment), (b) at acute phase (48 hours +/- 6 hours after the first dose of study combination), and (c) at chronic phase (at the end of cycle 1 on day 28).

A 12-lead ECG and an echocardiogram (ECHO) are conducted at baseline within 4 weeks prior to the first study treatment.

The use of concomitant strong CYP3A4 inducers can decrease dasatinib plasma concentrations and should be avoided (*e*.*g*., dexamethasone, phenytoin, carbamazepine, rifampin, rifabutin, phenobarbital, St. John's Wort, and the like). CYP3A4 inhibitors (e.g. ketoconazole, itraconazole, clarithromycin, atazanavir, indinavir, nefazedone, nelfinavir, ritonavir, saquinavir, telithromycin, voriconazole, and the like) can increase dasatinib plasma concentrations. Grapefruit and pomegranate juice can also increase dasatinib plasma concentrations and should be avoided.

Toxicities are described according to the NCI-CTCAE Version 3.0. Dose-limiting toxicity (DLT) is defined as any clinically grade 3 or 4 non-hematologic toxicity as defined in the NCI CTC v3.0, expected and believed to be related to the study medications (except nausea and vomiting, electrolyte imbalances responsive to appropriate regimens or alopecia), any grade 4 hematologic toxicity lasting at least three weeks or longer (as defined by the NCI-CTC v3.0) or associated bleeding and/or sepsis; any grade 4 nausea or vomiting > 5 days despite maximum anti-nausea regimens, and any other grade 3 non-hematologic toxicity including symptoms/signs of vascular leak or cytokine release syndrome; or any severe or life-threatening complication or abnormality not defined in the NCI-CTCAE v3.0 that is attributable to the therapy. The MTD is defined by the DLTs that occur in the first cycle (4 weeks-induction phase). The use of growth factors is acceptable during the study.

Efficacy is evaluated using the following criteria. Subjects with lymphoma are measured per the WHO criteria, and all others are evaluated using RECIST criteria version 1.1.

This is a standard 3+3 protocol with eight dose levels (-1 and 1-6) in the dasatinib, bevacizumab and paclitaxel arm and two dose levels (-1 and 1) in the dasatinib, bevacizumab, paclitaxel and methylnaltrexone arm. The protocol allows dose expansion as described above. Optional peripheral blood markers of angiogenesis and other serum biomarkers can be conducted to gather additional information about treatment effects on angiogenesis. These include, for example, VEGF levels, VCAM-1 levels and soluble VEGFR-2 levels. Circulating cytokines can be measured by commercially available ELISAs. Other peripheral blood correlates can be performed as described (Alessandri et al. 1999. Clin Exp Metastasis 17:655-662; Di Cicco et al. 2008. Curr Cancer Drug Targets 8:199-206). Peripheral blood markers can be examined at the following time-points for subjects doing DCE-MRI: (1) at baseline (within one week prior to Day 1 of Cycle 1 of treatment), (2) 48 hours +/- 6 hours after dose on Day 1, Cycle 1, and (3) Day 28 of Cycle 1 and/or of Cycle 2. Peripheral blood markers can be examined at the following time-points for subjects not doing DCE-MRI: (1) at baseline (within two weeks prior to Day 1 of Cycle 1 of treatment), (2) Day 6-8, Cycle 1, and (3) Day 27-28 of Cycle 1 and/or of Cycle 2.

Optional tumor biopsies, skin biopsies and peripheral blood mononuclear cell (PBMC) levels can be evaluated for a variety of markers to determine efficacy. These include, for example, VEGF, VEGFR-2, VEGF, VEGFR-2, phospho-VEGFR-2, microvessel density, CD 31, hypoxia-inducible factor-1 (HIF-1), caspase-3, p53, Ki67, autophagy-related events, total Src, pSrcY500 (negative regulation), pSrc Y419 (positive regulation), and the like. Examples of methodologies that can be used include immunohistochemistry, Western blotting, electron microscopy, assessing the degradation rate of long-lived proteins, enzyme sequestration assay, or LC3 protein assessment. Studies can be performed by electron microscopy, immunofluorescence and Western blotting as described (Talloczy et al. 2002. Proc Natl Acad Sci USA 99:190-195; Janku et al. 2011. Nat Rev Clin Oncol 8:528-539). Samples are obtained at the baseline and between Day 8 and Day 15 of cycle 1.

Optional Src mutation analysis (exon 12 codon 531) can be performed to assess whether Src mutations correlate with therapeutic effect of Src inhibition.

Optional monitoring of circulated tumor cells to monitor response are conducted at the baseline and the end of every even cycle.

Optional gene expression profiles predicting dasatinib sensitivity can be performed.

Optional tumor biopsies will be obtained during screening period before the first dose of study medication. In one arm of the study, subjects are not be treated with opioids for at least seven days. Biomarker studies include quantitative assessment of tumor *mu* opioid receptor (MOR) with immunohistochemistry and MOR AI18G Single Nucleotide Polymorphism (SNP) Analysis.

Tissue Microarrays (TMAs) can be built based on de-identified patient cancer samples. Expression of MOR is determined in TMAs built from these tumor tissues using immunohistochemical (IHC) techniques known in the art. Commercially available antibodies are utilized. IHC staining is scored qualitatively by two independent pathologists using a 4-point scale (0 = negative, 1 = weak, 2 = moderate, 3 = strong) as previously described.

Analysis of ordinal expression levels utilizes the Wilcoxon signed-rank test to compare expression between the primary tumor and center and normal tissue and between the primary tumor center and metastatic lymph nodes. A samples size of n=200 paired samples can provide 85% power to detect mean differences of 0.32. Ordinal logistic regression models of IHC staining score with tissue type (tumor or normal) and histology as covariates are used. If there are adequate numbers of each subtype analysis, the Wilcoxon signed-rank tests separately can be performed separately in each of the histology groups. Survival analyses, both overall and after sub-grouping subjects by gender, ethnicity, overall survival and histology are performed. Kaplan-Meier survival curves are plotted by MOR staining score, histology, age, ethnicity, gender and overall survival as covariates.

For *mu* opioid receptor (MOR) A118G Single Nucleotide Polymorphism (SNP) analysis, TMAs are built as stated above based on de-identified patient cancer samples. About 1 µg of DNA per sample is utilized to examine the association between various cancer parameters (see below) and A118G SNP (rsI799971, located within the first exon) of the *mu* opioid receptor gene OPRMI. Genotyping is performed on all patient samples and is repeated on a 10% random sample of participants. Using these techniques, the association between the presence of the A1I8G MOR SNP with gender, ethnicity, tumor location and overall survival is examined. The association between expression levels of MOR and other biomarkers can be assessed by computing Spearman rank correlation co-efficients. Survival analyses, both overall and after sub-grouping subjects by gender, ethnicity, overall survival and histology are examined. Kaplan-Meier survival curves can be plotted by A/A, A/G or G/G 118 MOR analysis, histology, age, ethnicity, gender and overall survival as covariates. Expression levels and/ or mutational status of MOR can also be considered as prognostic factors. The effects of MOR parameters and other relevant biomarkers within each subgroup are examined to determine whether MOR has prognostic value and whether these effects are consistent across various demographical or clinical subgroups.

Optional plasma and peripheral mononuclear blood cells (PBMCs) samples are obtained before the first dose of study medication and around day 15 and day 29 of Cycle 1.

Individual plasma concentration-time data for paclitaxel and dasatinib are used to generate pharmacokinetic parameter estimates using both compartmental and non-compartmental methods. The peak plasma concentration (Cₘₐₓ) and the time to peak concentration (Tₘₐₓ) are determined by observation of the data. The area under the plasma concentration-time curve (AUC) from 0 to 24 hours post-dose (AUC₀₋₂₄) is also calculated. Drug clearance (Cl) is determined by dose/AUC; elimination half-life (t_{1/2}) is calculated by 0.693/k; and the apparent volume of distribution is calculated by Cl/k. The accumulation ratio for each agent is calculated as the ratio of AUC₀₋₂₄ on Cycle 1, Day 1 vs. Cycle 1, Day 15.

Blood sampling schema is based on both agents being administered concurrently. For paclitaxel administered as a 1 hour infusion, blood samples are taken on days 1 and 15 of cycle 1 just before the administration of the dose, then 30 min (± 5 min) and 50 min (± 5 min) post start of infusion, then 10 min (± 5 min), 30 min (± 5 min), 1 hr (± 10 min), 2 hr (± 10 min), 4 hr (± 10 min), 8 hr (± 2 hr), and 24 hr (± 2 hr) after the end of infusion. For dasatinib administered orally, blood samples are taken on days 1 and 15 of cycle 1 just before the administration of the dose, then 50 min (± 10 min), 2 hr (± 10 min), 5 hr (± 10 min), 9 hr (± 2 hr), and 24 hr (± 2 hr) postdose. For methylnaltrexone administered subcutaneously, blood samples are taken on days 1 and 15 of cycle 1 just before the administration of the dose, then 50 min (± 10 min), 2 hr (± 10 min), 5 hr (± 10 min), 9 hr (± 2 hr), and 24 hr (± 2 hr) postdose.

All subjects are monitored for new and ongoing adverse events until 28 days after the last dose of study treatment. All serious adverse events are followed until resolution. Subjects who discontinue from study treatment for reasons other than disease progression or withdrawal of consent from study follow-up are followed up until disease progression or death is documented. All subjects who enter the study are followed up until death unless they withdraw their consent from study follow-up.

### EXAMPLE 6

A patient is diagnosed with cancer and is undergoing opioid therapy with at least one opioid. To determine suitability of the patient for treatment with a *mu* opioid receptor antagonist, the patient is administered a composition comprising a *mu* opioid receptor antagonist (*e*.*g*., a PAMORA, naloxone, or naltrexone), and time to laxation is evaluated. If the patient experiences a bowel movement or laxation response within about 0-1, 0-4, 0-8, 0-12 or 0-24 hours of administration of the composition, the patient is determined to be a suitable candidate for treatment. The patient is then commenced on a daily regimen of *mu* opioid receptor antagonist treatment to prevent or attenuate further disease progression or spread of the cancer. The regimen is continued until the patient enters remission or optionally, until the patient's end of life.

### EXAMPLE 7

A patient is diagnosed with cancer and is undergoing opioid therapy with at least one opioid. To determine suitability of the subject for treatment with a *mu* opioid receptor antagonist, the subject is administered a composition comprising a *mu* opioid receptor antagonist (*e*.*g*., a PAMORA, naloxone, or naltrexone), and time to laxation is evaluated. If the subject experiences a bowel movement or laxation response within about 1 hour of administration of the composition, the subject is determined to be a suitable candidate for treatment. The subject is then commenced on a daily regimen of *mu* opioid receptor antagonist treatment to prevent or attenuate further disease progression or spread of the cancer. The regimen is continued until the subject enters remission or optionally, until the subject's end of life.

### EXAMPLE 8

A subject is diagnosed with cancer and is undergoing opioid therapy with at least one opioid. To determine suitability of the subject for treatment with a *mu* opioid receptor antagonist, the subject is administered a composition comprising a *mu* opioid receptor antagonist (*e.g.,* a PAMORA, including, but not limited to, *e.g.,* naloxone, naltrexone or methylnaltrexone), and time to laxation is evaluated. If the subject experiences a bowel movement or laxation response within about 4 hours of administration of the composition, the subject is determined to be a suitable candidate for treatment. The subject is then commenced on a daily regimen of *mu* opioid receptor antagonist treatment to prevent or attenuate further disease progression or spread of the cancer. The regimen is continued until the subject enters remission or optionally, until the subject's end of life.

### EXAMPLE 9

A subject is diagnosed with cancer and is undergoing opioid therapy with at least one opioid. To determine suitability of the subject for treatment with a *mu* opioid receptor antagonist, the subject is administered a composition comprising a *mu* opioid receptor antagonist (*e*.*g*., a PAMORA, naloxone, or naltrexone), and time to laxation is evaluated. If the subject experiences a bowel movement or laxation response within about 8 hours of administration of the composition, the subject is determined to be a suitable candidate for treatment. The subject is then commenced on a daily regimen of *mu* opioid receptor antagonist treatment to prevent or attenuate further disease progression or spread of the cancer. The regimen is continued until the subject enters remission or optionally, until the subject's end of life.

### EXAMPLE 10

A subject is diagnosed with cancer and is undergoing opioid therapy with at least one opioid. To determine suitability of the subject for treatment with a *mu* opioid receptor antagonist, the subject is administered a composition comprising a *mu* opioid receptor antagonist (*e*.*g*., a PAMORA, naloxone, or naltrexone), and time to laxation is evaluated. If the subject experiences a bowel movement or laxation response within about 12 hours of administration of the composition, the subject is determined to be a suitable candidate for treatment. The subject is then commenced on a daily regimen of *mu* opioid receptor antagonist treatment to prevent or attenuate further disease progression or spread of the cancer. The regimen is continued until the subject enters remission or optionally, until the subject's end of life.

### EXAMPLE 11

A subject is diagnosed with cancer and is undergoing opioid therapy with at least one opioid. To determine suitability of the subject for treatment with a *mu* opioid receptor antagonist, the subject is administered a composition comprising a *mu* opioid receptor antagonist (*e*.*g*., a PAMORA, naloxone, or naltrexone), and time to laxation is evaluated. If the subject experiences a bowel movement or laxation response within about 24 hours of administration of the composition, the subject is determined to be a suitable candidate for treatment. The subject is then commenced on a daily regimen of *mu* opioid receptor treatment to prevent or attenuate further disease progression or spread of the cancer. The regimen is continued until the subject enters remission or optionally, until the subject's end of life.

### EXAMPLE 12

A subject is diagnosed with cancer and is undergoing opioid therapy with at least one opioid. To determine suitability of the subject for treatment with a *mu* opioid receptor antagonist, the subject is administered a composition comprising a *mu* opioid receptor antagonist (*e*.*g*., a PAMORA, naloxone, or naltrexone), and time to laxation is evaluated. If the subject experiences a bowel movement or laxation response within about 4 hours after at least two doses of the composition, the subject is determined to be a suitable candidate for treatment. The subject is then commenced on a daily regimen of *mu* opioid receptor antagonist treatment to prevent or attenuate further disease progression or spread of the cancer. The regimen is continued until the subject enters remission or optionally, until the subject's end of life.

### EXAMPLE 13

A subject is diagnosed with pancreatic cancer and administered opioid therapy prior to or while undergoing cancer therapy. The subject is also administered a composition comprising a *mu* opioid receptor antagonist *(e.g.,* a PAMORA, naloxone, or naltrexone). In some embodiments, after administration of the composition comprising the *mu* opioid receptor antagonist, the subject experiences a bowel movement or laxation response within about 0-1, 0-4, 0-8, 0-12 or 0-24 hours after administration of at least one dose of the composition. As a result of administration of the composition, the subject also experiences attenuation of further disease progression, remission of the cancer and/or prolonged survival. The administration of the composition is continued for the duration of the subject's life.

### EXAMPLE 14

A subject is diagnosed with breast cancer and administered opioid therapy prior to or while undergoing cancer therapy. The subject is also administered a composition comprising a *mu* opioid receptor antagonist *(e.g.,* a PAMORA, naloxone, or naltrexone). In some embodiments, after administration of the composition comprising the *mu* opioid receptor antagonist, the subject experiences a bowel movement or laxation response within about 0-1, 0-4, 0-8, 0-12 or 0-24 hours after administration of at least one dose of the composition. As a result of administration of the composition, the subject also experiences attenuation of further disease progression, remission of the cancer and/or prolonged survival. The administration of the composition is continued for the duration of the subject's life.

### EXAMPLE 15

A subject is diagnosed with colon cancer and administered opioid therapy prior to or while undergoing cancer therapy. The subject is also administered a composition comprising a *mu* opioid receptor antagonist *(e.g.,* a PAMORA, naloxone, or naltrexone). In some embodiments, after administration of the composition comprising the *mu* opioid receptor antagonist, the subject experiences a bowel movement or laxation response within about 0-1, 0-4, 0-8, 0-12 or 0-24 hours after administration of at least one dose of the composition. As a result of administration of the composition, the subject also experiences attenuation of further disease progression, remission of the cancer and/or prolonged survival. The administration of the composition is continued for the duration of the subject's life.

### EXAMPLE 16

A subject is diagnosed with prostate cancer and administered opioid therapy prior to or while undergoing cancer therapy. The subject is also administered a composition comprising a *mu* opioid receptor antagonist *(e.g.,* a PAMORA, naloxone, or naltrexone). In some embodiments, after administration of the composition comprising the *mu* opioid receptor antagonist, the subject experiences a bowel movement or laxation response within about 0-1, 0-4, 0-8, 0-12 or 0-24 hours after administration of at least one dose of the composition. As a result of administration of the composition, the subject also experiences attenuation of further disease progression, remission of the cancer and/or prolonged survival. The administration of the composition is continued for the duration of the subject's life.

### EXAMPLE 17

A subject is diagnosed with lung cancer and administered opioid therapy prior to or while undergoing cancer therapy. The subject is also administered a composition comprising a *mu* opioid receptor antagonist *(e.g.,* a PAMORA, naloxone, or naltrexone). In some embodiments, after administration of the composition comprising the *mu* opioid receptor antagonist, the subject experiences a bowel movement or laxation response within about 0-1, 0-4, 0-8, 0-12 or 0-24 hours after administration of at least one dose of the composition. As a result of administration of the composition, the subject also experiences attenuation of further disease progression, remission of the cancer and/or prolonged survival. The administration of the composition is continued for the duration of the subject's life.

### EXAMPLE 18

A subject is diagnosed with cancer. To determine suitability of the subject for treatment with a *mu* opioid receptor antagonist, the subject is administered a composition comprising a *mu* opioid receptor antagonist *(e.g.,* a PAMORA, naloxone, or naltrexone), and time to laxation is evaluated. If the subject experiences a bowel movement or laxation response within about 0-1, 0-4, 0-8, 0-12 or 0-24 hours of administration of the composition, the subject is determined to be a suitable candidate for treatment. The subject is then commenced on a daily regimen of *mu* opioid receptor antagonist treatment to prevent or attenuate further disease progression or spread of the cancer. The regimen is continued until the subject enters remission or optionally, until the subject's end of life.

### EXAMPLE 19

A subject is diagnosed with cancer. To determine suitability of the subject for treatment with a *mu* opioid receptor antagonist, the subject is administered a composition comprising a *mu* opioid receptor antagonist *(e.g.,* a PAMORA, naloxone, or naltrexone), and time to laxation is evaluated. If the subject experiences a bowel movement or laxation response within about 1 hour of administration of the composition, the subject is determined to be a suitable candidate for treatment. The subject is then commenced on a daily regimen of *mu* opioid receptor antagonist treatment to prevent or attenuate further disease progression or spread of the cancer. The regimen is continued until the subject enters remission or optionally, until the subject's end of life.

### EXAMPLE 20

A subject is diagnosed with cancer. To determine suitability of the subject for treatment with a *mu* opioid receptor antagonist, the subject is administered a composition comprising a *mu* opioid receptor antagonist *(e.g.,* a PAMORA, including, but not limited to, e.g., naloxone, naltrexone or methylnaltrexone), and time to laxation is evaluated. If the subject experiences a bowel movement or laxation response within about 4 hours of administration of the composition, the subject is determined to be a suitable candidate for treatment. The subject is then commenced on a daily regimen of *mu* opioid receptor antagonist treatment to prevent or attenuate further disease progression or spread of the cancer. The regimen is continued until the subject enters remission or optionally, until the subject's end of life.

### EXAMPLE 21

A subject is diagnosed with cancer. To determine suitability of the subject for treatment with a *mu* opioid receptor antagonist, the subject is administered a composition comprising a *mu* opioid receptor antagonist (*e.g.,* a PAMORA, naloxone, or naltrexone), and time to laxation is evaluated. If the subject experiences a bowel movement or laxation response within about 8 hours of administration of the composition, the subject is determined to be a suitable candidate for treatment. The subject is then commenced on a daily regimen of *mu* opioid receptor antagonist treatment to prevent or attenuate further disease progression or spread of the cancer. The regimen is continued until the subject enters remission or optionally, until the subject's end of life.

### EXAMPLE 22

A subject is diagnosed with cancer. To determine suitability of the subject for treatment with a *mu* opioid receptor antagonist, the subject is administered a composition comprising a *mu* opioid receptor antagonist *(e.g.,* a PAMORA, naloxone, or naltrexone), and time to laxation is evaluated. If the subject experiences a bowel movement or laxation response within about 12 hours of administration of the composition, the subject is determined to be a suitable candidate for treatment. The subject is then commenced on a daily regimen of *mu* opioid receptor antagonist treatment to prevent or attenuate further disease progression or spread of the cancer. The regimen is continued until the subject enters remission or optionally, until the subject's end of life.

### EXAMPLE 23

A subject is diagnosed with cancer. To determine suitability of the subject for treatment with a *mu* opioid receptor antagonist, the subject is administered a composition comprising a *mu* opioid receptor antagonist *(e.g.,* a PAMORA, naloxone, or naltrexone), and time to laxation is evaluated. If the subject experiences a bowel movement or laxation response within about 24 hours of administration of the composition, the subject is determined to be a suitable candidate for treatment. The subject is then commenced on a daily regimen of *mu* opioid receptor treatment to prevent or attenuate further disease progression or spread of the cancer. The regimen is continued until the subject enters remission or optionally, until the subject's end of life.

### EXAMPLE 24

A subject is diagnosed with cancer. To determine suitability of the subject for treatment with a *mu* opioid receptor antagonist, the subject is administered a composition comprising a *mu* opioid receptor antagonist *(e.g.,* a PAMORA, naloxone, or naltrexone), and time to laxation is evaluated. If the subject experiences a bowel movement or laxation response within about 4 hours after at least two doses of the composition, the subject is determined to be a suitable candidate for treatment. The subject is then commenced on a daily regimen of *mu* opioid receptor antagonist treatment to prevent or attenuate further disease progression or spread of the cancer. The regimen is continued until the subject enters remission or optionally, until the subject's end of life.

### EXAMPLE 25

A subject is diagnosed with pancreatic cancer. The subject is administered a composition comprising a *mu* opioid receptor antagonist *(e.g.,* a PAMORA, naloxone, or naltrexone). In some embodiments, after administration of the composition comprising the *mu* opioid receptor antagonist, the subject experiences a bowel movement or laxation response within about 0-1, 0-4, 0-8, 0-12 or 0-24 hours after administration of at least one dose of the composition. As a result of administration of the composition, the subject also experiences attenuation of further disease progression, remission of the cancer and/or prolonged survival. The administration of the composition is continued for the duration of the subject's life.

### EXAMPLE 26

A subject is diagnosed with breast cancer. The subject is administered a composition comprising a *mu* opioid receptor antagonist *(e.g.,* a PAMORA, naloxone, or naltrexone). In some embodiments, after administration of the composition comprising the *mu* opioid receptor antagonist, the subject experiences a bowel movement or laxation response within about 0-1, 0-4, 0-8, 0-12 or 0-24 hours after administration of at least one dose of the composition. As a result of administration of the composition, the subject also experiences attenuation of further disease progression, remission of the cancer and/or prolonged survival. The administration of the composition is continued for the duration of the subject's life.

### EXAMPLE 27

A subject is diagnosed with colon cancer. The subject is administered a composition comprising a *mu* opioid receptor antagonist *(e.g.,* a PAMORA, naloxone, or naltrexone). In some embodiments, after administration of the composition comprising the *mu* opioid receptor antagonist, the subject experiences a bowel movement or laxation response within about 0-1, 0-4, 0-8, 0-12 or 0-24 hours after administration of at least one dose of the composition. As a result of administration of the composition, the subject also experiences attenuation of further disease progression, remission of the cancer and/or prolonged survival. The administration of the composition is continued for the duration of the subject's life.

### EXAMPLE 28

A subject is diagnosed with prostate cancer. The subject is administered a composition comprising a *mu* opioid receptor antagonist (*e.g.,* a PAMORA, naloxone, or naltrexone). In some embodiments, after administration of the composition comprising the *mu* opioid receptor antagonist, the subject experiences a bowel movement or laxation response within about 0-1, 0-4, 0-8, 0-12 or 0-24 hours after administration of at least one dose of the composition. As a result of administration of the composition, the subject also experiences attenuation of further disease progression, remission of the cancer and/or prolonged survival. The administration of the composition is continued for the duration of the subject's life.

### EXAMPLE 29

A subject is diagnosed with lung cancer. The subject is administered a composition comprising a *mu* opioid receptor antagonist *(e.g.,* a PAMORA, naloxone, or naltrexone). In some embodiments, after administration of the composition comprising the *mu* opioid receptor antagonist, the subject experiences a bowel movement or laxation response within about 0-1, 0-4, 0-8, 0-12 or 0-24 hours after administration of at least one dose of the composition. As a result of administration of the composition, the subject also experiences attenuation of further disease progression, remission of the cancer and/or prolonged survival. The administration of the composition is continued for the duration of the subject's life.

### EXAMPLE 30

A subject is diagnosed with cancer. A diagnostic test is carried out on the subject to determine his or her suitability for therapy with a *mu* opioid receptor antagonist. As part of the diagnostic test, the subject is administered a composition containing a *mu* opioid receptor antagonist (*e*.*g*., a PAMORA, naloxone, or naltrexone), and time to first bowel movement or laxation response after administration of the composition is assessed. If the subject experiences a bowel movement or laxation response within about 0 - 1, 0 - 4, 0.5 - 4, 0 - 8, 0 - 12 or 0 - 24 hours after administration of at least one dose of the composition, the subject is considered a suitable candidate for *mu* opioid receptor antagonist therapy.

### EXAMPLE 31

A subject is diagnosed with cancer. A diagnostic test is carried out on the subject to determine his or her suitability for therapy with a *mu* opioid receptor antagonist. As part of the diagnostic test, the subject is administered a composition containing a *mu* opioid receptor antagonist (*e*.*g*., a PAMORA, naloxone, or naltrexone), and time to first bowel movement or laxation response after administration of the composition is assessed. If the subject experiences a bowel movement or laxation response within about 0 - 1, 0 - 4, 0.5 - 4, 0 - 8, 0 - 12 or 0 - 24 hours after administration of at least two out of four doses of the composition, the subject is considered a suitable candidate for *mu* opioid receptor antagonist therapy.

### EXAMPLE 32

A subject is diagnosed with cancer. A diagnostic test is carried out on the subject to determine his or her suitability for therapy with a *mu* opioid receptor antagonist. As part of the diagnostic test, the subject is administered a composition containing a *mu* opioid receptor antagonist (*e*.*g*., a PAMORA, naloxone, or naltrexone), and time to first bowel movement or laxation response after administration of the composition is assessed. If the subject experiences a bowel movement or laxation response within about 0 - 1, 0 - 4, 0.5 - 4, 0 - 8, 0 - 12 or 0 - 24 hours after administration of at least four out of seven doses of the composition, the subject is considered a suitable candidate for *mu* opioid receptor antagonist therapy.

## Claims

1. A *mu* opioid receptor antagonist for use in a method of treating cancer in a subject, increasing the survival of a subject suffering from cancer, slowing or stopping the growth of a tumor in a subject, or inhibiting or slowing the proliferation of tumor cells in a subject, wherein the subject is a fast responder to administration of said *mu* opioid receptor antagonist for treatment of constipation.

2. The *mu* opioid receptor antagonist for use of claim 1, wherein the fast responder is a subject who has a bowel movement or laxation response within about 0 to 1 hour, 0 to 4 hours, 30 minutes to 4 hours, 0 to 8 hours, 0 to 12 hours, or 0 to 24 hours after a single-dose administration of the *mu* opioid receptor antagonist.

3. The *mu* opioid receptor antagonist for use of claim 1 or 2, wherein the subject is also being administered at least one opioid.

4. A composition comprising a *mu* opioid receptor antagonist for use in a method of treating a subject suffering from cancer, comprising identifying the subject as a fast responder to administration of said *mu* opioid receptor antagonist for treatment of constipation, and administering said composition to the subject to prolong survival from cancer.

5. The composition for use of claim 4, wherein identifying the subject as a fast responder comprises administering a composition comprising the *mu* opioid receptor antagonist to a subject having constipation, optionally, opioid induced constipation, and determining the time to a first bowel movement; wherein if the time to a first bowel movement is within about 0-1 hour, 0-4 hours, 30 minutes to 4 hours, 0-8 hours, 0-12 hours or 0 -24 hours of administering the composition to the subject, the subject is a fast responder.

6. The composition for use of claim 5, further comprising administering an opioid prior to or concomitantly with the composition comprising the *mu* opioid receptor antagonist.

7. The *mu* opioid receptor antagonist or composition for use of any one of the preceding claims, wherein
(a) the *mu* opioid receptor antagonist is selected from the group consisting of a tertiary derivative of noroxymorphone, a quaternary derivative of noroxymorphone, a quaternary derivative of benzomorphans, and an N-substituted piperidine;
(b) the *mu* opioid receptor antagonist is a peripherally acting *mu* opioid receptor antagonist (PAMORA);
(c) the *mu* opioid receptor antagonist is a PAMORA selected from the group consisting of naloxegol, alvimopan, axelopran and methylnaltrexone;
(d) the *mu* opioid receptor antagonist comprises a tertiary opioid antagonist;
(e) the *mu* opioid receptor antagonist comprises a tertiary opioid antagonist which is naloxone or naltrexone;
(f) the *mu* opioid receptor antagonist comprises a tertiary opioid antagonist and a PAMORA; or
(g) the *mu* opioid receptor antagonist comprises a compound of formula I: 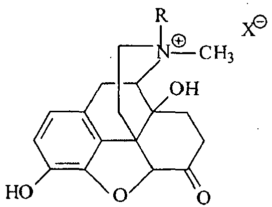 wherein R is allyl, chlorallyl, cyclopropylmethyl or propargyl, and X^{e} is a suitable anion.

8. The *mu* opioid receptor antagonist or composition for use of any one of the preceding claims, wherein tumor growth, tumor metastasis or abnormal proliferation of cells in the subject is opioid- induced; is activated or enhanced by *mu* opioid receptor activity; or is induced by VEGF.

9. The *mu* opioid receptor antagonist for use of any one of claims 1-3, wherein the *mu* opioid receptor antagonist is contained in a composition.

10. The composition for use of any one of claims 4-9, wherein the composition is in the form of a tablet, a capsule, a sachet, a liquid solution, powder for suspension, or a packaged composition.

11. The *mu* opioid receptor antagonist or composition for use of any one of the preceding claims, wherein the *mu* opioid receptor antagonist is orally administered at about 150 mg, about 300 mg, or about 450 mg of methylnaltrexone, or a salt thereof, optionally as at least one tablet comprising 150 mg of methylnaltrexone, or a salt thereof.

12. The *mu* opioid receptor antagonist or composition for use of any one of the preceding claims, wherein the *mu* opioid receptor antagonist is administered:
(a) at a daily dose of from about 0.075 mg/kg body weight to about 0.45 mg/kg body weight;
(b) at a daily dose of about 0.075 mg/kg body weight, about 0.15 mg/kg body weight, about 0.30 mg/kg body weight, or about 0.45 mg/kg body weight;
(c) from about 0.075 mg/kg body weight to about 0.45 mg/kg body weight at least once every other day; or
(d) at a dose of about 0.075 mg/kg body weight, about 0.15 mg/kg body weight, about 0.30 mg/kg body weight, or about 0.45 mg/kg body weight at least once every other day;
optionally for at least about 2 weeks, about 4 weeks, about 14 weeks, about 16 weeks, about 24 weeks, for the duration of the subject's life, or for the duration of the subject's cancer treatment.

13. The *mu* opioid receptor antagonist or composition for use of any one of the preceding claims, wherein the subject is **characterized by** at least one of the following:
(a) has received opioid treatment prior to administration of the *mu* opioid receptor antagonist or composition;
(b) has received opioid treatment prior to administration of the *mu* opioid receptor antagonist or composition for at least one month, or for at least 1 day, 7 days, 14 days, or 30 days;
(c) has received opioid treatment prior to administration of the *mu* opioid receptor antagonist or composition at about 10 to 300 mg, about 20 to 200 mg, or about 25 to 100 mg of oral morphine equivalents per day;
(d) will start opioid treatment in less than 1, 2, 3, or 4 weeks;
(e) has had opioid induced constipation for at least one day, from 1 hour to about 30 days, or for at least 30 days;
(f) has experienced less than 3 rescue free bowel movements for at least one week; or
(g) has experienced less than 3 rescue free bowel movements for at least four consecutive weeks.

14. The *mu* opioid receptor antagonist or composition for use of any one of the preceding claims, wherein the subject is also administered a cancer or an anti-tumor therapy that does not comprise a *mu* opioid receptor antagonist; wherein, optionally
(a) the cancer or anti-tumor therapy comprises a chemotherapeutic agent, radiotherapy, an anti-angiogenic agent, surgery or a combination thereof;
(b) the cancer or anti-tumor therapy comprises dasatinib, bevacizumab, paclitaxel, or a combination thereof;
(c) the cancer or anti-tumor therapy comprises an anti-angiogenic agent selected from an agent that inhibits the activity of VEGF, an anti-VEGF antibody, thalidomide, SU5416, ribozyme, SU6668, or a combination thereof;
(d) the cancer or anti-tumor therapy comprises an inhibitor of Src phosphorylation; or
(e) the *mu* opioid receptor antagonist comprises methylnaltrexone, and the cancer or anti-tumor therapy comprises one or more of dasatinib, bevacizumab, or paclitaxel.

15. The mu opioid receptor antagonist or composition for use of any one of the preceding claims, wherein administration of the *mu* opioid receptor antagonist or composition blocks Src phosphorylation and/ or inhibits or attenuates epithelial mesenchymal transition, optionally, wherein the epithelial mesenchymal transition is opioid-induced, growth-factor induced, or both.

16. The *mu* opioid receptor antagonist or composition for use of any one of the preceding claims, wherein the subject suffers from one or more of a carcinoma, sarcoma, lymphoma, leukemia or blastoma; or alternatively, wherein the subject suffers from one or more of a cancer of: breast, liver, head and neck, esophageal, stomach, small intestine, colon, rectal, anal, skin, glandular, circulatory, prostate, pancreas, hematopoietic, bone marrow, bone, cartilage, fat, nerve, lung, or lymph.

## Patentansprüche

1. *mu*-Opioidrezeptorantagonist zur Verwendung in einem Verfahren zur Behandlung von Krebs bei einem Subjekt, zum Erhöhen des Überlebens eines an Krebs leidenden Subjekts, zum Verlangsamen oder Stoppen des Wachstums eines Tumors bei einem Subjekt oder zum Inhibieren oder Verlangsamen der Proliferation von Tumorzellen bei einem Subjekt, wobei das Subjekt eines ist, das auf die Verabreichung des mu-Opioidrezeptorantagonisten zur Behandlung von Konstipation schnell anspricht.

2. *mu*-Opioidrezeptorantagonist zur Verwendung nach Anspruch 1, wobei das Subjekt, das schnell anspricht, ein Subjekt ist, das innerhalb von etwa 0 bis 1 Stunde, 0 bis 4 Stunden, 30 Minuten bis 4 Stunden, 0 bis 8 Stunden, 0 bis 12 Stunden oder 0 bis 24 Stunden nach einer Einzeldosisverabreichung des *mu-*Opioidrezeptorantagonisten eine Darmbewegungs- oder Darmentleerungsantwort aufweist.

3. *mu*-Opioidrezeptorantagonist zur Verwendung nach Anspruch 1 oder 2, wobei dem Subjekt auch wenigstens ein Opioid verabreicht wird.

4. Zusammensetzung, die einen *mu*-Opioidrezeptorantagonisten umfasst, zur Verwendung in einem Verfahren zur Behandlung eines an Krebs leidenden Subjekts, umfassend das Identifizieren des Subjekts als eines, das auf die Verabreichung des *mu-*Opioidrezeptorantagonisten zur Behandlung von Konstipation schnell anspricht, und Verabreichen der Zusammensetzung an das Subjekt, um das Überleben mit Krebs zu verlängern.

5. Zusammensetzung zur Verwendung nach Anspruch 4, wobei das Identifizieren des Subjekts als eines, das schnell anspricht, Verabreichen einer Zusammensetzung, die den *mu*-Opioidrezeptorantagonisten an ein Subjekt mit Konstipation, gegebenenfalls opioidinduzierter Konstipation, und Bestimmen der Zeit bis zu einer ersten Darmbewegung umfasst; wobei, falls die Zeit bis zu einer ersten Darmbewegung innerhalb von etwa 0 bis 1 Stunde, 0 bis 4 Stunden, 30 Minuten bis 4 Stunden, 0 bis 8 Stunden, 0 bis 12 Stunden oder 0 bis 24 Stunden nach der Verabreichung der Zusammensetzung an das Subjekt liegt, das Subjekt eines ist, das schnell anspricht.

6. Zusammensetzung zur Verwendung nach Anspruch 5, weiterhin umfassend Verabreichen eines Opioids vor oder gleichzeitig mit der Zusammensetzung, die den *mu*-Opioidrezeptorantagonisten umfasst.

7. *mu*-Opioidrezeptorantagonist oder Zusammensetzung zur Verwendung nach einem der vorangehenden Ansprüche, worin
(a) der *mu*-Opioidrezeptorantagonist ausgewählt ist aus der Gruppe, bestehend aus einem tertiären Derivat von Noroxymorphon, einem quaternären Derivat von Noroxymorphon, einem quaternären Derivat von Benzomorphanen und einem N-substituierten Piperidin;
(b) der *mu*-Opioidrezeptorantagonist ein peripher wirkender *mu*-Opioidrezeptorantagonist (PAMORA) ist;
(c) der *mu*-Opioidrezeptorantagonist ein PAMORA, ausgewählt aus der Gruppe, bestehend aus Naloxegol, Alvimopan, Axelopran und Methylnaltrexon, ist;
(d) der *mu*-Opioidrezeptorantagonist einen tertiären Opioidrezeptorantagonisten umfasst;
(e) der *mu*-Opioidrezeptorantagonist einen tertiären Opioidrezeptorantagonisten, der Naloxon oder Naltrexon ist, umfasst;
(f) der *mu*-Opioidrezeptorantagonist einen tertiären Opioidrezeptorantagonisten und einen PAMORA umfasst; oder
(g) der *mu*-Opioidrezeptorantagonist eine Verbindung der Formel I umfasst: 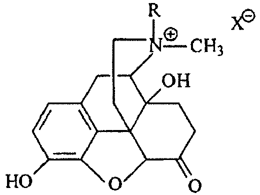 worin R Allyl, Chlorallyl, Cyclopropylmethyl oder Propargyl ist und X⁻ ein geeignetes Anion ist.

8. *mu*-Opioidrezeptorantagonist oder Zusammensetzung zur Verwendung nach einem der vorangehenden Ansprüche, wobei Tumorwachstum, Tumormetastase oder abnormale Proliferation von Zellen bei dem Subjekt opioidinduziert ist; durch *mu*-Opioidrezeptoraktivität aktiviert oder verstärkt ist; oder durch VEGF induziert ist.

9. *mu*-Opioidrezeptorantagonist zur Verwendung nach einem der Ansprüche 1 bis 3, wobei der mu-Opioidrezeptorantagonist in einer Zusammensetzung enthalten ist.

10. Zusammensetzung zur Verwendung nach einem der Ansprüche 4 bis 9, wobei die Zusammensetzung in der Form einer Tablette, einer Kapsel, einem Sachet, einer flüssigen Lösung, einem Pulver zur Suspension oder als eine verpackte Zusammensetzung vorliegt.

11. *mu*-Opioidrezeptorantagonist oder Zusammensetzung zur Verwendung nach einem der vorangehenden Ansprüche, wobei der *mu-*Opioidrezeptorantagonist bei etwa 150 mg, etwa 300 mg oder etwa 450 mg Methylnaltrexon oder eines Salzes davon, gegebenenfalls als wenigstens eine Tablette, die 150 mg Methylnaltrexon oder ein Salz davon umfasst, oral verabreicht wird.

12. *mu*-Opioidrezeptorantagonist oder Zusammensetzung zur Verwendung nach einem der vorangehenden Ansprüche, wobei der *mu-*Opioidrezeptorantagonist folgendermaßen verabreicht wird:
(a) bei einer täglichen Dosis von etwa 0,075 mg/kg Körpergewicht bis etwa 0,45 mg/kg Körpergewicht;
(b) bei einer täglichen Dosis von etwa 0,075 mg/kg Körpergewicht, etwa 0,15 mg/kg Körpergewicht, etwa 0,30 mg/kg Körpergewicht oder etwa 0,45 mg/kg Körpergewicht;
(c) von etwa 0,075 mg/kg Körpergewicht bis etwa 0,45 mg/kg Körpergewicht mindestens einmal alle zwei Tage; oder
(d) bei einer Dosis von etwa 0,075 mg/kg Körpergewicht, etwa 0,15 mg/kg Körpergewicht, etwa 0,30 mg/kg Körpergewicht oder etwa 0,45 mg/kg Körpergewicht mindestens einmal alle zwei Tage;
gegebenenfalls für wenigstens etwa 2 Wochen, etwa 4 Wochen, etwa 14 Wochen, etwa 16 Wochen, etwa 24 Wochen, für die Dauer des Lebens des Subjekts oder für die Dauer der Krebsbehandlung des Subjekts.

13. *mu*-Opioidrezeptorantagonist oder Zusammensetzung zur Verwendung nach einem der vorangehenden Ansprüche, wobei das Subjekt durch wenigstens einen der folgenden Punkte charakterisiert ist:
(a) es hat vor der Verabreichung des *mu*-Opioidrezeptorantagonisten oder der Zusammensetzung eine Opioidbehandlung erhalten;
(b) es hat vor der Verabreichung des *mu*-Opioidrezeptorantagonisten oder der Zusammensetzung für wenigstens einen Monat oder für wenigstens 1 Tag, 7 Tage, 14 Tage oder 30 Tage eine Opioidbehandlung erhalten;
(c) es hat vor der Verabreichung des *mu*-Opioidrezeptorantagonisten oder der Zusammensetzung bei etwa 10 bis 300 mg, etwa 20 bis 200 mg oder etwa 25 bis 100 mg oralen Morphinäquivalenten pro Tag eine Opioidbehandlung erhalten;
(d) es wird eine Opioidbehandlung in weniger als 1, 2, 3 oder 4 Woche(n) beginnen;
(e) es hat eine opioidinduzierte Konstipation für wenigstens einen Tag, von 1 Stunde bis etwa 30 Tage oder für wenigstens 30 Tage;
(f) es hat weniger als 3 Darmbewegungen ohne Notfallbehandlung für wenigstens eine Woche erfahren; oder
(g) es hat weniger als 3 Darmbewegungen ohne Notfallbehandlung für wenigstens vier aufeinanderfolgende Wochen erfahren.

14. *mu*-Opioidrezeptorantagonist oder Zusammensetzung zur Verwendung nach einem der vorangehenden Ansprüche, wobei dem Subjekt auch eine Krebs- oder eine Antitumortherapie, die keinen *mu*-Opioidrezeptorantagonisten umfasst, verabreicht wird; wobei gegebenenfalls
(a) die Krebs- oder Antitumortherapie ein chemotherapeutisches Mittel, eine Radiotherapie, ein antiangiogenes Mittel, eine Operation oder eine Kombination davon umfasst;
(b) die Krebs- oder Antitumortherapie Dasatinib, Bevacizumab, Paclitaxel oder eine Kombination davon umfasst;
(c) die Krebs- oder Antitumortherapie ein antiangiogenes Mittel, ausgewählt aus einem Mittel, das die Aktivität von VEGF inhibiert, einem Anti-VEGF-Antikörper, Thalidomid, SU5416, Ribozym, SU6668 oder eine Kombination davon umfasst;
(d) die Krebs- oder Antitumortherapie einen Inbibitor von Src-Phosphorylierung umfasst; oder
(e) der *mu*-Opioidrezeptorantagonist Methylnaltrexon umfasst, und die Krebs- oder Antitumortherapie eines oder mehrere von Dasatinib, Bevacizumab oder Paclitaxel umfasst.

15. *mu*-Opioidrezeptorantagonist oder Zusammensetzung zur Verwendung nach einem der vorangehenden Ansprüche, wobei die Verabreichung des *mu*-Opioidrezeptorantagonisten oder der Zusammensetzung die Src-Phosphorylierung blockiert und/oder die Epithelial-mesenchymale Transition inhibiert oder abschwächt, wobei die Epithelial-mesenchymale Transition gegebenenfalls opioidinduziert, wachstumsfaktorinduziert oder beides ist.

16. *mu*-Opioidrezeptorantagonist oder Zusammensetzung zur Verwendung nach einem der vorangehenden Ansprüche, wobei das Subjekt an einem oder mehreren von Karzinom, Sarkom, Lymphom, Leukämie oder Blastom leidet; oder wobei das Subjekt alternativ an einer oder mehreren der folgenden Krebsarten leidet: Brust, Leber, Kopf und Nacken, Ösophagus, Magen, Dünndarm, Colon, rektal, anal, Haut, glandulär, Kreislauf, Prostata, Pankreas, hämatopoetisch, Knochenmark, Knochen, Knorpel, Fett, Nerven, Lunge oder Lymphe.

## Revendications

1. Antagoniste de récepteurs µ-opioïdes pour utilisation dans un procédé de traitement du cancer chez un sujet, d'augmentation de la survie d'un sujet souffrant du cancer, de ralentissement ou d'arrêt de la croissance d'une tumeur chez un sujet ou d'inhibition ou de ralentissement de la prolifération de cellules tumorales chez un sujet, dans lequel le sujet est un répondeur rapide à l'administration dudit antagoniste de récepteurs µ-opioïdes pour le traitement de la constipation.

2. Antagoniste de récepteurs µ-opioïdes pour utilisation selon la revendication 1, dans lequel le répondeur rapide est un sujet qui présente une réponse en matière de selles ou de défécation en environ de 0 à 1 heure, de 0 à 4 heures, de 30 minutes à 4 heures, de 0 à 8 heures, de 0 à 12 heures ou de 0 à 24 heures après l'administration d'une seule dose de l'antagoniste de récepteurs µ-opioïdes.

3. Antagoniste de récepteurs µ-opioïdes pour utilisation selon la revendication 1 ou 2, dans lequel on administre également au sujet au moins un opioïde.

4. Composition comprenant un antagoniste de récepteurs µ-opioïdes pour utilisation dans un procédé de traitement d'un sujet souffrant du cancer, comprenant l'identification du sujet comme répondeur rapide à l'administration dudit antagoniste de récepteurs µ-opioïdes pour le traitement de la constipation, et l'administration de ladite composition au sujet pour prolonger la survie à l'égard du cancer.

5. Composition pour utilisation selon la revendication 4, dans laquelle l'identification du sujet comme répondeur rapide comprend l'administration d'une composition comprenant l'antagoniste de récepteurs µ-opioïdes à un sujet présentant une constipation, éventuellement une constipation induite par un opioïde, et la détermination du moment où se produisent les premières selles ; dans laquelle, si le moment où se produisent les premières selles se situe à environ de 0 à 1 heure, de 0 à 4 heures, de 30 minutes à 4 heures, de 0 à 8 heures, de 0 à 12 heures ou de 0 à 24 heures après l'administration de la composition au sujet, le sujet est un répondeur rapide.

6. Composition pour utilisation selon la revendication 5, comprenant en outre l'administration d'un opioïde avant ou simultanément avec la composition comprenant l'antagoniste de récepteurs µ-opioïdes.

7. Antagoniste de récepteurs µ-opioïdes ou composition pour utilisation selon l'une quelconque des revendications précédentes, dans lequel ou laquelle :
(a) l'antagoniste de récepteurs µ-opioïdes est sélectionné dans le groupe constitué d'un dérivé tertiaire de la noroxymorphone, d'un dérivé quaternaire de la noroxymorphone, d'un dérivé quaternaire des benzomorphanes et d'une pipéridine N-substituée ;
(b) l'antagoniste de récepteurs µ-opioïdes est un antagoniste de récepteurs µ-opioïdes à action périphérique (PAMORA) ;
(c) l'antagoniste de récepteurs µ-opioïdes est un PAMORA sélectionné dans le groupe constitué du naloxegol, de l'alvimopane, de l'axéloprane et du méthylnaltrexone ;
(d) l'antagoniste de récepteurs µ-opioïdes comprend un antagoniste d'opioïde tertiaire ;
(e) l'antagoniste de récepteurs µ-opioïdes comprend un antagoniste d'opioïde tertiaire qui est la naloxone ou le naltrexone ;
(f) l'antagoniste de récepteurs µ-opioïdes comprend un antagoniste d'opioïde tertiaire et un PAMORA ; ou
(g) l'antagoniste de récepteurs µ-opioïdes comprend un composé de formule I : 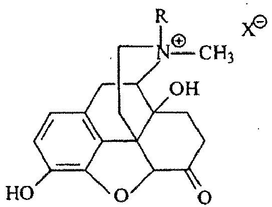 dans laquelle R est un groupement allyle, chlorallyle, cyclopropylméthyle ou propargyle et x^{Θ} est un anion approprié.

8. Antagoniste de récepteurs µ-opioïdes ou composition pour utilisation selon l'une quelconque des revendications précédentes, dans lequel ou laquelle une croissance tumorale, une métastase tumorale ou une prolifération anormale de cellules chez le sujet est induite par un opioïde ; est activée ou renforcée par l'activité de récepteurs µ-opioïdes ; ou est induite par le facteur de croissance de l'endothélium vasculaire (VEGF).

9. Antagoniste de récepteurs µ-opioïdes pour utilisation selon l'une quelconque des revendications 1 à 3, dans lequel l'antagoniste de récepteurs µ-opioïdes est contenu dans une composition.

10. Composition pour utilisation selon l'une quelconque des revendications 4 à 9, dans laquelle la composition se présente sous la forme d'un comprimé, d'une capsule, d'un sachet, d'une solution liquide, d'une poudre pour suspension ou d'une composition conditionnée.

11. Antagoniste de récepteurs µ-opioïdes ou composition pour utilisation selon l'une quelconque des revendications précédentes, dans lequel ou laquelle l'antagoniste de récepteurs µ-opioïdes est administré par voie buccale à raison d'environ 150 mg, d'environ 300 mg, ou d'environ 450 mg de méthylnaltrexone ou d'un sel de celui-ci, éventuellement sous la forme d'au moins un comprimé comprenant 150 mg de méthylnaltrexone ou d'un sel de celui-ci.

12. Antagoniste de récepteurs µ-opioïdes ou composition pour utilisation selon l'une quelconque des revendications précédentes, dans lequel ou laquelle l'antagoniste de récepteurs µ-opioïdes est administré :
(a) à une dose quotidienne d'environ 0,075 mg/kg de poids corporel à environ 0,45 mg/kg de poids corporel ;
(b) à une dose quotidienne d'environ 0,075 mg/kg de poids corporel, d'environ 0,15 mg/kg de poids corporel, d'environ 0,30 mg/kg de poids corporel ou d'environ 0,45 mg/kg de poids corporel ;
(c) à raison d'environ 0,075 mg/kg de poids corporel à environ 0,45 mg/kg de poids corporel au moins une fois tous les deux jours ; ou
(d) à une dose d'environ 0,075 mg/kg de poids corporel, d'environ 0,15 mg/kg de poids corporel, d'environ 0,30 mg/kg de poids corporel, ou d'environ 0,45 mg/kg de poids corporel au moins une fois tous les deux jours ;
éventuellement pendant au moins environ 2 semaines, environ 4 semaines, environ 14 semaines, environ 16 semaines, environ 24 semaines, pendant la durée de vie du sujet, ou pendant la durée du traitement du cancer du sujet.

13. Antagoniste de récepteurs µ-opioïdes ou composition pour utilisation selon l'une quelconque des revendications précédentes, dans lequel ou laquelle le sujet est **caractérisé par** au moins l'une des situations suivantes :
(a) il a reçu un traitement d'opioïde avant l'administration de l'antagoniste de récepteurs µ-opioïdes ou de la composition ;
(b) il a reçu un traitement d'opioïde avant l'administration de l'antagoniste de récepteurs µ-opioïdes ou de la composition pendant au moins un mois, ou pendant au moins 1 jour, 7 jours, 14 jours ou 30 jours ;
(c) il a reçu un traitement d'opioïde avant l'administration de l'antagoniste de récepteurs µ-opioïdes ou de la composition à raison d'environ 10 à 300 mg, d'environ 20 à 200 mg, ou d'environ 25 à 100 mg d'équivalents de morphine buccaux par jour ;
(d) il commencera un traitement d'opioïde dans moins de 1, 2, 3 ou 4 semaines ;
(e) il a présenté une constipation induite par un opioïde pendant au moins un jour, de 1 heure à environ 30 jours, ou pendant au moins 30 jours ;
(f) il a subi moins de 3 selles spontanées pendant au moins une semaine ; ou
(g) il a subi moins de 3 selles spontanées pendant au moins quatre semaines consécutives.

14. Antagoniste de récepteurs µ-opioïdes ou composition pour utilisation selon l'une quelconque des revendications précédentes, dans lequel ou laquelle on administre également au sujet une thérapie anticancéreuse ou antitumorale qui ne comprend pas un antagoniste de récepteurs µ-opioïdes ; dans lequel ou laquelle, éventuellement
(a) la thérapie anticancéreuse ou antitumorale comprend un agent chimiothérapeutique, une radiothérapie, un agent anti-angiogénique, une chirurgie ou une combinaison de ceux-ci ;
(b) la thérapie anticancéreuse ou antitumorale comprend l'administration de dasatinib, de bévacizumab, de paclitaxel ou d'une combinaison de ceux-ci ;
(c) la thérapie anticancéreuse ou antitumorale comprend un agent anti-angiogénique sélectionné parmi un agent qui inhibe l'activité du VEGF, un anticorps anti-VEGF, la thalidomide, le SU5416, le ribozyme, le SU6668 ou une combinaison de ceux-ci ;
(d) la thérapie anticancéreuse ou antitumorale comprend in inhibiteur de phosphorylation de Src ; ou
(e) l'antagoniste de récepteurs µ-opioïdes comprend le méthylnaltrexone, et la thérapie anticancéreuse ou antitumorale comprend un ou plusieurs du dasatinib, du bévacizumab ou du paclitaxel.

15. Antagoniste de récepteurs µ-opioïdes ou composition pour utilisation selon l'une quelconque des revendications précédentes, dans lequel ou laquelle l'administration de l'antagoniste de récepteurs µ-opioïdes ou de la composition bloque la phosphorylation de Src et/ou inhibe ou atténue la transition épithéliale mésenchymateuse, éventuellement, dans lequel ou laquelle la transition épithéliale mésenchymateuse est induite par un opioïde, induite par un facteur de croissance, ou les deux.

16. Antagoniste de récepteurs µ-opioïdes ou composition pour utilisation selon l'une quelconque des revendications précédentes, dans lequel ou laquelle le sujet souffre d'un ou plusieurs parmi un carcinome, un sarcome, un lymphome, une leucémie ou un blastome ; ou, alternativement, dans lequel ou laquelle le sujet souffre d'une ou plusieurs formes de cancer parmi : du sein, du foie, de la tête et du cou, de l'œsophage, de l'estomac, de l'intestin grêle, du colon, au plan rectal, au plan anal, de la peau, au plan glandulaire, au plan circulatoire, de la prostate, du pancréas, au plan hématopoïétique, de la moëlle osseuse, des os, des cartilages, des graisses, des nerfs, des poumons ou de la lymphe.
